(19)

**Europäisches Patentamt**
**European Patent Office**
**Office européen des brevets**

(11) **EP 4 146 833 B1**

(12) **EUROPEAN PATENT SPECIFICATION**

(45) Date of publication and mention
of the grant of the patent:
**28.01.2026 Bulletin 2026/05**

(21) Application number: **21728437.1**

(22) Date of filing: **05.05.2021**

(51) International Patent Classification (IPC):
**C12Q 1/70** (2006.01)   **C12Q 1/6844** (2018.01)

(52) Cooperative Patent Classification (CPC):
(C-Sets available)
**C12Q 1/701; C12Q 1/6844;** C12Q 2600/118
(Cont.)

(86) International application number:
**PCT/EP2021/061918**

(87) International publication number:
**WO 2021/224355 (11.11.2021 Gazette 2021/45)**

(54) **DIAGNOSTIC PRIMERS, KITS AND METHODS FOR VIRAL DETECTION**

DIAGNOSTISCHE PRIMER, KITS UND VERFAHREN ZUR VIRALEN DETEKTION

AMORCES DE DIAGNOSTIC, TROUSSES ET METHODES POUR LA DETECTION VIRALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.05.2020 GB 202006663
05.05.2020 GB 202006664**

(43) Date of publication of application:
**15.03.2023 Bulletin 2023/11**

(73) Proprietor: **Genomtec SA**
**51-317 Wroclaw (PL)**

(72) Inventors:
• **TOKARSKI, Miron**
**49-300 Brzeg (PL)**
• **PIELKA, Izabela**
**42-274 Konopiska (PL)**
• **MALODOBRA-MAZUR, Malgorzata**
**51-354 Wroclaw (PL)**

(74) Representative: **Keltie LLP**
**No. 1 London Bridge**
**London SE1 9BA (GB)**

(56) References cited:
• **YUN HEE BAEK ET AL: "Development of a
reverse transcription-loop-mediated isothermal
amplification as a rapid early-detection method
for novel SARS-CoV-2", EMERGING MICROBES
& INFECTIONS, vol. 9, no. 1, 1 January 2020
(2020-01-01), pages 998 - 1007, XP055730864,
DOI: 10.1080/22221751.2020.1756698**
• **WEI E. HUANG ET AL: "RT-LAMP for rapid
diagnosis of coronavirus SARS-CoV-2",
MICROBIAL BIOTECHNOLOGY, vol. 13, no. 4, 25
April 2020 (2020-04-25), GB, pages 950 - 961,
XP055733461, ISSN: 1751-7915, DOI: 10.1111/
1751-7915.13586**
• **LIN YU ET AL: "Rapid colorimetric detection of
COVID-19 coronavirus using a reverse tran-
scriptional loop-mediated isothermal
amplification (RT-LAMP) diagnostic plat-form:
iLACO", MEDRXIV, 24 February 2020
(2020-02-24), XP055730128, Retrieved from the
Internet <URL:https://www.medrxiv.org/content/
10.1101/2020.02.20.20025874v1.full.pdf> DOI:
10.1101/2020.02.20.20025874**

Remarks:
The complete document including Reference
Table(s) and the Sequence Listing(s) can be
downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C12Q 1/6844, C12Q 2527/107**

**Description**

**FIELD OF INVENTION**

The present invention is concerned with diagnostic primers for viral detection, as well as kits, methods and uses associated with such primers. In particular, the present invention relates to sets of primers for the detection of Severe acute respiratory syndrome coronavirus 2; methods for its detection; and the utilization of such primer sets. The invention is intended to be useful in medical diagnostics.

**BACKGROUND OF THE INVENTION**

Severe acute respiratory syndrome coronavirus 2 belongs to broad family of viruses - coronaviruses (Coronaviridae). This virus is a positive-sense single-stranded RNA (+ssRNA). Severe acute respiratory syndrome coronavirus 2 (hereafter SARS-CoV-2) is a member of the subgenus Sarbecovirus (beta-CoV lineage B). It is unique among known betacoronaviruses in its incorporation of a polybasic cleavage site, a characteristic known to increase pathogenicity and transmissibility in other viruses.

SARS-CoV-2 is responsible for infectious, respiratory disease - coronavirus disease 2019 (COVID-19). This has spread globally during a matter of months, causing the 2019-2020 pandemic. Common symptoms include fever, cough, shortness of breath, but in severe cases the disease causes viral pneumonia and multi-organ failure. Currently, there is no vaccine, or any specific antiviral treatment for COVID-19. The standard method of diagnosis is Real-Time reverse transcription polymerase chain reaction (RT-PCR). The combination of chest CT scan, risk factors and symptoms also allows diagnosis. The standard method is time-consuming, while methods other than genetic identification lack the sensitivity and specificity to provide accurate diagnosis.

Therefore, there is a need to develop new, faster, more sensitive and/or more specific methods of SARS-CoV-2 diagnosis, which will significantly improve the current rate of diagnosis. Molecular methods based on the detection of specific fragments of the SARS-CoV-2 genome are among the most sensitive and specific methods for the pathogen diagnosis. The ideal solution would be a test developed for the needs of basic health care units, at the primary care where patients enter the diagnostic pathway, enabling rapid diagnosis (for example, in less than 20 minutes) and consequently targeted therapy prescription.

The Loop-mediated isothermal amplification (LAMP) method is a single-tube technique for the amplification of DNA/RNA and has been described, for example, in WO0028082A1 and WO0224902A1. A reverse transcription loop-mediated isothermal amplification method (RT-LAMP) has similarly been described, for example, in WO0177317A1, CN103146847B, US8389221B2, and CN102286637B.

The above-mentioned documents do not describe methods for the detection of Severe acute respiratory syndrome coronavirus 2.

Baek et al. reported a reverse transcription loop-mediated isothermal amplification (RT-LAMP) assay for the specific detection of SARS-CoV-2. The primer sets for RT-LAMP assay were designed to target the nucleocapsid gene of the viral RNA, and displayed a detection limit of $10^2$ RNA copies (YH Baek et al. (2020), "Development of a reverse transcription-loop-mediated isothermal amplification as a rapid early-detection method for novel SARS-CoV-2", Emerging Microbes & Infections, 9:1, 998-1007). Baek et al. reported an assay time of between 30 and 60 minutes. The assay of Baek et al. employed an indirect, colorimetric measurement to determine assay results.

Huang et al ("RT-LAMP for rapid diagnosis of coronavirus SARS-CoV-2",MICROBIAL BIOTECHNOLOGY, vol. 13, no. 4, 25 April 2020, pages 950-961) discloses the use of RT-LAMP for the rapid diagnosis of SARS-CoV-2. Four sets of LAMP primers (6 primers in each set) were designed targeting the viral RNA in the regions of orf1 ab, S gene and N gene.

Yu et al ("Rapid colorimetric detection of COVID-19 coronavirus using a reverse tran-scriptional loop-mediated isothermal amplification (RT-LAMP) diagnostic plat-form: iLACO",medRxiv, 24 February 2020) describes a RT-LAMP based method called iLACO (isothermal LAMP based method for COVID-19). RT-LAMP primers were designed selecting a region of the ORF1ab gene as target region.

Therefore, the need to provide a set of primers enabling the POC diagnostic method for SARS-CoV-2 (by the RT-LAMP method of nucleic acid amplification) intended for laboratory-independent healthcare facilities use still very much exists. The proposed RT-LAMP-based diagnostic primers allow identification of the SARS-CoV-2 virus with a very low limit of detection ($\geq 50\,GE/\mu l$) in a short time ($\leq 20\,min$). The proposed set of primers for intellectual protection solves the earlier discussed problem of real-time quantification obtained for low copies of the pathogen's RNA in rapid time.

**SUMMARY OF THE INVENTION**

In a first aspect, there is provided a primer set for detecting SARS-CoV-2 using a reverse transcription loop-mediated isothermal amplification (RT-LAMP) method, comprising:

i) at least a first F3 primer comprising a nucleotide sequence at least 90% identical to SEQ ID NO: 1;

ii) at least a first B3 primer comprising a nucleotide sequence at least 90% identical to SEQ ID NO: 2;

iii) at least a first F2 primer comprising a nucleotide sequence at least 90% identical to SEQ ID NO: 3;

iv) at least a first B2 primer comprising a nucleotide sequence at least 90% identical to SEQ ID NO: 4;

v) at least a first F1c primer comprising a nucleotide sequence at least 90% identical to SEQ ID NO: 5 or to SEQ ID NO: 8; and

vi) at least a first B1c primer comprising a nucleotide sequence at least 90% identical to SEQ ID NO: 6.

In a still further aspect, there is provided a kit for detecting Severe acute respiratory syndrome coronavirus 2 (SARS-CoV-2) in a sample, the kit comprising reagents for amplifying RNA in a sample using a RT-LAMP technique and a primer set according to any of the above aspects.

In a yet further aspect, there is provided a method of detecting SARS-CoV-2 in a sample, comprising amplifying by isothermal amplification at least a portion of the SARS-CoV-2 genome using a primer set according to any of the above aspects.

Disclosed but not part of the claimed invention is a set of primers for amplifying the nucleotide sequence of the N gene of the SARS-CoV-2 virus (SEQ ID NO: 14). The set is characterized by comprising the internal primers with the following nucleotide sequences a) and b), as well as the set of external primers comprising the following nucleotide sequences c) and d) and being specific for selected region of the Severe acute respiratory syndrome coronavirus 2 N gene:

a) 5' TCSYYTACTGCTGCCTGGAG 3' - (the nucleotide sequence SEQ ID NO: 5 or the sequence created by single nucleotide changes, substitution or deletion of a single nucleotide connected or not connected by TTTT bridge to the sequence 5' CGTTCCTCATCACGTAGTCG 3' - (the nucleotide sequence SEQ ID NO: 3 or the sequence created by single nucleotide changes, substitution or deletion of a single nucleotide);

b) 5' TCTCCTGCTAGAATGGCTGGCA 3' - (the nucleotide sequence SEQ ID NO: 6 or the sequence created by single nucleotide changes, substitution or deletion of a single nucleotide) connected or not connected by TTTT bridge to the sequence 5' TCTGTCAAGCAGCAGCAAAG 3' - (the nucleotide sequence SEQ ID NO: 4 or the sequence created by single nucleotide changes, substitution or deletion of a single nucleotide);

c) 5' CGGCAGTCAAGCCTCTTC 3' the nucleotide sequence SEQ ID NO: 1 or the sequence created by single nucleotide changes, substitution or deletion of a single nucleotide and

d) 5' TTGCTCTCAAGCTGGTTCAA 3' the nucleotide sequence SEQ ID NO: 2 or the sequence created by single nucleotide changes, substitution or deletion of a single nucleotide

Complementary with the above nucleotide sequence, disclosed but not part of the claimed invention is a loop primer sequence comprising the Severe acute respiratory syndrome coronavirus 2 N gene SEQ ID NO: 7: - 5' ATGG CGGTGATGCTGCTCT 3' or sequence resulting from single nucleotide exchanges, substitution or deletion of a single nucleotide.

Disclosed but not part of the claimed invention is a method for Severe acute respiratory syndrome coronavirus 2 detection, characterized by amplification of a selected region of the SARS-CoV-2 genome (that is, a fragment of the N gene) using the set of primers characterized in the first subject of the invention, wherein the method of detection employed is RT-LAMP (reverse transcription LAMP).

In a preferred embodiment, the nucleic acid amplification is carried out at 64°C-70°C, in particular 68°C, for 40 min.

Disclosed but not part of the claimed invention, the end-point type reaction is carried out at the condition mentioned above, and ended at 80°C, 5 min.

Disclosed but not part of the claimed invention is a method for the detection of SARS-CoV-2 infection, wherein the method uses RT-LAMP based nucleic acid amplification with a specific set of primers (first subject of the invention), detecting and amplifying a specific region of the SARS-CoV-2 genome (described in the second subject of the invention).

Disclosed but not part of the claimed invention is the reaction mix used for detection of infection caused by SARS-CoV-2, comprising the set of primers described in the first subject of the present invention.

Disclosed but not part of the claimed invention, the reaction mix for detection of infection caused by SARS-CoV-2 comprises 10 $\mu$l WarmStart LAMP KIT (RNA&DNA) (NEB).

Disclosed but not part of the claimed invention, the individual amplification primers as defined in the first object of the invention, wherein the primers have the following concentrations: 0.13 $\mu$M F3 (SEQ ID NO: 1), 0.13 $\mu$M B3 (SEQ ID NO: 2), 1.0 $\mu$M FIP (SEQ ID NO: 5 or 8 and 3), 1.0 $\mu$M BIP (SEQ ID NO: 6 and 4), 0.25 $\mu$M LoopB (SEQ ID NO: 7).

Additionally, the other components of the reaction mix include (at given concentrations): double-stranded DNA fluorescent marker - EvaGreen ≤1X or Fluorescent Dye ≤0,4 ul or Green Fluorescent Dye ≤0,8 ul or Syto-13 ≤16 $\mu$M or SYTO-82 ≤16 $\mu$M or other double-stranded DNA fluorescent dye at a concentration not inhibiting the amplification reaction.

**BRIEF DESCRIPTION OF THE FIGURES**

Figure 1 shows the sequence of the N gene of SARS-CoV-2, as well as the alignment of the primers discussed herein for amplification of that sequence.

Figure 2 shows the first step for virus detection, cDNA synthesis by reverse transcriptase.

Figure 3 shows the amplification steps of the RT-LAMP procedure.

Figures 4 and 5 show the sensitivity characteristics of the LAMP method of amplifying the N gene of SARS-CoV-2 by gel electrophoresis and by a trace of the real-time amplification of a series of standard dilutions.

Figure 6 illustrates the specificity of the method of the invention measured by setting a LAMP-based reaction (end-point) with DNA standards of numerous pathogens.

Figure 7 shows the alignment of multiple N sequences from several clinical samples.

Figure 8 shows the sequence of the S gene of SARS-CoV-2, as well as the alignment of the primers discussed herein for amplification of that sequence.

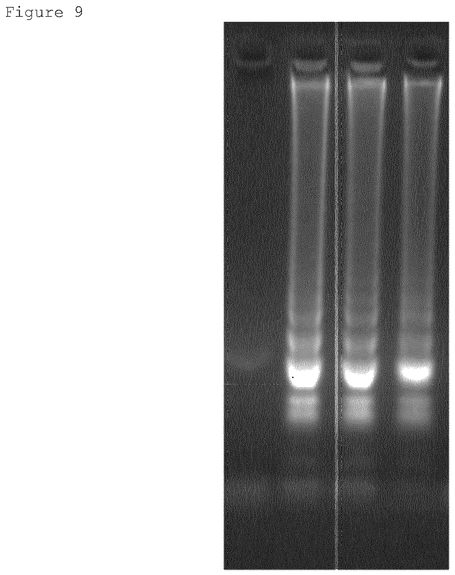

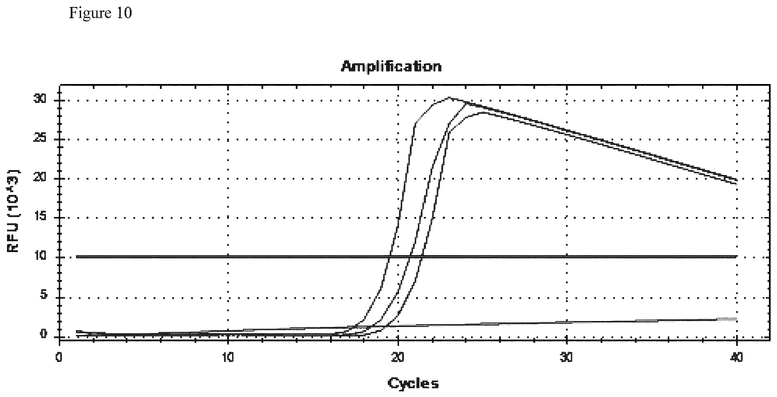

Figures 9 and 10 show the sensitivity characteristics of the LAMP method of amplifying the S gene of SARS-CoV-2 by gel electrophoresis and by a trace of the real-time amplification of a series of standard dilutions.

**DETAILED DESCRIPTION**

The advantage of the proposed set of primer as stated in the invention for the detection of SARS-CoV-2, as well as the method of detection of SARS-CoV-2 infection and the method of detection of amplification products specific to the SARS-CoV-2 genome, is its ability to be used in POC laboratory-independent medical diagnostic setting, detecting specific pathogen on a portable genetic analyzer (subject of another intellectual property filling) with an extremely high sensitivity and specificity. In turn, the use of fluorescent dye to detect the amplified genetic product(s) increases the sensitivity of the method, allows the detection limit to be reduced (to 50 genome equivalents (GE)/$\mu$l), and enables quantitative measurement of the SARS CoV-2 in the test sample.

**N gene detection**

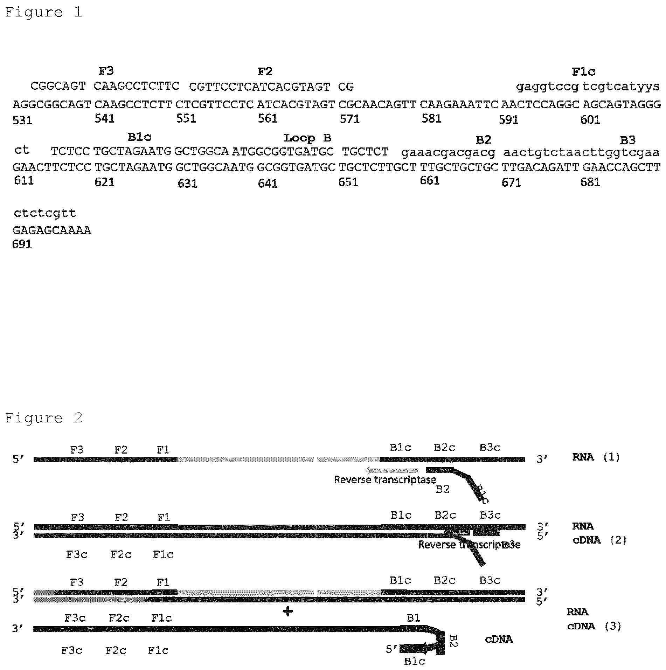

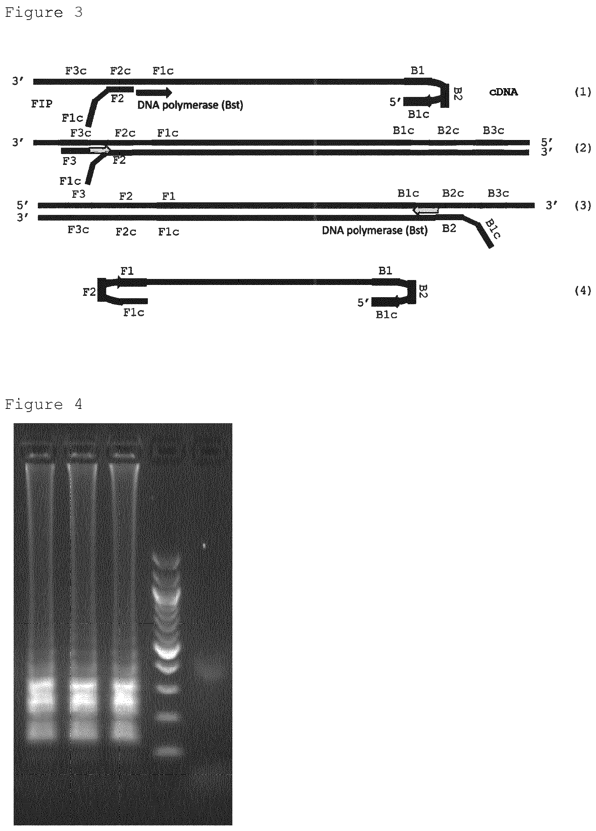

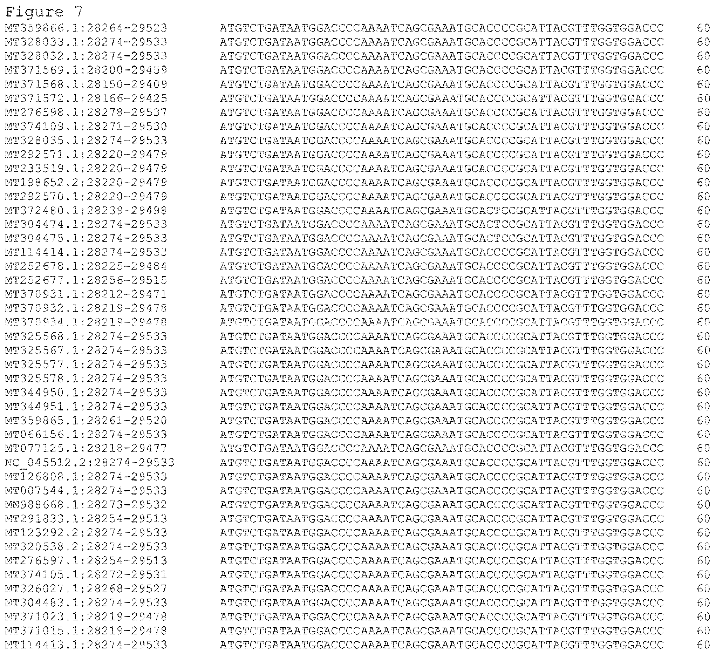

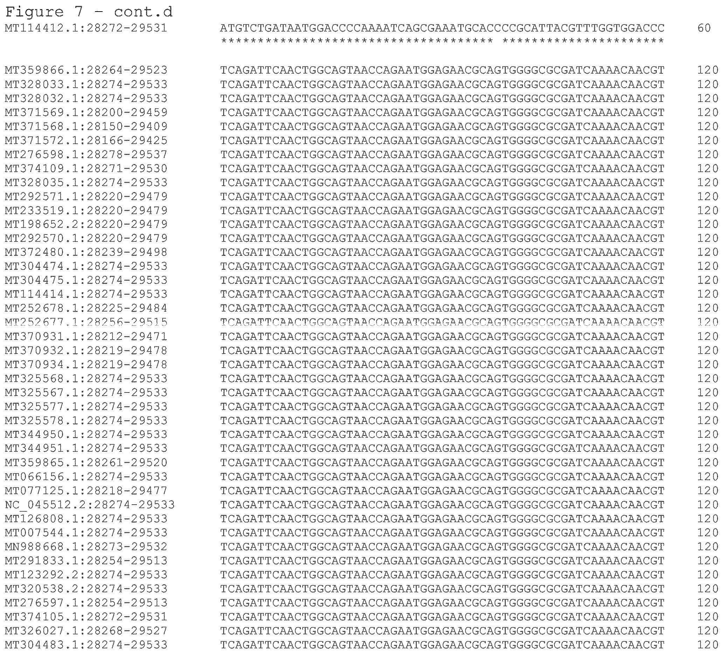

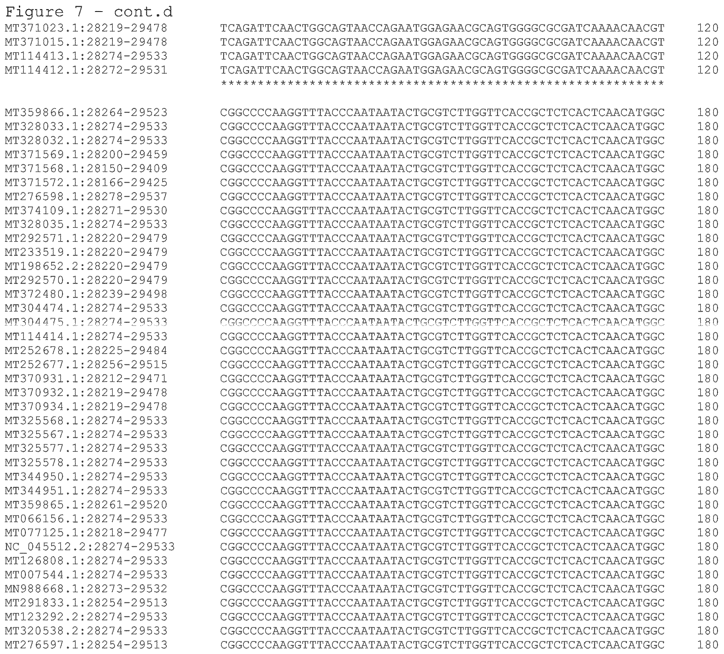

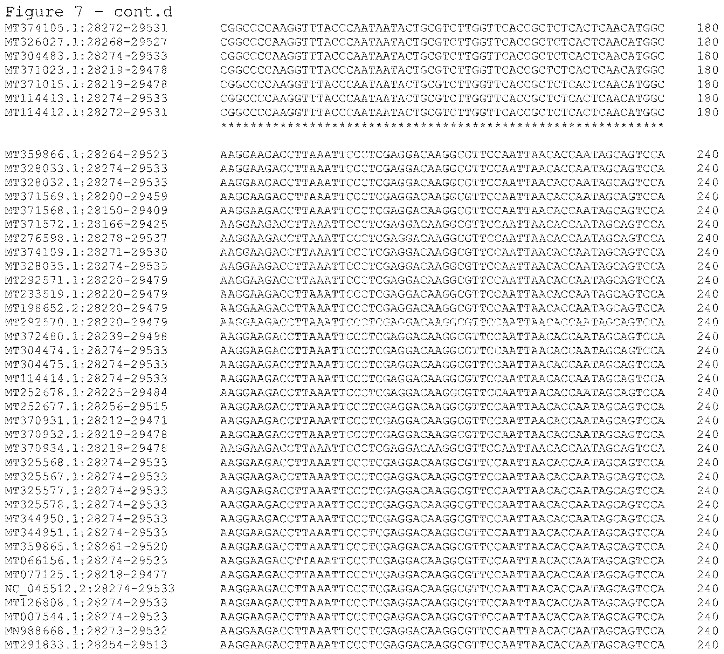

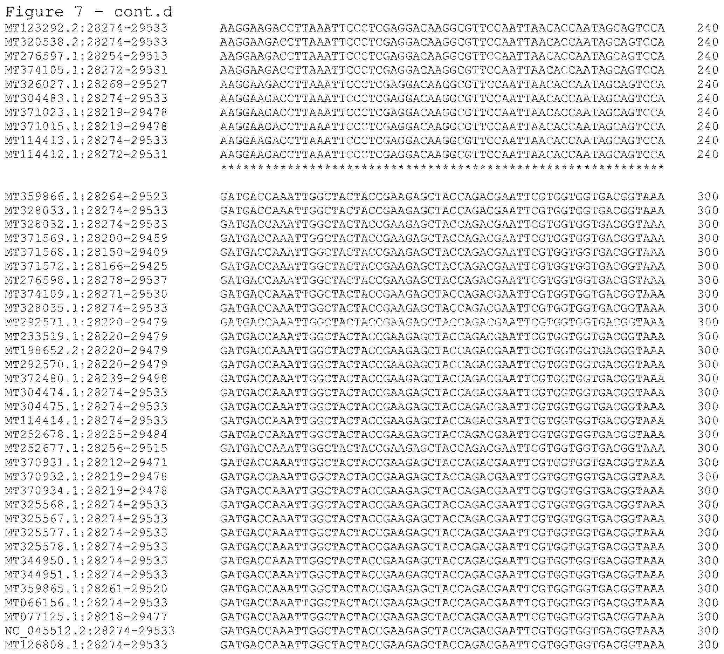

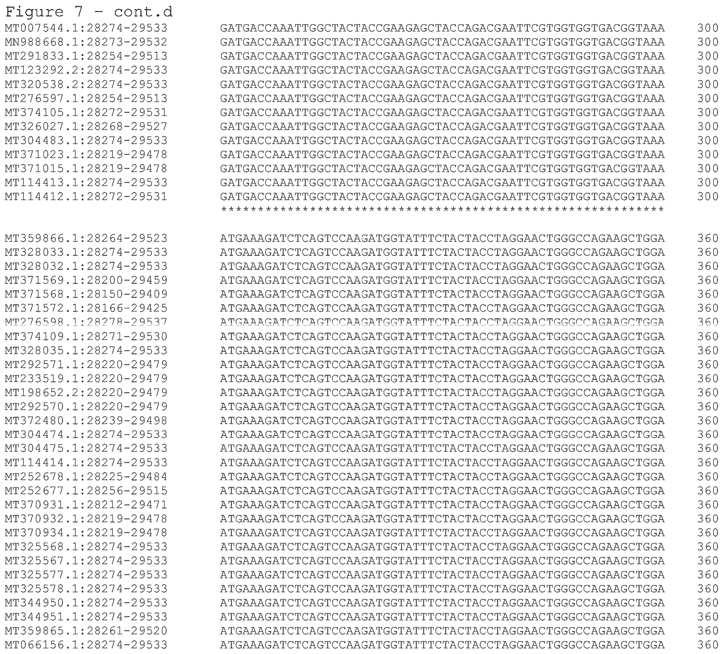

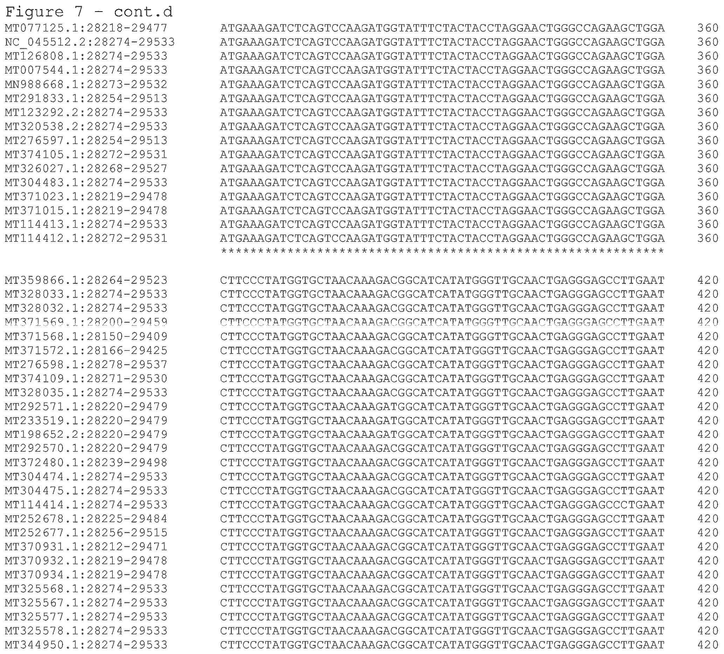

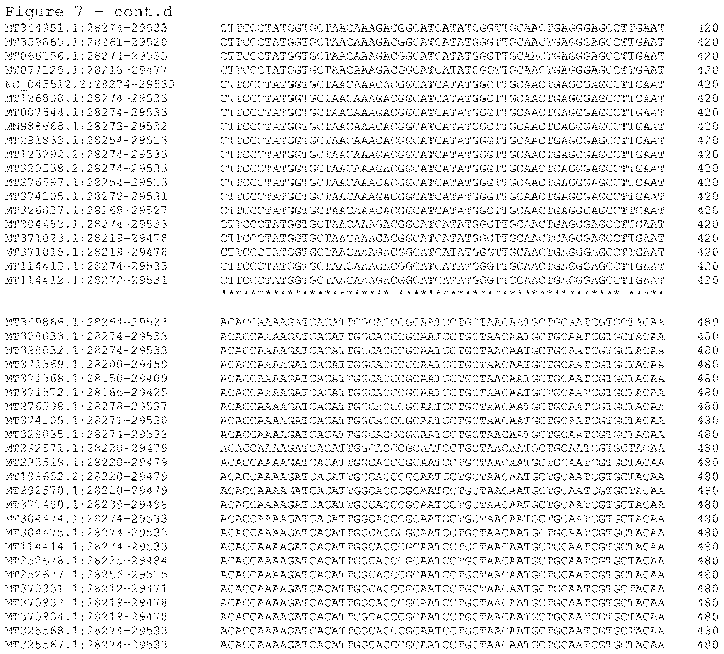

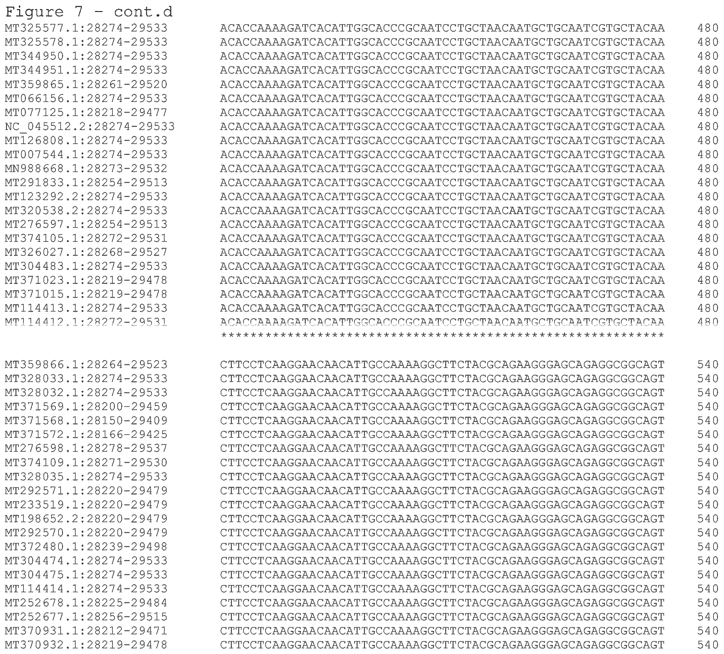

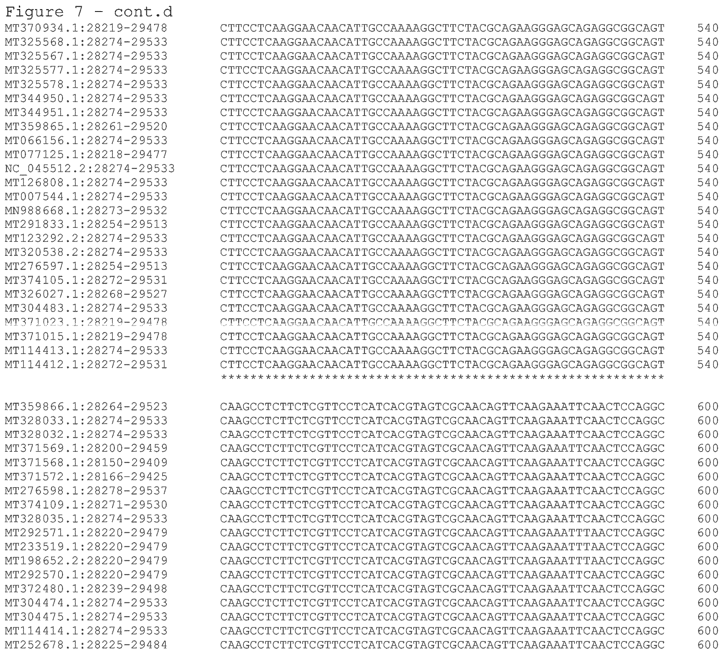

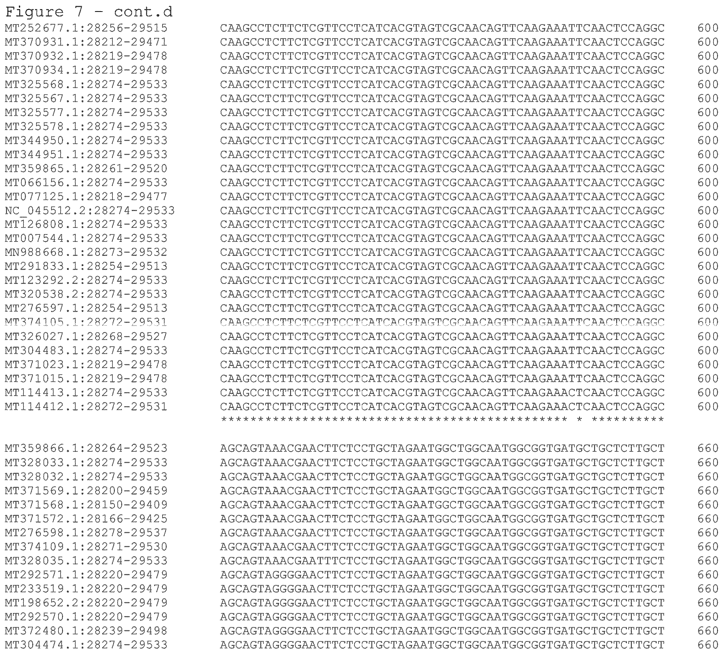

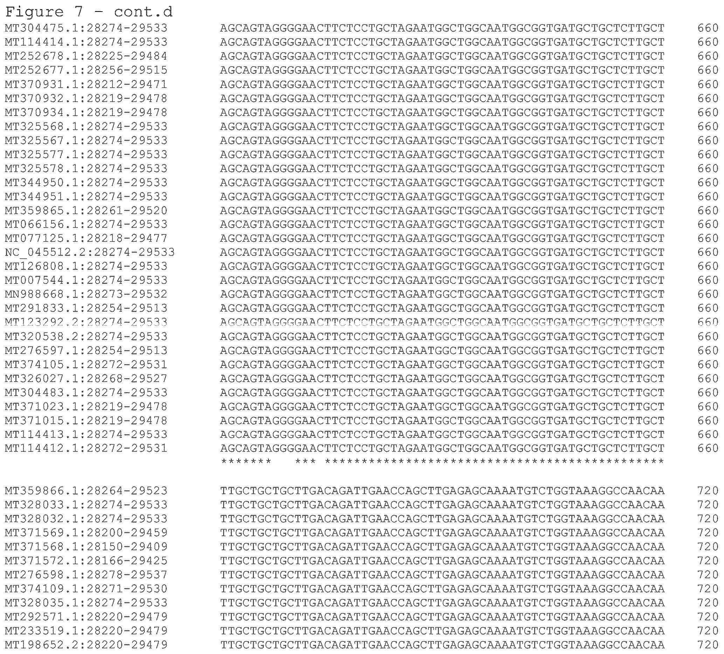

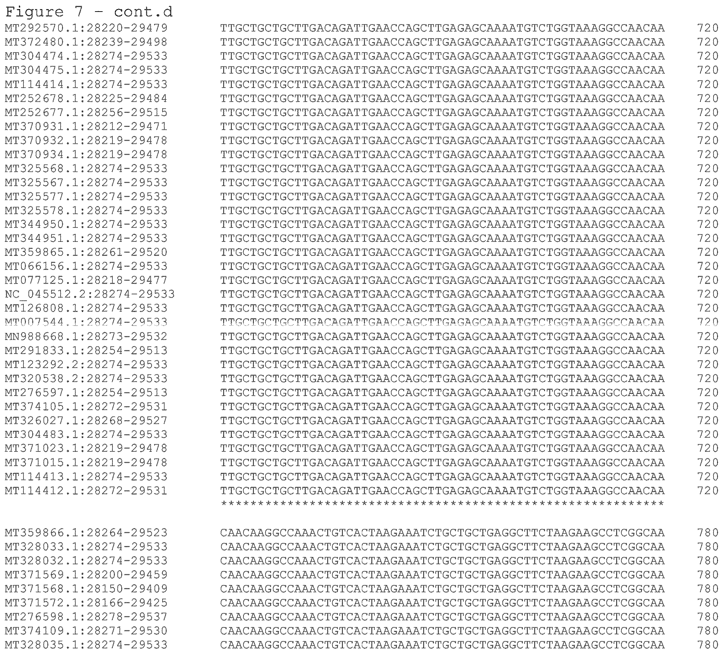

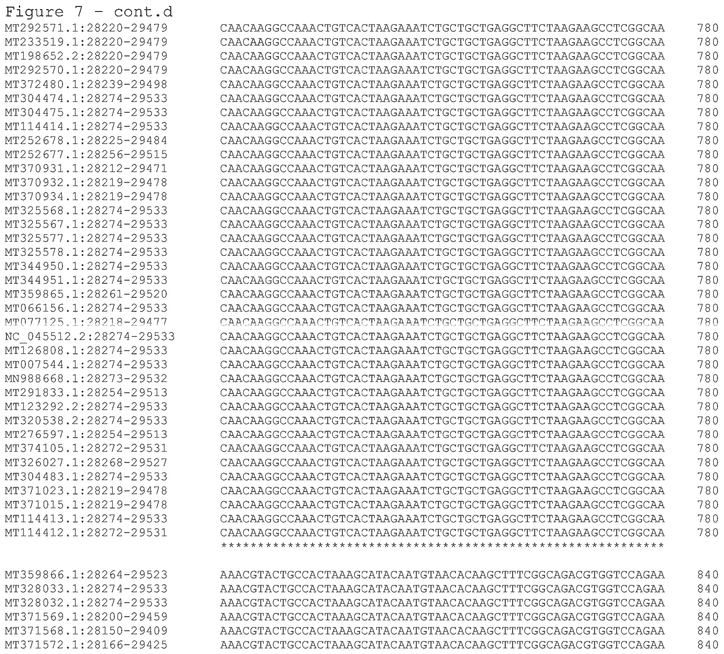

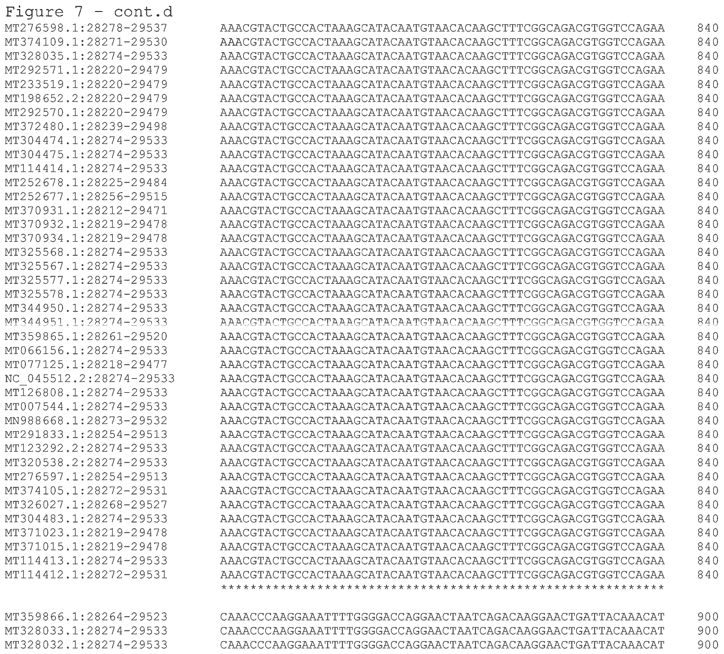

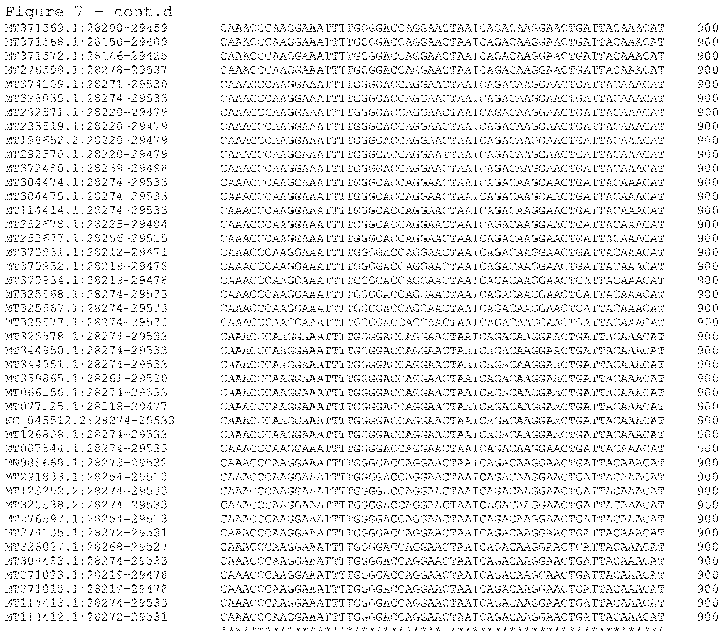

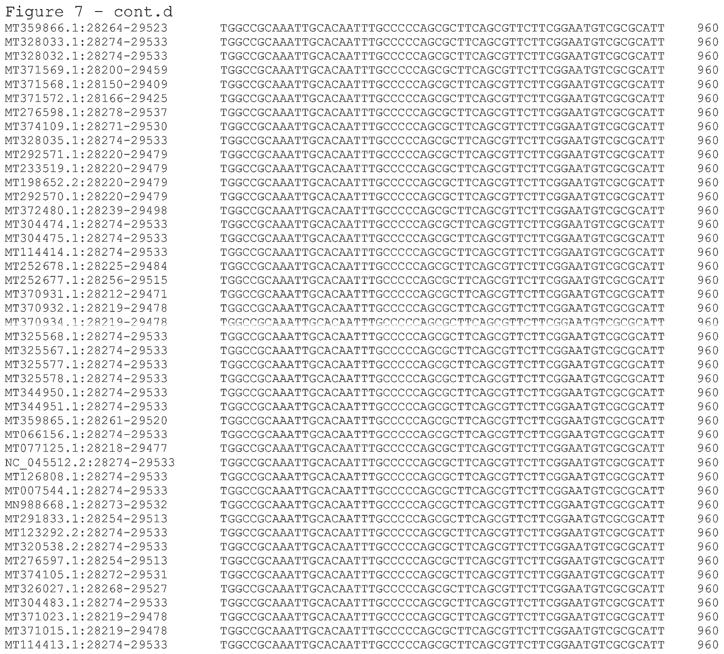

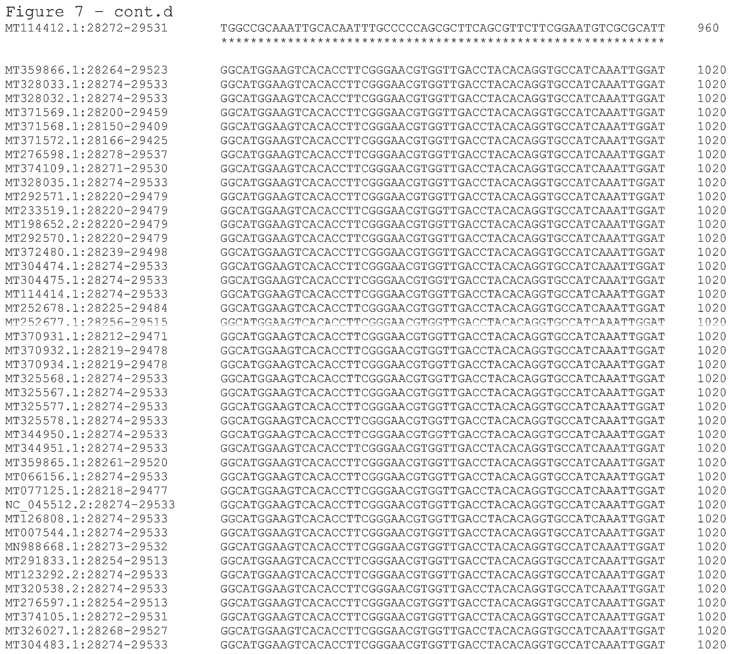

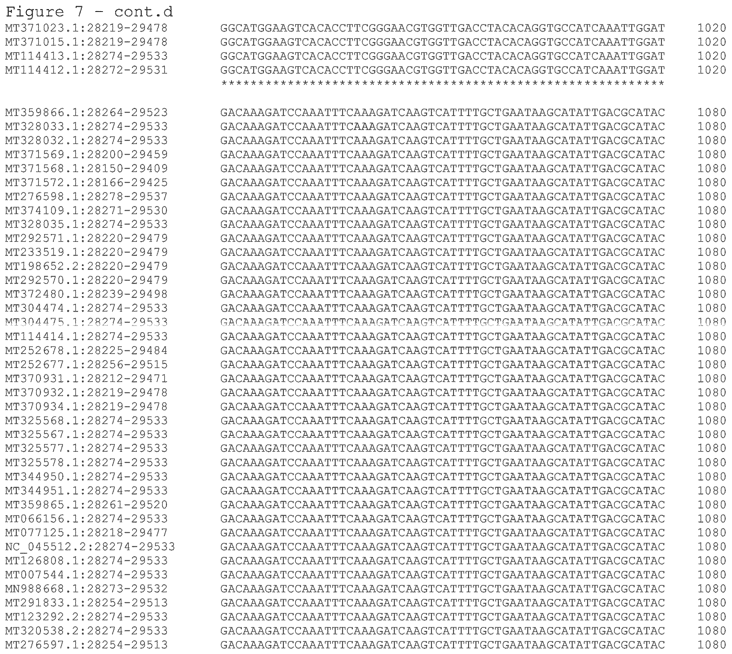

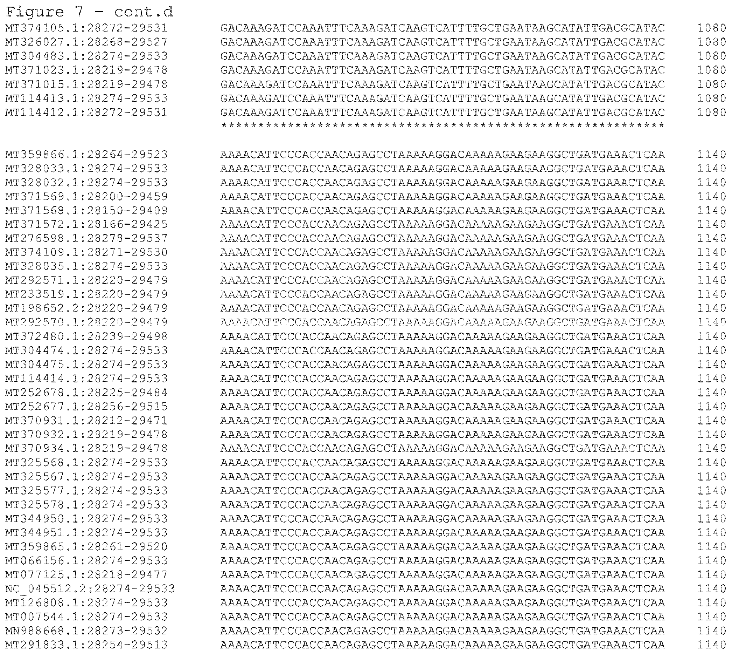

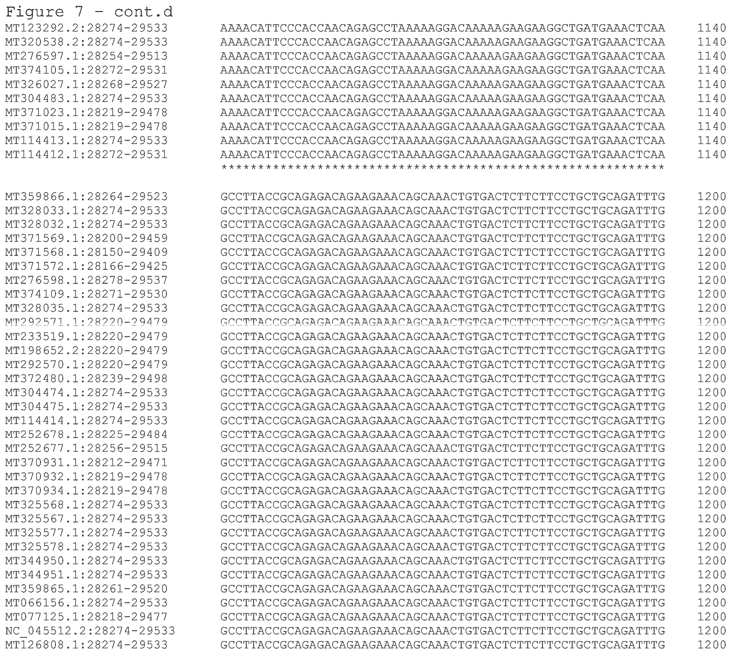

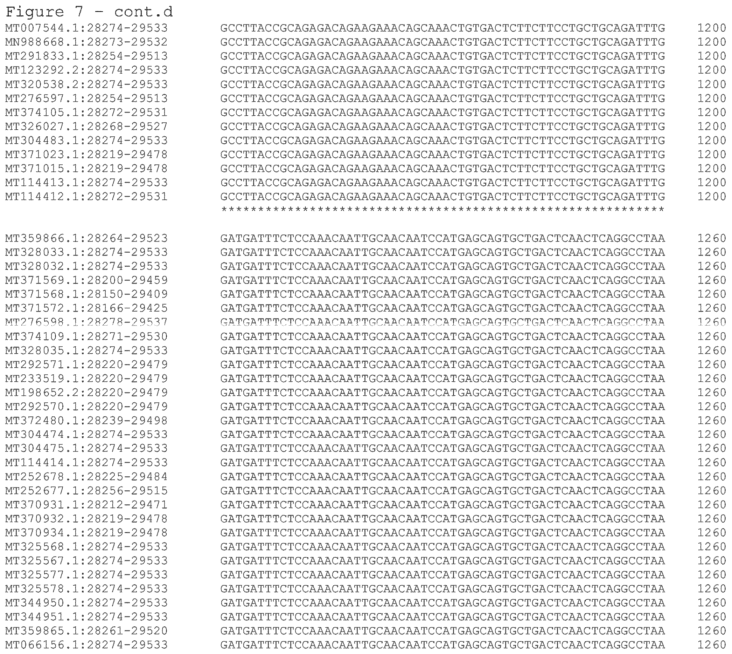

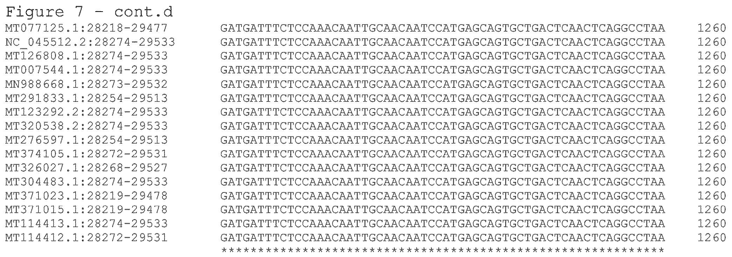

For the SARS-CoV-2 detection using RT-LAMP, five primers are used that are specific to detect a target sequence / region of the pathogen's nucleic acid sequence (the N gene of SARS-CoV-2, Fig. 1 and SEQ ID NO: 14; see also the alignment of multiple N sequences from several clinical samples in Fig. 7). The first step for virus detection is cDNA synthesis by reverse transcriptase (Fig. 2). The process is initialized by B2 primer that binds to the specific region of the N gene, and the reverse transcriptase continues the synthesis of the cDNA (1). Next, the outer B3 primer binds to the outside region of the BIP primer (2), and the new cDNA is synthesized by reverse transcriptase, concurrently releasing the single-stranded cDNA from the previous step (3). The amplification (Fig. 3) is initiated by one of the inner primers (F2) that creates with F1c the FIP primer (1). After the initialization step, the strand-displacement DNA polymerase (especially Bst polymerase) extends the FIP primer. This step ends with a synthesis of the first LAMP product, which is then displaced by the synthesis of the second product initiated by one of the outer primers F3 (2). The strand synthesized from the F3 primer together with the cDNA from the initial step of the process forms double stranded DNA (3). The product displaced by F3 primer contains complementary sequences on both ends which enable self-annealing and forming dumbbell-like structure, which is then subject to LAMP cycling amplification (4). As the amplification proceeds, the products that are synthesized create concatemer structures that serve for even more sites for amplification initiations. The result of multiple site initiation is the accumulation of double-stranded DNA of different length and structure (so-called cauliflower structures) that can be next detected using intercalating fluorescent dyes as the real-time approach.

It is not anticipated that sequences exactly identical or exactly complementary to those recited in the sequences disclosed herein must necessarily be used for the invention to be effective. For example, the primers used can comprise sequences which are at least 90%, at least 95%, at least 98%, at least 99% or 100% identical to the sequences disclosed herein, and/or which differ from those sequences as a result of single nucleotide exchanges, substitution or deletion of a single nucleotide. One or more of the primers can consist of the sequences disclosed herein. IUPAC sequence convention is used, such that, for example, 'S' stands for 'strong' bases (C, G) and 'Y' stands for pyrimidine bases (C, T). Figure 7 illustrates multiple sequence alignment of SARS-CoV-2 N gene from several clinical samples.

The F1c and F2 primers are generally connected to form one double primer, known as the Forward Inner Primer (FIP). Likewise, B1c and B2 primers are generally connected to form one double primer, known as the Backward Inner Primer (BIP). These primers may be directly connected, or may be connected by a nucleotide bridge. Such a bridge may be between one and ten nucleotides in length. In particular, it has been observed that certain sequences, such as a TTTT sequence, can help in the formation of loop structures. Accordingly, in some embodiment the bridge within the FIP and/or BIP double primers may be a TTTT nucleotide sequence.

Exemplary implementations of the invention and supporting data are shown in the drawings, in which Fig. 4 shows the sensitivity characteristics of the LAMP method, where a specific signal was obtained with the RNA standard (Twist Synthetic SARS-CoV-2 RNA Control 1; Twist Bioscience) in the range of 1000 - 50 copies / μl, while the NTC reaction was negative; Fig. 1: Line 1: 50 SARS-CoV-2 copies; line 2: 100 SARS-CoV-2 copies; line 3: 1000 SARS-CoV-2 copies; line 4: DNA molecular-weight size marker (Quick-Load® Purple 100 bp DNA Ladder, NewEngland Biolabs); line 5: NTC.

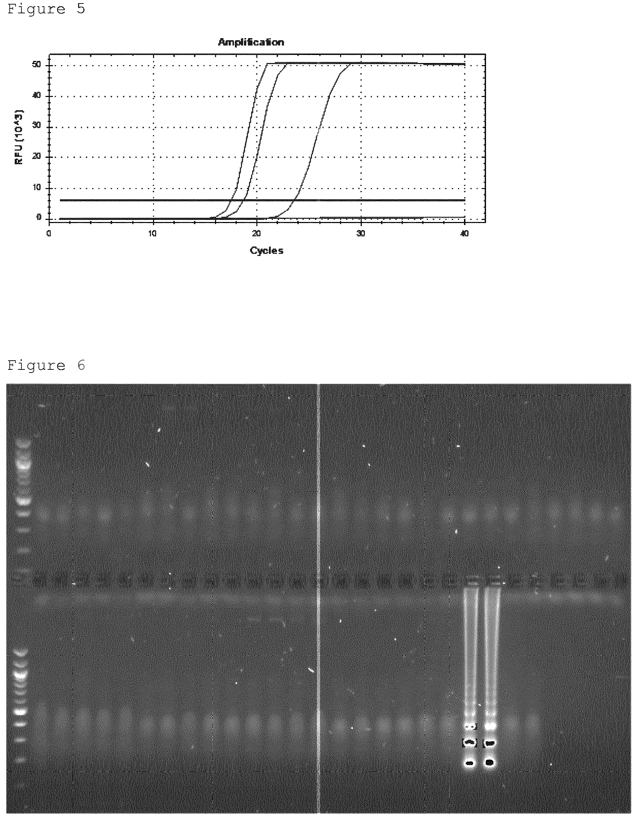

Fig. 5 illustrates the sensitivity of the LAMP method of the invention measured by tracking a trace of the real-time amplification of a series of the standard dilutions (Twist Synthetic SARS-CoV-2 RNA Control 1 (Twist Bioscience) in the range of 1,000 - 50 copies / μl of the DNA standard tested.

The results of SARS-CoV-2 detected in real-time are presented in Table 1, giving indication of the minimum time necessary to detect the fluorescence signal.

Fig. 6 illustrates the specificity of the method of the invention measured by setting a LAMP-based reaction (end-point) with DNA standards of numerous pathogens, often present in biological material as the physiological pathogens, pathogens which may appear as the result of co-infection, or which have similar genomic sequences.

Line 1: DNA molecular-weight marker (Quick-Load® Purple 100 bp DNA Ladder, NewEngland Biolabs); Lines 2 and 3: Mycoplasma genitalium; Lines 4 and 5: Streptococcus pyogenes; Lines 6 and 7: Enterococcus faecalis; Lines 8 and 9: Moraxella catarrhalis; Lines 10 and 11: Legionella pneumophila; Lines 12 and 13: Escherichia coli; Lines 14 and 15: Candida albicans; Lines 16 and 17: Mycoplasma pneumoniae; Lines 18 and 19: Klebsiella pneumoniae; Lines 20 and 21: Staphylococcus aureus methicilin sensitive (MSSA); Lines 22 and 23: Enterococcus faecium; Lines 24 and 25: Acinetobacter baumannii; Lines 26 and 27: Mycoplasma hominis; Line 28 and 29: Ureoplasma urealyticum; Lines 30 and 31: Haemophilus influenzae; Lines 32 and 33: Human genomic DNA; Lines 34 and 35: Bordetella pertussis; Lines 36 and 37: Staphylococcus aureus methicilin resistant (MRSA); Lines 38 and 39: Pseudomonas aeruginosa; Lines 40 and 41: Listeria monocytogenes ; Line 42 and 43: Haemophilus ducreyi; Lines 44 and 45: Campylobacter jejuni; Lines 46 and 47: Chlamydiophila pneumoniae; Lines 48 and 49: Twist Synthetic SARS-CoV-2 RNA Control 2 (Twist Bioscience); Lines 50 and 51: NTC (no-template control).

## S gene detection

In addition to the sequences and methods discussed above to amplify the N gene of the SARS-CoV-2 virus, there is also provided herewith a set of primers for amplifying the nucleotide sequence of the S gene of the SARS-CoV-2 virus (SEQ ID NO: 27). The set is characterized by comprising the internal primers with the following nucleotide sequences a) and b), as well as the set of external primers comprising the following nucleotide sequences c) and d) and being specific for selected region of the Severe acute respiratory syndrome coronavirus 2 S gene:

a) 5' CATGGAACCAAGTAACATTGGAAAA 3' - (the nucleotide sequence SEQ ID NO: 19 or the sequence created by single nucleotide changes, substitution or deletion of a single nucleotide connected or not connected by TTTT bridge to the sequence 5' TTTTCAGATCCTCAGTTTTACATTC 3' - (the nucleotide sequence SEQ ID NO: 17 or the sequence created by single nucleotide changes, substitution or deletion of a single nucleotide);

b) 5' CTCTGGGACCAATGGTACTAAGAG 3' - (the nucleotide sequence SEQ ID NO: 20 or the sequence created by single nucleotide changes, substitution or deletion of a single nucleotide) connected or not connected by TTTT bridge to the sequence 5' GACTTCTCAGTGGAAGCA 3' - (the nucleotide sequence SEQ ID NO: 18 or the sequence created by single nucleotide changes, substitution or deletion of a single nucleotide);

c) 5' GGTGTTTATTACCCTGACAAAG 3' the nucleotide sequence SEQ ID NO: 15 or the sequence created by single nucleotide changes, substitution or deletion of a single nucleotide and

d) 5' GTACCAAAAATCCAGCCTC 3' the nucleotide sequence SEQ ID NO: 16 or the sequence created by single nucleotide changes, substitution or deletion of a single nucleotide

Complementary with the above nucleotide sequence, the set of primers can further comprise the below loop primer sequences comprising sequence complementary to the Severe acute respiratory syndrome coronavirus 2 S gene SEQ ID NO: 21: - 5' GAAAGGTAAGAACAAGTCCTGAGT 3' and sequence identical to the Severe acute respiratory syndrome coronavirus 2 S gene SEQ ID NO: 22: 5 CTGTCCTACCATTTAATGATGGTGT 3' or sequences resulting from single nucleotide exchanges, substitution or deletion of a single nucleotide.

Aside from the use of different primers as discussed, and the presence of an additional loop primer sequence (that is, SEQ ID NO: 21), this set of primers for amplifying the nucleotide sequence of the S gene of the SARS-CoV-2 virus can be used in the same way as described above for the primers corresponding to the N gene, and/or as further described below.

Accordingly, further provided is a method for Severe acute respiratory syndrome coronavirus 2 detection, characterized by amplification of a selected region of the SARS-CoV-2 genome (that is, a fragment of the S gene) using the set of primers characterized above, wherein the method of detection employed is RT-LAMP (reverse transcription LAMP).

In a preferred embodiment, the nucleic acid amplification is carried out at 60°C, for 40 min.

In another preferred embodiment of the invention, the end-point type reaction is carried out at the condition mentioned above, and ended at 80°C, 5 min.

## EXAMPLES

### Example 1 - N gene - Primer sequences

The specific sequences of oligonucleotides used to detect Severe acute respiratory syndrome coronavirus 2 genetic material (the N gene) utilizing LAMP technology are shown on Fig. 1, Fig. 2 and Fig.3 and characterized below:

1. Oligonucleotide sequence SARS-CoV-2 NF3: 5' CGGCAGTCAAGCCTCTTC 3' (SEQ ID NO: 1) is a sequence identical to the Severe acute respiratory syndrome coronavirus 2 N gene (strand 5'-3'), which from the 3' end is adjacent to the F2 primer.

2. Oligonucleotide sequence SARS-CoV-2 NB3: 5' TTGCTCTCAAGCTGGTTCAA 3' (SEQ ID NO: 2) is a complementary fragment of Severe acute respiratory syndrome coronavirus 2 N gene (strand 5'-3') separated by 127 nucleotides from the 3' end of oligonucleotide 1.

3. Oligonucleotide sequence SARS-CoV-2 NF2: 5' CGTTCCTCATCACGTAGTCG 3' (SEQ ID NO: 3) is a sequence identical to the Severe acute respiratory syndrome coronavirus 2 N gene (strand 5'-3') sequence separated by 1 nucleotide from the 3'end of oligonucleotide 1.

4. Oligonucleotide sequence SARS-CoV-2 NB2: 5' TCTGTCAAGCAGCAGCAAAG 3' (SEQ ID NO: 4) is a complementary fragment of Severe acute respiratory syndrome coronavirus 2 N gene (strand 5'-3') separated by 107 nucleotides from the 3' end of oligonucleotide 1.

5. Oligonucleotide sequence SARS-CoV-2 NF1c: 5' TCSYYTACTGCTGCCTGGAG 3' (SEQ ID NO: 5) is a complementary fragment of Severe acute respiratory syndrome coronavirus 2 N gene (strand 5'-3') separated by 41 nucleotides from the 3' end of oligonucleotide 1. In a particular embodiment, the sequence SARS-CoV-2 NF1': 5' TCCCCTACTGCTGCCTGGAG 3' (SEQ ID NO: 8) can be used.

6. Oligonucleotide sequence SARS-CoV-2 NB1c: 5' TCTCCTGCTAGAATGGCTGGCA 3' (SEQ ID NO: 6) is a sequence identical to the Severe acute respiratory syndrome coronavirus 2 N gene separated by 64 nucleotides from the 3' end of oligonucleotide 1.

7. Oligonucleotide sequence SARS-CoV-2 NLoopB: 5' ATGGCGGTGATGCTGCTCT 3' (SEQ ID NO: 7).

The F1c and F2 oligonucleotide sequences are preferably linked by a TTTT bridge and used in the form of a Forward Inner Primer (FIP); for example: 5' TCSYYTACTGCTGCCTGGAGNNNNCGTTCCTCATCACGTAGTCG 3' (SEQ ID NO: 9); 5' TCSYYTACTGCTGCCTGGAGTTTTCGTTCCTCATCACGTAGTCG 3' (SEQ ID NO: 10); or most particularly 5' TCCCCTACTGCTGCCTGGAGTTTTCGTTCCTCATCACGTAGTCG 3' (SEQ ID NO: 11) - where N is any nucleotide.

The B1c and B2 oligonucleotide sequences are preferably linked via a TTTT bridge and are used in the form of a Backward Inner Primer (BIP); for example: 5' TCTCCTGCTAGAATGGCTGGCANNNNTCTGTCAAGCAGCAGCAAAG 3' (SEQ ID NO: 12); or most particularly 5' TCTCCTGCTAGAATGGCTGGCATTTTTCTGTCAAGCAGCAGCAAAG 3' (SEQ ID NO: 13) - where N is any nucleotide.

### Example 2

Severe acute respiratory syndrome coronavirus 2 N gene amplification method utilizing LAMP technology with oligonucleotides characterized in Example 1, with the following composition of the reaction mixture:

10,0 µl WarmStart LAMP KIT (RNA&DNA)
0,13 µM F3

0,13 μM B3
1,00 μM FIP
1,00 μM BIP
0,25 μM LoopB

Fluorescent dye interacting with double-stranded DNA:

EvaGreen ≤1X or Fluorescent dye 50X (New England Biolabs) at 0,4 ul, or Green Fluorescent Dye (Lucigen) at ≤0,8 ul, or
Syto-13 ≤16 μM or SYTO-82 ≤16 μM, or other fluorescent dye interacting with double-stranded DNA at a concentration that does not inhibit the amplification reaction.

DNA template ≥ 50 copies / reaction

### Example 3

Severe acute respiratory syndrome coronavirus 2 N gene amplification method using oligonucleotides characterized in Example 1 and Example 2 in LAMP technology with the composition of the reaction mixture characterized in Example 3 with the following temperature profile:

1) 68 °C, 40 min
2) preferably at 80 °C end-point reactions, 5 min. Such end point reactions aim to denature the polymerase in order to stop amplification.

### Example 4

The method of amplification and detection of the Severe acute respiratory syndrome coronavirus 2 N gene using oligonucleotides characterized in Example 1 in LAMP technology with the composition of the reaction mixture characterized in Example 2 with the temperature profile characterized in Example 3 and the detection method described below.
Fluorescent dye has the ability to interact with double-stranded DNA present in the reaction mixture in the amount of 1 μL EvaGreen 20X - 0,8 μl or concentration ≤1X; ≤16 μM for Green Fluorescent Dye (Lucigen), SYTO-13 and SYTO-82 respectively; measurement affected include the Real-Time and / or end-point reactions. Excitation wavelength in the range similar to FAM dye include: 490-500 nm (optimally 494 nm) for EvaGreen dyes, Fluorescent dye 50X (New England Biolabs), Green Fluorescent Dye (Lucigen); 535 nm (optimally 541 nm) for SYTO-13 and SYTO-82. Emission wavelength in the range 509-530 nm (optimally 518 nm) for EvaGreen dyes and Green Fluorescent Dye (Lucigen); 556 nm (optimally 560 nm) for SYTO-13 and SYTO-82 - detection method. Changes in detection were recorded 1 minute from the start of the reaction for Severe acute respiratory syndrome coronavirus 2 and negative control.

### Example 5 Method sensitivity

Sensitivity was determined by setting-up RealTime-RT-LAMP for a series of dilutions of the Twist Synthetic SARS-CoV-2 RNA Control 1 (Twist Bioscience); with a minimum amount of 50 copies of viral RNA in the reaction mixture, where product was measured in a real time - Figure 2 (RealTime-RT-LAMP for a series of dilutions).
The time after which it is possible to detect the emitted fluorescence corresponding to a specific SARS-CoV-2 genome fragment for given copy number is shown in Table 1.
Conclusion: the characterized primers enable the detection of Severe acute respiratory syndrome coronavirus 2 by detecting a fragment of the N gene with a minimum of 50 copies / reaction mixture.

**Table 1:** Time needed to detect fluorescence for individual dilutions of the Severe acute respiratory syndrome coronavirus 2 RNA standard (Twist Synthetic SARS-CoV-2 RNA Control 2, Twist Bioscience).

| Sample | Time of exceeding the fluorescence baseline [min] |
|---|---|
| SARS-CoV-2 NTC | Undetermined |
| SARS-CoV-2 50 copies | 23,57 |
| SARS-CoV-2 100 copies | 18,67 |

(continued)

| Sample | Time of exceeding the fluorescence baseline [min] |
|---|---|
| SARS-CoV-2 1 000 copies | 17,49 |

The superiority of the amplification method and set of primers (oligonucleotides) characterized in this patent over other tests based on the Real Time-RT-LAMP technology lies in its high sensitivity shown in Figure 1 and the dramatic reduction of the analysis time shown in Figure 2 and Table 1 when compared to standard RT-PCR techniques.

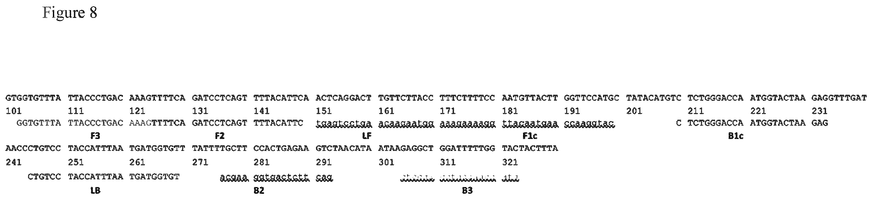

**Example 6** - **S gene** - **Primer sequences** The specific sequences of oligonucleotides used to detect Severe acute respiratory syndrome coronavirus 2 genetic material utilizing LAMP technology are shown in Fig. 8 and characterized below:

1. Oligonucleotide sequence SARS-CoV-2 SF3: 5' GGTGTTTATTACCCTGACAAAG3' (SEQ ID NO: 15) is a sequence identical to the Severe acute respiratory syndrome coronavirus 2 S gene (strand 5'-3'), which from the 3' end is adjacent to the F2 primer.

2. Oligonucleotide sequence SARS-CoV-2 SB3: 5' GTACCAAAAATCCAGCCTC3' (SEQ ID NO: 16) is a complementary fragment of Severe acute respiratory syndrome coronavirus 2 S gene (strand 5'-3') separated by 180 nucleotides from the 3' end of oligonucleotide 1.

3. Oligonucleotide sequence SARS-CoV-2 SF2: 5' TTTTCAGATCCTCAGTTTTACATTC 3' (SEQ ID NO: 17) is a sequence identical to the Severe acute respiratory syndrome coronavirus 2 S gene (strand 5'-3') sequence immediately adjacent to the 3 'end of oligonucleotide 1.

4. Oligonucleotide sequence SARS-CoV-2 SB2: 5' GACTTCTCAGTGGAAGCA 3' (SEQ ID NO: 18) is a complementary fragment of Severe acute respiratory syndrome coronavirus 2 S gene (strand 5'-3') separated by 151 nucleotides from the 3' end of oligonucleotide 1.

5. Oligonucleotide sequence SARS-CoV-2 SF1c: 5' CATGGAACCAAGTAACATTGGAAAA 3' (SEQ ID NO: 19) is a complementary fragment of Severe acute respiratory syndrome coronavirus 2 S gene (strand 5'-3') separated by 50 nucleotides from the 3' end of oligonucleotide 1.

6. Oligonucleotide sequence SARS-CoV-2 SB1c: 5' CTCTGGGACCAATGGTACTAAGAG 3' (SEQ ID NO: 20) is a sequence identical to the Severe acute respiratory syndrome coronavirus 2 S gene separated by 85 nucleotides from the 3' end of oligonucleotide 1.

7. Oligonucleotide sequence SARS-CoV-2 SLoopF: 5' GAAAGGTAAGAACAAGTCCTGAGT 3' (SEQ ID NO: 21).

8. Oligonucleotide sequence SARS-CoV-2 SLoopB: 5' CTGTCCTACCATTTAATGATGGTGT 3' (SEQ ID NO: 22).

The F1c and F2 oligonucleotide sequences are preferably linked by a TTTT bridge and used in the form of a Forward Inner Primer (FIP); for example: 5' CATGGAACCAAGTAACATTGGAAAANNNNTTTTCAGATCCTCAGTTTTACATTC 3' (SEQ ID NO: 23); or most particularly 5' CATGGAACCAAGTAACATTGGAAAATTTTTTTTCAGATCCTCAGTTTTACATTC 3' (SEQ ID NO: 24) - where N is any nucleotide.

The B1c and B2 oligonucleotide sequences are preferably linked via a TTTT bridge and are used in the form of a Backward Inner Primer (BIP); for example: 5' CTCTGGGACCAATGGTACTAAGAGNNNNGACTTCTCAGTGGAAGCA 3' (SEQ ID NO: 25); or most particularly 5' CTCTGGGACCAATGGTACTAAGAGTTTTGACTTCTCAGTGGAAGCA 3' (SEQ ID NO: 26) - where N is any nucleotide.

**Example 7**

Severe acute respiratory syndrome coronavirus 2 S gene amplification method utilizing LAMP technology with oligonucleotides characterized in Example 6, with the following composition of the reaction mixture:

10.0 µl WarmStart LAMP KIT (RNA&DNA)
0.13 µM F3
0.13 µM B3
1.06 µM FIP
1.06 µM BIP
0.27 µM LoopF
0.27 µM LoopB

Fluorescent dye interacting with double-stranded DNA:

EvaGreen ≤1X or Fluorescent dye 50X (New England Biolabs) at 0,4 ul, or Green Fluorescent Dye (Lucigen) at ≤0,8 ul, or

Syto-13 ≤16 μM or SYTO-82 ≤16 μM, or other fluorescent dye interacting with double-stranded DNA at a concentration that does not inhibit the amplification reaction.

DNA template ≥ 25 copies / reaction

**Example 8**

Severe acute respiratory syndrome coronavirus 2 S gene amplification method using oligonucleotides characterized in Example 6 in LAMP technology with the composition of the reaction mixture characterized in Example 7 with the following temperature profile:

1) 60 °C, 40 min
2) preferably at 80 °C end-point reactions, 5 min. Such end point reactions aim to denature the polymerase in order to stop amplification.

**Example 9**

The method of amplification and detection of the Severe acute respiratory syndrome coronavirus 2 S gene using oligonucleotides characterized in Example 6 in LAMP technology with the composition of the reaction mixture characterized in Example 5 with the temperature profile characterized in Example 8 and the detection method described below.
Fluorescent dye has the ability to interact with double-stranded DNA present in the reaction mixture as described in Example 4 above.

**Example 10 Method sensitivity**

Sensitivity was determined by setting-up RealTime-RT-LAMP for a series of dilutions of the Twist Synthetic SARS-CoV-2 RNA Control 2 (Twist Bioscience); with a minimum amount of 25 copies of viral RNA in the reaction mixture, where product was measured in a real time - Figure 2 (RealTime-RT-LAMP for a series of dilutions).
The time after which it is possible to detect the emitted fluorescence corresponding to a specific SARS-CoV-2 genome fragment for given copy number is shown in Table 2.
Conclusion: the characterized primers enable the detection of Severe acute respiratory syndrome coronavirus 2 by detecting a fragment of the S gene with a minimum of 25 copies / reaction mixture.

**Table 2:** Time needed to detect fluorescence for individual dilutions of the Severe acute respiratory syndrome coronavirus 2 RNA standard (Twist Synthetic SARS-CoV-2 RNA Control 2, Twist Bioscience).

| Sample | Time of exceeding the fluorescence baseline [min] |
|---|---|
| SARS-CoV-2 NTC | Undetermined |
| SARS-CoV-2 25 copies | 19.48 |
| SARS-CoV-2 50 copies | 20.68 |
| SARS-CoV-2 100 copies | 21.38 |

The superiority of the amplification method and set of primers (oligonucleotides) characterized in this patent over other tests based on the Real Time-RT-LAMP technology lies in its high sensitivity shown in Figure 8 and the dramatic reduction of the analysis time shown in Table 2 when compared to standard RT-PCR techniques.

**Claims**

1.  A primer set for detecting SARS-CoV-2 using a reverse transcription loop- mediated isothermal amplification (RT-LAMP) method, comprising:

    i) at least a first F3 primer comprising a nucleotide sequence at least 90% identical to SEQ ID NO: 1;
    ii) at least a first B3 primer comprising a nucleotide sequence at least 90% identical to SEQ ID NO: 2;
    iii) at least a first F2 primer comprising a nucleotide sequence at least 90% identical to SEQ ID NO: 3;

iv) at least a first B2 primer comprising a nucleotide sequence at least 90% identical to SEQ ID NO: 4;
v) at least a first F1c primer comprising a nucleotide sequence at least 90% identical to SEQ ID NO: 5 or to SEQ ID NO: 8; and
vi) at least a first B1c primer comprising a nucleotide sequence at least 90% identical to SEQ ID NO: 6.

2. The primer set according to claim 1, wherein:

i) the first F3 primer comprises a nucleotide sequence at least 95%, or at least 98%, identical to SEQ ID NO: 1;
ii) the first B3 primer comprises a nucleotide sequence at least 95%, or at least 98%, identical to SEQ ID NO: 2;
iii) the first F2 primer comprises a nucleotide sequence at least 95%, or at least 98%, identical to SEQ ID NO: 3;
iv) the first B2 primer comprises a nucleotide sequence at least 95%, or at least 98%, identical to SEQ ID NO: 4;
v) the first F1c primer comprises a nucleotide sequence at least 95%, or at least 98%, identical to SEQ ID NO: 5 or to SEQ ID NO: 8; and/or
vi) the first B1c primer comprises a nucleotide sequence at least 95%, or at least 98%, identical to SEQ ID NO: 6.

3. The primer set according to claim 1 or claim 2, wherein:

i) the first F3 primer comprises SEQ ID NO: 1 or a sequence resulting from a single nucleotide exchange, substitution or deletion of a single nucleotide;
ii) the first B3 primer comprises SEQ ID NO: 2 or a sequence resulting from a single nucleotide exchange, substitution or deletion of a single nucleotide;
iii) the first F2 primer comprises SEQ ID NO: 3 or a sequence resulting from a single nucleotide exchange, substitution or deletion of a single nucleotide;
iv) the first B2 primer comprises SEQ ID NO: 4 or a sequence resulting from a single nucleotide exchange, substitution or deletion of a single nucleotide;
v) the first F1c primer comprises SEQ ID NO: 5 or SEQ ID NO: 8 or a sequence resulting from a single nucleotide exchange, substitution or deletion of a single nucleotide; and/or
vi) the first B1c primer comprises SEQ ID NO: 6 or a sequence resulting from a single nucleotide exchange, substitution or deletion of a single nucleotide.

4. The primer set according to claim 1 or claim 2, wherein:

i) the first F3 primer comprises or consists of a nucleotide sequence identical to SEQ ID NO: 1;
ii) the first B3 primer comprises or consists of a nucleotide sequence identical to SEQ ID NO: 2;
iii) the first F2 primer comprises or consists of a nucleotide sequence identical to SEQ ID NO: 3;
iv) the first B2 primer comprises or consists of a nucleotide sequence identical to SEQ ID NO: 4;
v) the first F1c primer comprises or consists of a nucleotide sequence identical to SEQ ID NO: 5 or to SEQ ID NO: 8; and/or
vi) the first B1c primer comprises or consists of a nucleotide sequence identical to SEQ ID NO: 6.

5. The primer set according to any one of claims 1 to 4, further comprising vii) a LoopB primer, wherein the LoopB primer:

a) comprises a nucleotide sequence at least 90% identical to SEQ ID NO: 7;
b) comprises a nucleotide sequence at least 95% identical to SEQ ID NO: 7;
c) comprises a nucleotide sequence at least 98% identical to SEQ ID NO: 7;
d) comprises a nucleotide sequence identical to SEQ ID NO: 7; or
e) consists of SEQ ID NO: 7.

6. The primer set according to any one of claims 1 to 5, wherein the F1c primer and the F2 primer are linked to form a Forward Inner Primer (FIP).

7. The primer set according to claim 6, wherein

the F1c primer and the F2 primer are linked by a nucleotide bridge, preferably wherein
the bridge is made of between one and ten nucleotides, more preferably wherein the bridge is TTTT.

8. The primer set according to claim 6, wherein the FIP primer has a sequence selected from SEQ ID Nos: 9, 10 or 11.

9. The primer set according to any one of claims 1 to 8, wherein the B1c primer and the B2 primer are linked to form a Backward Inner Primer (BIP), optionally wherein the B1c primer and the B2 primer are linked by a nucleotide bridge, preferably wherein the bridge is made of between one and ten nucleotides, more preferably wherein the bridge is TTTT.

10. The primer set according to claim 9, wherein the BIP primer has a sequence selected from SEQ ID Nos: 12 and 13.

11. A kit for detecting Severe acute respiratory syndrome coronavirus 2 (SARS-CoV-2) in a sample, the kit comprising:

    reagents for amplifying RNA in a sample using a RT-LAMP technique; and
    a primer set according to any one of claims 1 to 10, optionally wherein
    one or more of the reagents or primers is dried or lyophilised; preferably wherein all of the primers are dried or lyophilised.

12. A method of detecting SARS-CoV-2 in a sample, comprising: amplifying by isothermal amplification at least a portion of the SARS-CoV-2 genome using a primer set according to any one of claims 1 to 10, optionally wherein the sample is obtained from a patient.

13. The method according to claim 12, further comprising detecting the amplified product by observing a fluorescence signal coming from the sample.

14. The method according to claim 12 or claim 13, wherein

    the amplification is carried out at between 65 and 70°C, preferably at about 68°C;
    wherein the amplification is carried out for 8-45 minutes, preferably for about 40 minutes; and/or
    the method further comprises a final step of holding at a temperature of between 75 and 85°C for 3 to 10 minutes, preferably at a temperature of about 80°C for about 5 minutes.

15. The method according to any one of claims 12 to 14, wherein the primers are used at the following reaction mixture concentrations:

    0.10 to 0.15 $\mu$M F3, preferably about 0.13 $\mu$M;
    0.10 to 0.15 $\mu$M B3, preferably about 0.13 $\mu$M;
    0.80 to 1.2 $\mu$M FIP, preferably about 1.0 $\mu$M;
    0.80 to 1.2 $\mu$M BIP, preferably about 1.00 $\mu$M; and/or
    0.2 to 0.3 $\mu$M LoopB, preferably about 0.25 $\mu$M.

**Patentansprüche**

1. Primer-Set zum Detektieren von SARS-CoV-2 unter Verwendung eines Reverse Transkription-Loop-vermittelten isothermalen Amplifizierungsverfahrens (RT-LAMP-Verfahrens), umfassend:

    i) mindestens einen ersten F3-Primer, umfassend eine Nukleotidsequenz, die mindestens 90 % identisch mit SEQ ID NO: 1 ist;
    ii) mindestens einen ersten B3-Primer, umfassend eine Nukleotidsequenz, die mindestens 90 % identisch mit SEQ ID NO: 2 ist;
    iii) mindestens einen ersten F2-Primer, umfassend eine Nukleotidsequenz, die mindestens 90 % identisch mit SEQ ID NO: 3 ist;
    iv) mindestens einen ersten B2-Primer, umfassend eine Nukleotidsequenz, die mindestens 90 % identisch mit SEQ ID NO: 4 ist;
    v) mindestens einen ersten F1c-Primer, umfassend eine Nukleotidsequenz, die mindestens 90 % identisch mit SEQ ID NO: 5 oder SEQ ID NO: 8 ist; und
    vi) mindestens einen ersten B1c-Primer, umfassend eine Nukleotidsequenz, die mindestens 90 % identisch mit SEQ ID NO: 6 ist.

2. Primer-Set nach Anspruch 1, wobei:

i) der erste F3-Primer eine Nukleotidsequenz umfasst, die mindestens 95 % oder mindestens 98 % identisch mit SEQ ID NO: 1 ist;

ii) der erste B3-Primer eine Nukleotidsequenz umfasst, die mindestens 95 % oder mindestens 98 % identisch mit SEQ ID NO: 2 ist;

iii) der erste F2-Primer eine Nukleotidsequenz umfasst, die mindestens 95 % oder mindestens 98 % identisch mit SEQ ID NO: 3 ist;

iv) der erste B2-Primer eine Nukleotidsequenz umfasst, die mindestens 95 % oder mindestens 98 % identisch mit SEQ ID NO: 4 ist;

v) der erste F1c-Primer eine Nukleotidsequenz umfasst, die mindestens 95 % oder mindestens 98 % identisch mit SEQ ID NO: 5 oder mit SEQ ID NO: 8 ist; und/oder

vi) der erste B1c-Primer eine Nukleotidsequenz umfasst, die mindestens 95 % oder mindestens 98 % identisch mit SEQ ID NO: 6 ist.

3. Primer-Set nach Anspruch 1 oder Anspruch 2, wobei:

i) der erste F3-Primer SEQ ID NO: 1 umfasst, oder eine Sequenz, die aus einem einzigen Nukleotidaustausch, einer Substitution oder Deletion eines einzigen Nukleotids resultiert;

ii) der erste B3-Primer SEQ ID NO: 2 umfasst, oder eine Sequenz, die aus einem einzigen Nukleotidaustausch, einer Substitution oder Deletion eines einzigen Nukleotids resultiert;

iii) der erste F2-Primer SEQ ID NO: 3 umfasst, oder eine Sequenz, die aus einem einzigen Nukleotidaustausch, einer Substitution oder Deletion eines einzigen Nukleotids resultiert;

iv) der erste B2-Primer SEQ ID NO: 4 umfasst, oder eine Sequenz, die aus einem einzigen Nukleotidaustausch, einer Substitution oder Deletion eines einzigen Nukleotids resultiert;

v) der erste F1c-Primer SEQ ID NO: 5 oder SEQ ID NO: 8 umfasst, oder eine Sequenz, die aus einem einzigen Nukleotidaustausch, Substitution oder Deletion eines einzigen Nukleotids resultiert; und/oder

vi) der erste B1c-Primer SEQ ID NO: 6 umfasst, oder eine Sequenz, die aus einem einzigen Nukleotidaustausch, einer Substitution oder Deletion eines einzigen Nukleotids resultiert.

4. Primer-Set nach Anspruch 1 oder Anspruch 2, wobei:

i) der erste F3-Primer eine Nukleotidsequenz umfasst oder daraus besteht, die identisch mit SEQ ID NO: 1 ist;

ii) der erste B3-Primer eine Nukleotidsequenz umfasst oder daraus besteht, die identisch mit SEQ ID NO: 2 ist;

iii) der erste F2-Primer eine Nukleotidsequenz umfasst oder daraus besteht, die identisch mit SEQ ID NO: 3 ist;

iv) der erste B2-Primer eine Nukleotidsequenz umfasst oder daraus besteht, die identisch mit SEQ ID NO: 4 ist;

v) der erste F1c-Primer eine Nukleotidsequenz umfasst oder daraus besteht, die identisch mit SEQ ID NO: 5 oder zu SEQ ID NO: 8 ist; und/oder

vi) der erste B1c-Primer eine Nukleotidsequenz umfasst oder daraus besteht, die identisch mit SEQ ID NO: 6 ist.

5. Primer-Set nach einem der Ansprüche 1 bis 4, des Weiteren umfassend vii) einen LoopB-Primer, wobei der LoopB-Primer:

a) eine Nukleotidsequenz umfasst, die mindestens 90 % identisch mit SEQ ID NO: 7 ist;

b) eine Nukleotidsequenz umfasst, die mindestens 95 % identisch mit SEQ ID NO: 7 ist;

c) eine Nukleotidsequenz umfasst, die mindestens 98 % identisch mit SEQ ID NO: 7 ist;

d) eine Nukleotidsequenz umfasst, die identisch mit SEQ ID NO: 7 ist; oder

e) aus SEQ ID NO: 7 besteht.

6. Primer-Set nach einem der Ansprüche 1 bis 5, wobei der F1c-Primer und der F2-Primer verknüpft sind, um einen Forward Inner Primer (FIP) zu bilden.

7. Primer-Set nach Anspruch 6, wobei

der F1c-Primer und der F2-Primer über eine Nukleotidbrücke verknüpft sind,

wobei die Brücke vorzugsweise aus zwischen einem und zehn Nukleotiden gebildet ist, wobei die Brücke bevorzugter TTTT ist.

8. Primer-Set nach Anspruch 6, wobei der FIP-Primer eine Sequenz ausgewählt aus SEQ ID NO: 9, 10 oder 11 hat.

9. Primer-Set nach einem der Ansprüche 1 bis 8, wobei der B1c-Primer und der B2-Primer verknüpft sind, um einen Backward Inner Primer (BIP) zu bilden, wobei gegebenenfalls der B1c-Primer und der B2-Primer mittels einer Nukleotidbrücke verknüpft sind, wobei die Brücke vorzugsweise aus zwischen einem und zehn Nukleotiden gebildet ist, wobei die Brücke bevorzugter TTTT ist.

10. Primer-Set nach Anspruch 9, wobei der BIP-Primer eine Sequenz ausgewählt aus SEQ ID NO: 12 und 13 hat.

11. Kit zum Detektieren von schwerem akutem Atemwegssyndrom-Coronavirus 2 (SARS-CoV-2) in einer Probe, wobei das Kit umfasst:

   Reagenzien zum Amplifizieren von RNA in einer Probe unter Verwendung einer RT-LAMP-Technik; und
   ein Primer-Set nach einem der Ansprüche 1 bis 10, wobei gegebenenfalls
   ein oder mehrere der Reagenzien oder Primer getrocknet oder lyophilisiert ist/sind; wobei vorzugsweise alle der Primer getrocknet oder lyophilisiert sind.

12. Verfahren zum Detektieren von SARS-CoV-2 in einer Probe, umfassend: Amplifizieren mindestens eines Abschnitts des Genoms von SARS-CoV-2 durch isothermale Amplifikation unter Verwendung eines Primer-Sets nach einem der Ansprüche 1 bis 10, wobei die Probe gegebenenfalls von einem Patienten erhalten wird.

13. Verfahren nach Anspruch 12, des Weiteren umfassend Detektieren des amplifizierten Produkts durch Ermitteln eines Fluoreszenzsignals, das aus der Probe kommt.

14. Verfahren nach Anspruch 12 oder Anspruch 13, wobei

   die Amplifikation bei zwischen 65 und 70 °C, vorzugsweise etwa 68 °C durchgeführt wird;
   wobei die Amplifikation für 8 bis 45 Minuten, vorzugsweise etwa 40 Minuten durchgeführt wird; und/oder
   das Verfahren des Weiteren einen letzten Schritt des Haltens auf einer Temperatur zwischen 75 und 85 °C für 3 bis 10 Minuten, vorzugsweise auf einer Temperatur von etwa 80 °C für etwa 5 Minuten umfasst.

15. Verfahren nach einem der Ansprüche 12 bis 14, wobei die Primer in den folgenden Konzentrationen in der Reaktionsmischung verwendet werden:

   0,10 bis 0,15 $\mu$M F3, vorzugsweise etwa 0,13 $\mu$M;
   0,10 bis 0,15 $\mu$M B3, vorzugsweise etwa 0,13 $\mu$M;
   0,80 bis 1,2 $\mu$M FIP, vorzugsweise etwa 1,0 $\mu$M;
   0,80 bis 1,2 $\mu$M BIP, vorzugsweise etwa 1,00 $\mu$M; und/oder
   0,2 bis 0,3 $\mu$M LoopB, vorzugsweise etwa 0,25 $\mu$M.

**Revendications**

1. Ensemble d'amorces pour détecter le SARS-CoV-2 à l'aide d'une méthode d'amplification isotherme médiée par boucles avec transcription inverse (RT-LAMP), comprenant :

   i) au moins une première amorce F3 comprenant une séquence nucléotidique identique à au moins 90 % à la SEQ ID NO : 1 ;
   ii) au moins une première amorce B3 comprenant une séquence nucléotidique identique à au moins 90 % à la SEQ ID NO : 2 ;
   iii) au moins une première amorce F2 comprenant une séquence nucléotidique identique à au moins 90 % à la SEQ ID NO : 3 ;
   iv) au moins une première amorce B2 comprenant une séquence nucléotidique identique à au moins 90 % à la SEQ ID NO : 4 ;
   v) au moins une première amorce F1c comprenant une séquence nucléotidique identique à au moins 90 % à la SEQ ID NO : 5 ou à la SEQ ID NO : 8 ; et
   vi) au moins une première amorce B1c comprenant une séquence nucléotidique identique à au moins 90 % à la SEQ ID NO : 6.

2. Ensemble d'amorces selon la revendication 1, dans lequel :

i) la première amorce F3 comprend une séquence nucléotidique identique à au moins 95 %, ou à au moins 98 %, à la SEQ ID NO : 1 ;

ii) la première amorce B3 comprend une séquence nucléotidique identique à au moins 95 %, ou à au moins 98 %, à la SEQ ID NO : 2 ;

iii) la première amorce F2 comprend une séquence nucléotidique identique à au moins 95 %, ou à au moins 98 %, à la SEQ ID NO : 3 ;

iv) la première amorce B2 comprend une séquence nucléotidique identique à au moins 95 %, ou à au moins 98 %, à la SEQ ID NO : 4 ;

v) la première amorce F1c comprend une séquence nucléotidique identique à au moins 95 %, ou à au moins 98 %, à la SEQ ID NO : 5 ou à la SEQ ID NO : 8 ; et/ou

vi) la première amorce B1c comprend une séquence nucléotidique identique à au moins 95 %, ou à au moins 98 %, à la SEQ ID NO : 6.

3. Ensemble d'amorces selon la revendication 1 ou la revendication 2, dans lequel :

i) la première amorce F3 comprend la SEQ ID NO : 1 ou une séquence résultant d'un échange d'un seul nucléotide, d'une substitution ou d'une délétion d'un seul nucléotide ;

ii) la première amorce B3 comprend la SEQ ID NO : 2 ou une séquence résultant d'un échange d'un seul nucléotide, d'une substitution ou d'une délétion d'un seul nucléotide ;

iii) la première amorce F2 comprend la SEQ ID NO : 3 ou une séquence résultant d'un échange d'un seul nucléotide, d'une substitution ou d'une délétion d'un seul nucléotide ;

iv) la première amorce B2 comprend la SEQ ID NO : 4 ou une séquence résultant d'un échange d'un seul nucléotide, d'une substitution ou d'une délétion d'un seul nucléotide ;

v) la première amorce F1c comprend la SEQ ID NO : 5 ou la SEQ ID NO : 8 ou une séquence résultant d'un échange d'un seul nucléotide, d'une substitution ou d'une délétion d'un seul nucléotide ; et/ou

vi) la première amorce B1c comprend la SEQ ID NO : 6 ou une séquence résultant d'un échange d'un seul nucléotide, d'une substitution ou d'une délétion d'un seul nucléotide.

4. Ensemble d'amorces selon la revendication 1 ou la revendication 2, dans lequel :

i) la première amorce F3 comprend ou consiste en une séquence nucléotidique identique à la SEQ ID NO : 1 ;

ii) la première amorce B3 comprend ou consiste en une séquence nucléotidique identique à la SEQ ID NO : 2 ;

iii) la première amorce F2 comprend ou consiste en une séquence nucléotidique identique à la SEQ ID NO : 3 ;

iv) la première amorce B2 comprend ou consiste en une séquence nucléotidique identique à la SEQ ID NO : 4 ;

v) la première amorce F1c comprend ou consiste en une séquence nucléotidique identique à la SEQ ID NO : 5 ou à la SEQ ID NO : 8 ; et/ou

vi) la première amorce B1c comprend ou consiste en une séquence nucléotidique identique à la SEQ ID NO : 6.

5. Ensemble d'amorces selon l'une quelconque des revendications 1 à 4, comprenant en outre vii) une amorce LoopB, dans lequel l'amorce LoopB :

a) comprend une séquence nucléotidique identique à au moins 90 % à la SEQ ID NO : 7 ;

b) comprend une séquence nucléotidique identique à au moins 95 % à la SEQ ID NO : 7 ;

c) comprend une séquence nucléotidique identique à au moins 98 % à la SEQ ID NO : 7 ;

d) comprend une séquence nucléotidique identique à la SEQ ID NO : 7 ; ou

e) consiste en la SEQ ID NO : 7.

6. Ensemble d'amorces selon l'une quelconque des revendications 1 à 5, dans lequel l'amorce F1c et l'amorce F2 sont liées pour former une amorce interne directe (FIP).

7. Ensemble d'amorces selon la revendication 6, dans lequel l'amorce F1c et l'amorce F2 sont reliées par un pont nucléotidique, de préférence dans lequel le pont est composé d'entre un et dix nucléotides, plus préférentiellement dans lequel le pont est TTTT.

8. Ensemble d'amorces selon la revendication 6, dans lequel l'amorce FIP a une séquence choisie parmi les séquences SEQ ID NOs : 9, 10 ou 11.

9. Ensemble d'amorces selon l'une quelconque des revendications 1 à 8, dans lequel l'amorce B1c et l'amorce B2 sont liées pour former une amorce interne inverse (BIP), éventuellement dans lequel l'amorce B1c et l'amorce B2 sont liées par un pont nucléotidique, de préférence dans lequel le pont est composé d'entre un et dix nucléotides, plus préférentiellement dans lequel le pont est TTTT.

10. Ensemble d'amorces selon la revendication 9, dans lequel l'amorce BIP a une séquence choisie parmi les séquences SEQ ID NOs : 12 et 13.

11. Kit pour détecter le coronavirus 2 du syndrome respiratoire aigu sévère (SARS-CoV-2) dans un échantillon, le kit comprenant :

des réactifs pour amplifier l'ARN dans un échantillon à l'aide d'une technique RT-LAMP ; et
un ensemble d'amorces selon l'une quelconque des revendications 1 à 10, dans lequel, en option,
un ou plusieurs des réactifs ou amorces sont séchés ou lyophilisés ; de préférence dans lequel toutes les amorces sont séchées ou lyophilisées.

12. Procédé de détection du SARS-CoV-2 dans un échantillon, comprenant : l'amplification par amplification isotherme d'au moins une partie du génome du SARS-CoV-2 à l'aide d'un ensemble d'amorces selon l'une quelconque des revendications 1 à 10, dans lequel, en option, l'échantillon est obtenu à partir d'un patient.

13. Procédé selon la revendication 12, comprenant en outre la détection du produit amplifié par observation d'un signal de fluorescence provenant de l'échantillon.

14. Procédé selon la revendication 12 ou la revendication 13, dans lequel

l'amplification est effectuée entre 65 et 70 °C, de préférence à environ 68 °C ;
dans lequel l'amplification est effectuée pendant 8 à 45 minutes, de préférence pendant environ 40 minutes ; et/ou
le procédé comprend en outre une étape finale de maintien à une température comprise entre 75 et 85 °C pendant 3 à 10 minutes, de préférence à une température d'environ 80 °C pendant environ 5 minutes.

15. Procédé selon l'une quelconque des revendications 12 à 14, dans lequel les amorces sont utilisées aux concentrations suivantes dans le mélange réactionnel :

0,10 à 0,15 $\mu$M de F3, de préférence environ 0,13 $\mu$M ;
0,10 à 0,15 $\mu$M de B3, de préférence environ 0,13 $\mu$M ;
0,80 à 1,2 $\mu$M de FIP, de préférence environ 1,0 $\mu$M ;
0,80 à 1,2 $\mu$M de BIP, de préférence environ 1,00 $\mu$M ; et/ou
0,2 à 0,3 $\mu$M de LoopB, de préférence environ 0,25 $\mu$M.

Figure 1

```
        F3                    F2                                    F1c
   CGGCAGT CAAGCCTCTTC CGTTCCTCATCACGTAGT CG                          gaggtccg tcgtcatyys
AGGCGGCAGT CAAGCCTCTT CTCGTTCCTC ATCACGTAGT CGCAACAGTT CAAGAAATTC AACTCCAGGC AGCAGTAGGG
531        541        551        561        571        581        591        601
                  B1c                   Loop B                    B2              B3
   ct    TCTCC TGCTAGAATG GCTGGCA ATGGCGGTGATGC TGCTCT  gaaacgacgacg aactgtctaacttggtcgaa
GAACTTCTCC TGCTAGAATG GCTGGCAATG GCGGTGATGC TGCTCTTGCT TTGCTGCTGC TTGACAGATT GAACCAGCTT
611        621        631        641        651        661        671        681

ctctcgtt
GAGAGCAAAA
691
```

Figure 2

Figure 3

Figure 4

Figure 5

Figure 6

Figure 7

```
MT359866.1:28264-29523   ATGTCTGATAATGGACCCCAAAAATCAGCGAAATGCACCCCGCATTACGTTTGGTGGACCC   60
MT328033.1:28274-29533   ATGTCTGATAATGGACCCCAAAAATCAGCGAAATGCACCCCGCATTACGTTTGGTGGACCC   60
MT328032.1:28274-29533   ATGTCTGATAATGGACCCCAAAAATCAGCGAAATGCACCCCGCATTACGTTTGGTGGACCC   60
MT371569.1:28200-29459   ATGTCTGATAATGGACCCCAAAAATCAGCGAAATGCACCCCGCATTACGTTTGGTGGACCC   60
MT371568.1:28150-29409   ATGTCTGATAATGGACCCCAAAAATCAGCGAAATGCACCCCGCATTACGTTTGGTGGACCC   60
MT371572.1:28166-29425   ATGTCTGATAATGGACCCCAAAAATCAGCGAAATGCACCCCGCATTACGTTTGGTGGACCC   60
MT276598.1:28278-29537   ATGTCTGATAATGGACCCCAAAAATCAGCGAAATGCACCCCGCATTACGTTTGGTGGACCC   60
MT374109.1:28271-29530   ATGTCTGATAATGGACCCCAAAAATCAGCGAAATGCACCCCGCATTACGTTTGGTGGACCC   60
MT328035.1:28274-29533   ATGTCTGATAATGGACCCCAAAAATCAGCGAAATGCACCCCGCATTACGTTTGGTGGACCC   60
MT292571.1:28220-29479   ATGTCTGATAATGGACCCCAAAAATCAGCGAAATGCACCCCGCATTACGTTTGGTGGACCC   60
MT233519.1:28220-29479   ATGTCTGATAATGGACCCCAAAAATCAGCGAAATGCACCCCGCATTACGTTTGGTGGACCC   60
MT198652.2:28220-29479   ATGTCTGATAATGGACCCCAAAAATCAGCGAAATGCACCCCGCATTACGTTTGGTGGACCC   60
MT292570.1:28220-29479   ATGTCTGATAATGGACCCCAAAAATCAGCGAAATGCACCCCGCATTACGTTTGGTGGACCC   60
MT372480.1:28239-29498   ATGTCTGATAATGGACCCCAAAAATCAGCGAAATGCACTCCGCATTACGTTTGGTGGACCC   60
MT304474.1:28274-29533   ATGTCTGATAATGGACCCCAAAAATCAGCGAAATGCACTCCGCATTACGTTTGGTGGACCC   60
MT304475.1:28274-29533   ATGTCTGATAATGGACCCCAAAAATCAGCGAAATGCACCCCGCATTACGTTTGGTGGACCC   60
MT114414.1:28274-29533   ATGTCTGATAATGGACCCCAAAAATCAGCGAAATGCACCCCGCATTACGTTTGGTGGACCC   60
MT252678.1:28225-29484   ATGTCTGATAATGGACCCCAAAAATCAGCGAAATGCACCCCGCATTACGTTTGGTGGACCC   60
MT252677.1:28256-29515   ATGTCTGATAATGGACCCCAAAAATCAGCGAAATGCACCCCGCATTACGTTTGGTGGACCC   60
MT370931.1:28212-29471   ATGTCTGATAATGGACCCCAAAAATCAGCGAAATGCACCCCGCATTACGTTTGGTGGACCC   60
MT370932.1:28219-29478   ATGTCTGATAATGGACCCCAAAAATCAGCGAAATGCACCCCGCATTACGTTTGGTGGACCC   60
MT370934.1:28219-29478   ATGTCTGATAATGGACCCCAAAAATCAGCGAAATGCACCCCGCATTACGTTTGGTGGACCC   60
MT325568.1:28274-29533   ATGTCTGATAATGGACCCCAAAAATCAGCGAAATGCACCCCGCATTACGTTTGGTGGACCC   60
MT325567.1:28274-29533   ATGTCTGATAATGGACCCCAAAAATCAGCGAAATGCACCCCGCATTACGTTTGGTGGACCC   60
MT325577.1:28274-29533   ATGTCTGATAATGGACCCCAAAAATCAGCGAAATGCACCCCGCATTACGTTTGGTGGACCC   60
MT325578.1:28274-29533   ATGTCTGATAATGGACCCCAAAAATCAGCGAAATGCACCCCGCATTACGTTTGGTGGACCC   60
MT344950.1:28274-29533   ATGTCTGATAATGGACCCCAAAAATCAGCGAAATGCACCCCGCATTACGTTTGGTGGACCC   60
MT344951.1:28274-29533   ATGTCTGATAATGGACCCCAAAAATCAGCGAAATGCACCCCGCATTACGTTTGGTGGACCC   60
MT359865.1:28261-29520   ATGTCTGATAATGGACCCCAAAAATCAGCGAAATGCACCCCGCATTACGTTTGGTGGACCC   60
MT066156.1:28274-29533   ATGTCTGATAATGGACCCCAAAAATCAGCGAAATGCACCCCGCATTACGTTTGGTGGACCC   60
MT077125.1:28218-29477   ATGTCTGATAATGGACCCCAAAAATCAGCGAAATGCACCCCGCATTACGTTTGGTGGACCC   60
NC_045512.2:28274-29533  ATGTCTGATAATGGACCCCAAAAATCAGCGAAATGCACCCCGCATTACGTTTGGTGGACCC   60
MT126808.1:28274-29533   ATGTCTGATAATGGACCCCAAAAATCAGCGAAATGCACCCCGCATTACGTTTGGTGGACCC   60
MT007544.1:28274-29533   ATGTCTGATAATGGACCCCAAAAATCAGCGAAATGCACCCCGCATTACGTTTGGTGGACCC   60
MN988668.1:28273-29532   ATGTCTGATAATGGACCCCAAAAATCAGCGAAATGCACCCCGCATTACGTTTGGTGGACCC   60
MT291833.1:28254-29513   ATGTCTGATAATGGACCCCAAAAATCAGCGAAATGCACCCCGCATTACGTTTGGTGGACCC   60
MT123292.2:28274-29533   ATGTCTGATAATGGACCCCAAAAATCAGCGAAATGCACCCCGCATTACGTTTGGTGGACCC   60
MT320538.2:28274-29533   ATGTCTGATAATGGACCCCAAAAATCAGCGAAATGCACCCCGCATTACGTTTGGTGGACCC   60
MT276597.1:28254-29513   ATGTCTGATAATGGACCCCAAAAATCAGCGAAATGCACCCCGCATTACGTTTGGTGGACCC   60
MT374105.1:28272-29531   ATGTCTGATAATGGACCCCAAAAATCAGCGAAATGCACCCCGCATTACGTTTGGTGGACCC   60
MT326027.1:28268-29527   ATGTCTGATAATGGACCCCAAAAATCAGCGAAATGCACCCCGCATTACGTTTGGTGGACCC   60
MT304483.1:28274-29533   ATGTCTGATAATGGACCCCAAAAATCAGCGAAATGCACCCCGCATTACGTTTGGTGGACCC   60
MT371023.1:28219-29478   ATGTCTGATAATGGACCCCAAAAATCAGCGAAATGCACCCCGCATTACGTTTGGTGGACCC   60
MT371015.1:28219-29478   ATGTCTGATAATGGACCCCAAAAATCAGCGAAATGCACCCCGCATTACGTTTGGTGGACCC   60
MT114413.1:28274-29533   ATGTCTGATAATGGACCCCAAAAATCAGCGAAATGCACCCCGCATTACGTTTGGTGGACCC   60
```

Figure 7 - cont.d

```
MT114412.1:28272-29531    ATGTCTGATAATGGACCCCAAAATCAGCGAAATGCACCCCGCATTACGTTTGGTGGACCC    60
                          ****************************************** *********************

MT359866.1:28264-29523    TCAGATTCAACTGGCAGTAACCAGAATGGAGAACGCAGTGGGGCGCGATCAAAACAACGT    120
MT328033.1:28274-29533    TCAGATTCAACTGGCAGTAACCAGAATGGAGAACGCAGTGGGGCGCGATCAAAACAACGT    120
MT328032.1:28274-29533    TCAGATTCAACTGGCAGTAACCAGAATGGAGAACGCAGTGGGGCGCGATCAAAACAACGT    120
MT371569.1:28200-29459    TCAGATTCAACTGGCAGTAACCAGAATGGAGAACGCAGTGGGGCGCGATCAAAACAACGT    120
MT371568.1:28150-29409    TCAGATTCAACTGGCAGTAACCAGAATGGAGAACGCAGTGGGGCGCGATCAAAACAACGT    120
MT371572.1:28166-29425    TCAGATTCAACTGGCAGTAACCAGAATGGAGAACGCAGTGGGGCGCGATCAAAACAACGT    120
MT276598.1:28278-29537    TCAGATTCAACTGGCAGTAACCAGAATGGAGAACGCAGTGGGGCGCGATCAAAACAACGT    120
MT374109.1:28271-29530    TCAGATTCAACTGGCAGTAACCAGAATGGAGAACGCAGTGGGGCGCGATCAAAACAACGT    120
MT328035.1:28274-29533    TCAGATTCAACTGGCAGTAACCAGAATGGAGAACGCAGTGGGGCGCGATCAAAACAACGT    120
MT292571.1:28220-29479    TCAGATTCAACTGGCAGTAACCAGAATGGAGAACGCAGTGGGGCGCGATCAAAACAACGT    120
MT233519.1:28220-29479    TCAGATTCAACTGGCAGTAACCAGAATGGAGAACGCAGTGGGGCGCGATCAAAACAACGT    120
MT198652.2:28220-29479    TCAGATTCAACTGGCAGTAACCAGAATGGAGAACGCAGTGGGGCGCGATCAAAACAACGT    120
MT292570.1:28220-29479    TCAGATTCAACTGGCAGTAACCAGAATGGAGAACGCAGTGGGGCGCGATCAAAACAACGT    120
MT372480.1:28239-29498    TCAGATTCAACTGGCAGTAACCAGAATGGAGAACGCAGTGGGGCGCGATCAAAACAACGT    120
MT304474.1:28274-29533    TCAGATTCAACTGGCAGTAACCAGAATGGAGAACGCAGTGGGGCGCGATCAAAACAACGT    120
MT304475.1:28274-29533    TCAGATTCAACTGGCAGTAACCAGAATGGAGAACGCAGTGGGGCGCGATCAAAACAACGT    120
MT114414.1:28274-29533    TCAGATTCAACTGGCAGTAACCAGAATGGAGAACGCAGTGGGGCGCGATCAAAACAACGT    120
MT252678.1:28225-29484    TCAGATTCAACTGGCAGTAACCAGAATGGAGAACGCAGTGGGGCGCGATCAAAACAACGT    120
MT252677.1:28256-29515    TCAGATTCAACTGGCAGTAACCAGAATGGAGAACGCAGTGGGGCGCGATCAAAACAACGT    120
MT370931.1:28212-29471    TCAGATTCAACTGGCAGTAACCAGAATGGAGAACGCAGTGGGGCGCGATCAAAACAACGT    120
MT370932.1:28219-29478    TCAGATTCAACTGGCAGTAACCAGAATGGAGAACGCAGTGGGGCGCGATCAAAACAACGT    120
MT370934.1:28219-29478    TCAGATTCAACTGGCAGTAACCAGAATGGAGAACGCAGTGGGGCGCGATCAAAACAACGT    120
MT325568.1:28274-29533    TCAGATTCAACTGGCAGTAACCAGAATGGAGAACGCAGTGGGGCGCGATCAAAACAACGT    120
MT325567.1:28274-29533    TCAGATTCAACTGGCAGTAACCAGAATGGAGAACGCAGTGGGGCGCGATCAAAACAACGT    120
MT325577.1:28274-29533    TCAGATTCAACTGGCAGTAACCAGAATGGAGAACGCAGTGGGGCGCGATCAAAACAACGT    120
MT325578.1:28274-29533    TCAGATTCAACTGGCAGTAACCAGAATGGAGAACGCAGTGGGGCGCGATCAAAACAACGT    120
MT344950.1:28274-29533    TCAGATTCAACTGGCAGTAACCAGAATGGAGAACGCAGTGGGGCGCGATCAAAACAACGT    120
MT344951.1:28274-29533    TCAGATTCAACTGGCAGTAACCAGAATGGAGAACGCAGTGGGGCGCGATCAAAACAACGT    120
MT359865.1:28261-29520    TCAGATTCAACTGGCAGTAACCAGAATGGAGAACGCAGTGGGGCGCGATCAAAACAACGT    120
MT066156.1:28274-29533    TCAGATTCAACTGGCAGTAACCAGAATGGAGAACGCAGTGGGGCGCGATCAAAACAACGT    120
MT077125.1:28218-29477    TCAGATTCAACTGGCAGTAACCAGAATGGAGAACGCAGTGGGGCGCGATCAAAACAACGT    120
NC_045512.2:28274-29533   TCAGATTCAACTGGCAGTAACCAGAATGGAGAACGCAGTGGGGCGCGATCAAAACAACGT    120
MT126808.1:28274-29533    TCAGATTCAACTGGCAGTAACCAGAATGGAGAACGCAGTGGGGCGCGATCAAAACAACGT    120
MT007544.1:28274-29533    TCAGATTCAACTGGCAGTAACCAGAATGGAGAACGCAGTGGGGCGCGATCAAAACAACGT    120
MN988668.1:28273-29532    TCAGATTCAACTGGCAGTAACCAGAATGGAGAACGCAGTGGGGCGCGATCAAAACAACGT    120
MT291833.1:28254-29513    TCAGATTCAACTGGCAGTAACCAGAATGGAGAACGCAGTGGGGCGCGATCAAAACAACGT    120
MT123292.2:28274-29533    TCAGATTCAACTGGCAGTAACCAGAATGGAGAACGCAGTGGGGCGCGATCAAAACAACGT    120
MT320538.2:28274-29533    TCAGATTCAACTGGCAGTAACCAGAATGGAGAACGCAGTGGGGCGCGATCAAAACAACGT    120
MT276597.1:28254-29513    TCAGATTCAACTGGCAGTAACCAGAATGGAGAACGCAGTGGGGCGCGATCAAAACAACGT    120
MT374105.1:28272-29531    TCAGATTCAACTGGCAGTAACCAGAATGGAGAACGCAGTGGGGCGCGATCAAAACAACGT    120
MT326027.1:28268-29527    TCAGATTCAACTGGCAGTAACCAGAATGGAGAACGCAGTGGGGCGCGATCAAAACAACGT    120
MT304483.1:28274-29533    TCAGATTCAACTGGCAGTAACCAGAATGGAGAACGCAGTGGGGCGCGATCAAAACAACGT    120
```

Figure 7 – cont.d

```
MT371023.1:28219-29478   TCAGATTCAACTGGCAGTAACCAGAATGGAGAACGCAGTGGGGCGCGATCAAAACAACGT   120
MT371015.1:28219-29478   TCAGATTCAACTGGCAGTAACCAGAATGGAGAACGCAGTGGGGCGCGATCAAAACAACGT   120
MT114413.1:28274-29533   TCAGATTCAACTGGCAGTAACCAGAATGGAGAACGCAGTGGGGCGCGATCAAAACAACGT   120
MT114412.1:28272-29531   TCAGATTCAACTGGCAGTAACCAGAATGGAGAACGCAGTGGGGCGCGATCAAAACAACGT   120
                         ************************************************************


MT359866.1:28264-29523   CGGCCCCAAGGTTTACCCAATAATACTGCGTCTTGGTTCACCGCTCTCACTCAACATGGC   180
MT328033.1:28274-29533   CGGCCCCAAGGTTTACCCAATAATACTGCGTCTTGGTTCACCGCTCTCACTCAACATGGC   180
MT328032.1:28274-29533   CGGCCCCAAGGTTTACCCAATAATACTGCGTCTTGGTTCACCGCTCTCACTCAACATGGC   180
MT371569.1:28200-29459   CGGCCCCAAGGTTTACCCAATAATACTGCGTCTTGGTTCACCGCTCTCACTCAACATGGC   180
MT371568.1:28150-29409   CGGCCCCAAGGTTTACCCAATAATACTGCGTCTTGGTTCACCGCTCTCACTCAACATGGC   180
MT371572.1:28166-29425   CGGCCCCAAGGTTTACCCAATAATACTGCGTCTTGGTTCACCGCTCTCACTCAACATGGC   180
MT276598.1:28278-29537   CGGCCCCAAGGTTTACCCAATAATACTGCGTCTTGGTTCACCGCTCTCACTCAACATGGC   180
MT374109.1:28271-29530   CGGCCCCAAGGTTTACCCAATAATACTGCGTCTTGGTTCACCGCTCTCACTCAACATGGC   180
MT328035.1:28274-29533   CGGCCCCAAGGTTTACCCAATAATACTGCGTCTTGGTTCACCGCTCTCACTCAACATGGC   180
MT292571.1:28220-29479   CGGCCCCAAGGTTTACCCAATAATACTGCGTCTTGGTTCACCGCTCTCACTCAACATGGC   180
MT233519.1:28220-29479   CGGCCCCAAGGTTTACCCAATAATACTGCGTCTTGGTTCACCGCTCTCACTCAACATGGC   180
MT198652.2:28220-29479   CGGCCCCAAGGTTTACCCAATAATACTGCGTCTTGGTTCACCGCTCTCACTCAACATGGC   180
MT292570.1:28220-29479   CGGCCCCAAGGTTTACCCAATAATACTGCGTCTTGGTTCACCGCTCTCACTCAACATGGC   180
MT372480.1:28239-29498   CGGCCCCAAGGTTTACCCAATAATACTGCGTCTTGGTTCACCGCTCTCACTCAACATGGC   180
MT304474.1:28274-29533   CGGCCCCAAGGTTTACCCAATAATACTGCGTCTTGGTTCACCGCTCTCACTCAACATGGC   180
MT304475.1:28274-29533   CGGCCCCAAGGTTTACCCAATAATACTGCGTCTTGGTTCACCGCTCTCACTCAACATGGC   180
MT114414.1:28274-29533   CGGCCCCAAGGTTTACCCAATAATACTGCGTCTTGGTTCACCGCTCTCACTCAACATGGC   180
MT252678.1:28225-29484   CGGCCCCAAGGTTTACCCAATAATACTGCGTCTTGGTTCACCGCTCTCACTCAACATGGC   180
MT252677.1:28256-29515   CGGCCCCAAGGTTTACCCAATAATACTGCGTCTTGGTTCACCGCTCTCACTCAACATGGC   180
MT370931.1:28212-29471   CGGCCCCAAGGTTTACCCAATAATACTGCGTCTTGGTTCACCGCTCTCACTCAACATGGC   180
MT370932.1:28219-29478   CGGCCCCAAGGTTTACCCAATAATACTGCGTCTTGGTTCACCGCTCTCACTCAACATGGC   180
MT370934.1:28219-29478   CGGCCCCAAGGTTTACCCAATAATACTGCGTCTTGGTTCACCGCTCTCACTCAACATGGC   180
MT325568.1:28274-29533   CGGCCCCAAGGTTTACCCAATAATACTGCGTCTTGGTTCACCGCTCTCACTCAACATGGC   180
MT325567.1:28274-29533   CGGCCCCAAGGTTTACCCAATAATACTGCGTCTTGGTTCACCGCTCTCACTCAACATGGC   180
MT325577.1:28274-29533   CGGCCCCAAGGTTTACCCAATAATACTGCGTCTTGGTTCACCGCTCTCACTCAACATGGC   180
MT325578.1:28274-29533   CGGCCCCAAGGTTTACCCAATAATACTGCGTCTTGGTTCACCGCTCTCACTCAACATGGC   180
MT344950.1:28274-29533   CGGCCCCAAGGTTTACCCAATAATACTGCGTCTTGGTTCACCGCTCTCACTCAACATGGC   180
MT344951.1:28274-29533   CGGCCCCAAGGTTTACCCAATAATACTGCGTCTTGGTTCACCGCTCTCACTCAACATGGC   180
MT359865.1:28261-29520   CGGCCCCAAGGTTTACCCAATAATACTGCGTCTTGGTTCACCGCTCTCACTCAACATGGC   180
MT066156.1:28274-29533   CGGCCCCAAGGTTTACCCAATAATACTGCGTCTTGGTTCACCGCTCTCACTCAACATGGC   180
MT077125.1:28218-29477   CGGCCCCAAGGTTTACCCAATAATACTGCGTCTTGGTTCACCGCTCTCACTCAACATGGC   180
NC_045512.2:28274-29533  CGGCCCCAAGGTTTACCCAATAATACTGCGTCTTGGTTCACCGCTCTCACTCAACATGGC   180
MT126808.1:28274-29533   CGGCCCCAAGGTTTACCCAATAATACTGCGTCTTGGTTCACCGCTCTCACTCAACATGGC   180
MT007544.1:28274-29533   CGGCCCCAAGGTTTACCCAATAATACTGCGTCTTGGTTCACCGCTCTCACTCAACATGGC   180
MN988668.1:28273-29532   CGGCCCCAAGGTTTACCCAATAATACTGCGTCTTGGTTCACCGCTCTCACTCAACATGGC   180
MT291833.1:28254-29513   CGGCCCCAAGGTTTACCCAATAATACTGCGTCTTGGTTCACCGCTCTCACTCAACATGGC   180
MT123292.2:28274-29533   CGGCCCCAAGGTTTACCCAATAATACTGCGTCTTGGTTCACCGCTCTCACTCAACATGGC   180
MT320538.2:28274-29533   CGGCCCCAAGGTTTACCCAATAATACTGCGTCTTGGTTCACCGCTCTCACTCAACATGGC   180
MT276597.1:28254-29513   CGGCCCCAAGGTTTACCCAATAATACTGCGTCTTGGTTCACCGCTCTCACTCAACATGGC   180
```

Figure 7 – cont.d

| | | |
|---|---|---|
| MT374105.1:28272-29531 | CGGCCCCAAGGTTTACCCAATAATACTGCGTCTTGGTTCACCGCTCTCACTCAACATGGC | 180 |
| MT326027.1:28268-29527 | CGGCCCCAAGGTTTACCCAATAATACTGCGTCTTGGTTCACCGCTCTCACTCAACATGGC | 180 |
| MT304483.1:28274-29533 | CGGCCCCAAGGTTTACCCAATAATACTGCGTCTTGGTTCACCGCTCTCACTCAACATGGC | 180 |
| MT371023.1:28219-29478 | CGGCCCCAAGGTTTACCCAATAATACTGCGTCTTGGTTCACCGCTCTCACTCAACATGGC | 180 |
| MT371015.1:28219-29478 | CGGCCCCAAGGTTTACCCAATAATACTGCGTCTTGGTTCACCGCTCTCACTCAACATGGC | 180 |
| MT114413.1:28274-29533 | CGGCCCCAAGGTTTACCCAATAATACTGCGTCTTGGTTCACCGCTCTCACTCAACATGGC | 180 |
| MT114412.1:28272-29531 | CGGCCCCAAGGTTTACCCAATAATACTGCGTCTTGGTTCACCGCTCTCACTCAACATGGC | 180 |
| | ************************************************************ | |

| | | |
|---|---|---|
| MT359866.1:28264-29523 | AAGGAAGACCTTAAATTCCCTCGAGGACAAGGCGTTCCAATTAACACCAATAGCAGTCCA | 240 |
| MT328033.1:28274-29533 | AAGGAAGACCTTAAATTCCCTCGAGGACAAGGCGTTCCAATTAACACCAATAGCAGTCCA | 240 |
| MT328032.1:28274-29533 | AAGGAAGACCTTAAATTCCCTCGAGGACAAGGCGTTCCAATTAACACCAATAGCAGTCCA | 240 |
| MT371569.1:28200-29459 | AAGGAAGACCTTAAATTCCCTCGAGGACAAGGCGTTCCAATTAACACCAATAGCAGTCCA | 240 |
| MT371568.1:28150-29409 | AAGGAAGACCTTAAATTCCCTCGAGGACAAGGCGTTCCAATTAACACCAATAGCAGTCCA | 240 |
| MT371572.1:28166-29425 | AAGGAAGACCTTAAATTCCCTCGAGGACAAGGCGTTCCAATTAACACCAATAGCAGTCCA | 240 |
| MT276598.1:28278-29537 | AAGGAAGACCTTAAATTCCCTCGAGGACAAGGCGTTCCAATTAACACCAATAGCAGTCCA | 240 |
| MT374109.1:28271-29530 | AAGGAAGACCTTAAATTCCCTCGAGGACAAGGCGTTCCAATTAACACCAATAGCAGTCCA | 240 |
| MT328035.1:28274-29533 | AAGGAAGACCTTAAATTCCCTCGAGGACAAGGCGTTCCAATTAACACCAATAGCAGTCCA | 240 |
| MT292571.1:28220-29479 | AAGGAAGACCTTAAATTCCCTCGAGGACAAGGCGTTCCAATTAACACCAATAGCAGTCCA | 240 |
| MT233519.1:28220-29479 | AAGGAAGACCTTAAATTCCCTCGAGGACAAGGCGTTCCAATTAACACCAATAGCAGTCCA | 240 |
| MT198652.2:28220-29479 | AAGGAAGACCTTAAATTCCCTCGAGGACAAGGCGTTCCAATTAACACCAATAGCAGTCCA | 240 |
| MT292570.1:28220-29479 | AAGGAAGACCTTAAATTCCCTCGAGGACAAGGCGTTCCAATTAACACCAATAGCAGTCCA | 240 |
| MT372480.1:28239-29498 | AAGGAAGACCTTAAATTCCCTCGAGGACAAGGCGTTCCAATTAACACCAATAGCAGTCCA | 240 |
| MT304474.1:28274-29533 | AAGGAAGACCTTAAATTCCCTCGAGGACAAGGCGTTCCAATTAACACCAATAGCAGTCCA | 240 |
| MT304475.1:28274-29533 | AAGGAAGACCTTAAATTCCCTCGAGGACAAGGCGTTCCAATTAACACCAATAGCAGTCCA | 240 |
| MT114414.1:28274-29533 | AAGGAAGACCTTAAATTCCCTCGAGGACAAGGCGTTCCAATTAACACCAATAGCAGTCCA | 240 |
| MT252678.1:28225-29484 | AAGGAAGACCTTAAATTCCCTCGAGGACAAGGCGTTCCAATTAACACCAATAGCAGTCCA | 240 |
| MT252677.1:28256-29515 | AAGGAAGACCTTAAATTCCCTCGAGGACAAGGCGTTCCAATTAACACCAATAGCAGTCCA | 240 |
| MT370931.1:28212-29471 | AAGGAAGACCTTAAATTCCCTCGAGGACAAGGCGTTCCAATTAACACCAATAGCAGTCCA | 240 |
| MT370932.1:28219-29478 | AAGGAAGACCTTAAATTCCCTCGAGGACAAGGCGTTCCAATTAACACCAATAGCAGTCCA | 240 |
| MT370934.1:28219-29478 | AAGGAAGACCTTAAATTCCCTCGAGGACAAGGCGTTCCAATTAACACCAATAGCAGTCCA | 240 |
| MT325568.1:28274-29533 | AAGGAAGACCTTAAATTCCCTCGAGGACAAGGCGTTCCAATTAACACCAATAGCAGTCCA | 240 |
| MT325567.1:28274-29533 | AAGGAAGACCTTAAATTCCCTCGAGGACAAGGCGTTCCAATTAACACCAATAGCAGTCCA | 240 |
| MT325577.1:28274-29533 | AAGGAAGACCTTAAATTCCCTCGAGGACAAGGCGTTCCAATTAACACCAATAGCAGTCCA | 240 |
| MT325578.1:28274-29533 | AAGGAAGACCTTAAATTCCCTCGAGGACAAGGCGTTCCAATTAACACCAATAGCAGTCCA | 240 |
| MT344950.1:28274-29533 | AAGGAAGACCTTAAATTCCCTCGAGGACAAGGCGTTCCAATTAACACCAATAGCAGTCCA | 240 |
| MT344951.1:28274-29533 | AAGGAAGACCTTAAATTCCCTCGAGGACAAGGCGTTCCAATTAACACCAATAGCAGTCCA | 240 |
| MT359865.1:28261-29520 | AAGGAAGACCTTAAATTCCCTCGAGGACAAGGCGTTCCAATTAACACCAATAGCAGTCCA | 240 |
| MT066156.1:28274-29533 | AAGGAAGACCTTAAATTCCCTCGAGGACAAGGCGTTCCAATTAACACCAATAGCAGTCCA | 240 |
| MT077125.1:28218-29477 | AAGGAAGACCTTAAATTCCCTCGAGGACAAGGCGTTCCAATTAACACCAATAGCAGTCCA | 240 |
| NC_045512.2:28274-29533 | AAGGAAGACCTTAAATTCCCTCGAGGACAAGGCGTTCCAATTAACACCAATAGCAGTCCA | 240 |
| MT126808.1:28274-29533 | AAGGAAGACCTTAAATTCCCTCGAGGACAAGGCGTTCCAATTAACACCAATAGCAGTCCA | 240 |
| MT007544.1:28274-29533 | AAGGAAGACCTTAAATTCCCTCGAGGACAAGGCGTTCCAATTAACACCAATAGCAGTCCA | 240 |
| MN988668.1:28273-29532 | AAGGAAGACCTTAAATTCCCTCGAGGACAAGGCGTTCCAATTAACACCAATAGCAGTCCA | 240 |
| MT291833.1:28254-29513 | AAGGAAGACCTTAAATTCCCTCGAGGACAAGGCGTTCCAATTAACACCAATAGCAGTCCA | 240 |

Figure 7 – cont.d

```
MT123292.2:28274-29533    AAGGAAGACCTTAAATTCCCTCGAGGACAAGGCGTTCCAATTAACACCAATAGCAGTCCA    240
MT320538.2:28274-29533    AAGGAAGACCTTAAATTCCCTCGAGGACAAGGCGTTCCAATTAACACCAATAGCAGTCCA    240
MT276597.1:28254-29513    AAGGAAGACCTTAAATTCCCTCGAGGACAAGGCGTTCCAATTAACACCAATAGCAGTCCA    240
MT374105.1:28272-29531    AAGGAAGACCTTAAATTCCCTCGAGGACAAGGCGTTCCAATTAACACCAATAGCAGTCCA    240
MT326027.1:28268-29527    AAGGAAGACCTTAAATTCCCTCGAGGACAAGGCGTTCCAATTAACACCAATAGCAGTCCA    240
MT304483.1:28274-29533    AAGGAAGACCTTAAATTCCCTCGAGGACAAGGCGTTCCAATTAACACCAATAGCAGTCCA    240
MT371023.1:28219-29478    AAGGAAGACCTTAAATTCCCTCGAGGACAAGGCGTTCCAATTAACACCAATAGCAGTCCA    240
MT371015.1:28219-29478    AAGGAAGACCTTAAATTCCCTCGAGGACAAGGCGTTCCAATTAACACCAATAGCAGTCCA    240
MT114413.1:28274-29533    AAGGAAGACCTTAAATTCCCTCGAGGACAAGGCGTTCCAATTAACACCAATAGCAGTCCA    240
MT114412.1:28272-29531    AAGGAAGACCTTAAATTCCCTCGAGGACAAGGCGTTCCAATTAACACCAATAGCAGTCCA    240
                          ************************************************************


MT359866.1:28264-29523    GATGACCAAATTGGCTACTACCGAAGAGCTACCAGACGAATTCGTGGTGGTGACGGTAAA    300
MT328033.1:28274-29533    GATGACCAAATTGGCTACTACCGAAGAGCTACCAGACGAATTCGTGGTGGTGACGGTAAA    300
MT328032.1:28274-29533    GATGACCAAATTGGCTACTACCGAAGAGCTACCAGACGAATTCGTGGTGGTGACGGTAAA    300
MT371569.1:28200-29459    GATGACCAAATTGGCTACTACCGAAGAGCTACCAGACGAATTCGTGGTGGTGACGGTAAA    300
MT371568.1:28150-29409    GATGACCAAATTGGCTACTACCGAAGAGCTACCAGACGAATTCGTGGTGGTGACGGTAAA    300
MT371572.1:28166-29425    GATGACCAAATTGGCTACTACCGAAGAGCTACCAGACGAATTCGTGGTGGTGACGGTAAA    300
MT276598.1:28278-29537    GATGACCAAATTGGCTACTACCGAAGAGCTACCAGACGAATTCGTGGTGGTGACGGTAAA    300
MT374109.1:28271-29530    GATGACCAAATTGGCTACTACCGAAGAGCTACCAGACGAATTCGTGGTGGTGACGGTAAA    300
MT328035.1:28274-29533    GATGACCAAATTGGCTACTACCGAAGAGCTACCAGACGAATTCGTGGTGGTGACGGTAAA    300
MT292571.1:28220-29479    GATGACCAAATTGGCTACTACCGAAGAGCTACCAGACGAATTCGTGGTGGTGACGGTAAA    300
MT233519.1:28220-29479    GATGACCAAATTGGCTACTACCGAAGAGCTACCAGACGAATTCGTGGTGGTGACGGTAAA    300
MT198652.2:28220-29479    GATGACCAAATTGGCTACTACCGAAGAGCTACCAGACGAATTCGTGGTGGTGACGGTAAA    300
MT292570.1:28220-29479    GATGACCAAATTGGCTACTACCGAAGAGCTACCAGACGAATTCGTGGTGGTGACGGTAAA    300
MT372480.1:28239-29498    GATGACCAAATTGGCTACTACCGAAGAGCTACCAGACGAATTCGTGGTGGTGACGGTAAA    300
MT304474.1:28274-29533    GATGACCAAATTGGCTACTACCGAAGAGCTACCAGACGAATTCGTGGTGGTGACGGTAAA    300
MT304475.1:28274-29533    GATGACCAAATTGGCTACTACCGAAGAGCTACCAGACGAATTCGTGGTGGTGACGGTAAA    300
MT114414.1:28274-29533    GATGACCAAATTGGCTACTACCGAAGAGCTACCAGACGAATTCGTGGTGGTGACGGTAAA    300
MT252678.1:28225-29484    GATGACCAAATTGGCTACTACCGAAGAGCTACCAGACGAATTCGTGGTGGTGACGGTAAA    300
MT252677.1:28256-29515    GATGACCAAATTGGCTACTACCGAAGAGCTACCAGACGAATTCGTGGTGGTGACGGTAAA    300
MT370931.1:28212-29471    GATGACCAAATTGGCTACTACCGAAGAGCTACCAGACGAATTCGTGGTGGTGACGGTAAA    300
MT370932.1:28219-29478    GATGACCAAATTGGCTACTACCGAAGAGCTACCAGACGAATTCGTGGTGGTGACGGTAAA    300
MT370934.1:28219-29478    GATGACCAAATTGGCTACTACCGAAGAGCTACCAGACGAATTCGTGGTGGTGACGGTAAA    300
MT325568.1:28274-29533    GATGACCAAATTGGCTACTACCGAAGAGCTACCAGACGAATTCGTGGTGGTGACGGTAAA    300
MT325567.1:28274-29533    GATGACCAAATTGGCTACTACCGAAGAGCTACCAGACGAATTCGTGGTGGTGACGGTAAA    300
MT325577.1:28274-29533    GATGACCAAATTGGCTACTACCGAAGAGCTACCAGACGAATTCGTGGTGGTGACGGTAAA    300
MT325578.1:28274-29533    GATGACCAAATTGGCTACTACCGAAGAGCTACCAGACGAATTCGTGGTGGTGACGGTAAA    300
MT344950.1:28274-29533    GATGACCAAATTGGCTACTACCGAAGAGCTACCAGACGAATTCGTGGTGGTGACGGTAAA    300
MT344951.1:28274-29533    GATGACCAAATTGGCTACTACCGAAGAGCTACCAGACGAATTCGTGGTGGTGACGGTAAA    300
MT359865.1:28261-29520    GATGACCAAATTGGCTACTACCGAAGAGCTACCAGACGAATTCGTGGTGGTGACGGTAAA    300
MT066156.1:28274-29533    GATGACCAAATTGGCTACTACCGAAGAGCTACCAGACGAATTCGTGGTGGTGACGGTAAA    300
MT077125.1:28218-29477    GATGACCAAATTGGCTACTACCGAAGAGCTACCAGACGAATTCGTGGTGGTGACGGTAAA    300
NC_045512.2:28274-29533   GATGACCAAATTGGCTACTACCGAAGAGCTACCAGACGAATTCGTGGTGGTGACGGTAAA    300
MT126808.1:28274-29533    GATGACCAAATTGGCTACTACCGAAGAGCTACCAGACGAATTCGTGGTGGTGACGGTAAA    300
```

Figure 7 – cont.d

| | | |
|---|---|---|
| MT007544.1:28274-29533 | GATGACCAAATTGGCTACTACCGAAGAGCTACCAGACGAATTCGTGGTGGTGACGGTAAA | 300 |
| MN988668.1:28273-29532 | GATGACCAAATTGGCTACTACCGAAGAGCTACCAGACGAATTCGTGGTGGTGACGGTAAA | 300 |
| MT291833.1:28254-29513 | GATGACCAAATTGGCTACTACCGAAGAGCTACCAGACGAATTCGTGGTGGTGACGGTAAA | 300 |
| MT123292.2:28274-29533 | GATGACCAAATTGGCTACTACCGAAGAGCTACCAGACGAATTCGTGGTGGTGACGGTAAA | 300 |
| MT320538.2:28274-29533 | GATGACCAAATTGGCTACTACCGAAGAGCTACCAGACGAATTCGTGGTGGTGACGGTAAA | 300 |
| MT276597.1:28254-29513 | GATGACCAAATTGGCTACTACCGAAGAGCTACCAGACGAATTCGTGGTGGTGACGGTAAA | 300 |
| MT374105.1:28272-29531 | GATGACCAAATTGGCTACTACCGAAGAGCTACCAGACGAATTCGTGGTGGTGACGGTAAA | 300 |
| MT326027.1:28268-29527 | GATGACCAAATTGGCTACTACCGAAGAGCTACCAGACGAATTCGTGGTGGTGACGGTAAA | 300 |
| MT304483.1:28274-29533 | GATGACCAAATTGGCTACTACCGAAGAGCTACCAGACGAATTCGTGGTGGTGACGGTAAA | 300 |
| MT371023.1:28219-29478 | GATGACCAAATTGGCTACTACCGAAGAGCTACCAGACGAATTCGTGGTGGTGACGGTAAA | 300 |
| MT371015.1:28219-29478 | GATGACCAAATTGGCTACTACCGAAGAGCTACCAGACGAATTCGTGGTGGTGACGGTAAA | 300 |
| MT114413.1:28274-29533 | GATGACCAAATTGGCTACTACCGAAGAGCTACCAGACGAATTCGTGGTGGTGACGGTAAA | 300 |
| MT114412.1:28272-29531 | GATGACCAAATTGGCTACTACCGAAGAGCTACCAGACGAATTCGTGGTGGTGACGGTAAA | 300 |
| | ************************************************************ | |
| | | |
| MT359866.1:28264-29523 | ATGAAAGATCTCAGTCCAAGATGGTATTTCTACTACCTAGGAACTGGGCCAGAAGCTGGA | 360 |
| MT328033.1:28274-29533 | ATGAAAGATCTCAGTCCAAGATGGTATTTCTACTACCTAGGAACTGGGCCAGAAGCTGGA | 360 |
| MT328032.1:28274-29533 | ATGAAAGATCTCAGTCCAAGATGGTATTTCTACTACCTAGGAACTGGGCCAGAAGCTGGA | 360 |
| MT371569.1:28200-29459 | ATGAAAGATCTCAGTCCAAGATGGTATTTCTACTACCTAGGAACTGGGCCAGAAGCTGGA | 360 |
| MT371568.1:28150-29409 | ATGAAAGATCTCAGTCCAAGATGGTATTTCTACTACCTAGGAACTGGGCCAGAAGCTGGA | 360 |
| MT371572.1:28166-29425 | ATGAAAGATCTCAGTCCAAGATGGTATTTCTACTACCTAGGAACTGGGCCAGAAGCTGGA | 360 |
| MT276598.1:28278-29537 | ATGAAAGATCTCAGTCCAAGATGGTATTTCTACTACCTAGGAACTGGGCCAGAAGCTGGA | 360 |
| MT374109.1:28271-29530 | ATGAAAGATCTCAGTCCAAGATGGTATTTCTACTACCTAGGAACTGGGCCAGAAGCTGGA | 360 |
| MT328035.1:28274-29533 | ATGAAAGATCTCAGTCCAAGATGGTATTTCTACTACCTAGGAACTGGGCCAGAAGCTGGA | 360 |
| MT292571.1:28220-29479 | ATGAAAGATCTCAGTCCAAGATGGTATTTCTACTACCTAGGAACTGGGCCAGAAGCTGGA | 360 |
| MT233519.1:28220-29479 | ATGAAAGATCTCAGTCCAAGATGGTATTTCTACTACCTAGGAACTGGGCCAGAAGCTGGA | 360 |
| MT198652.2:28220-29479 | ATGAAAGATCTCAGTCCAAGATGGTATTTCTACTACCTAGGAACTGGGCCAGAAGCTGGA | 360 |
| MT292570.1:28220-29479 | ATGAAAGATCTCAGTCCAAGATGGTATTTCTACTACCTAGGAACTGGGCCAGAAGCTGGA | 360 |
| MT372480.1:28239-29498 | ATGAAAGATCTCAGTCCAAGATGGTATTTCTACTACCTAGGAACTGGGCCAGAAGCTGGA | 360 |
| MT304474.1:28274-29533 | ATGAAAGATCTCAGTCCAAGATGGTATTTCTACTACCTAGGAACTGGGCCAGAAGCTGGA | 360 |
| MT304475.1:28274-29533 | ATGAAAGATCTCAGTCCAAGATGGTATTTCTACTACCTAGGAACTGGGCCAGAAGCTGGA | 360 |
| MT114414.1:28274-29533 | ATGAAAGATCTCAGTCCAAGATGGTATTTCTACTACCTAGGAACTGGGCCAGAAGCTGGA | 360 |
| MT252678.1:28225-29484 | ATGAAAGATCTCAGTCCAAGATGGTATTTCTACTACCTAGGAACTGGGCCAGAAGCTGGA | 360 |
| MT252677.1:28256-29515 | ATGAAAGATCTCAGTCCAAGATGGTATTTCTACTACCTAGGAACTGGGCCAGAAGCTGGA | 360 |
| MT370931.1:28212-29471 | ATGAAAGATCTCAGTCCAAGATGGTATTTCTACTACCTAGGAACTGGGCCAGAAGCTGGA | 360 |
| MT370932.1:28219-29478 | ATGAAAGATCTCAGTCCAAGATGGTATTTCTACTACCTAGGAACTGGGCCAGAAGCTGGA | 360 |
| MT370934.1:28219-29478 | ATGAAAGATCTCAGTCCAAGATGGTATTTCTACTACCTAGGAACTGGGCCAGAAGCTGGA | 360 |
| MT325568.1:28274-29533 | ATGAAAGATCTCAGTCCAAGATGGTATTTCTACTACCTAGGAACTGGGCCAGAAGCTGGA | 360 |
| MT325567.1:28274-29533 | ATGAAAGATCTCAGTCCAAGATGGTATTTCTACTACCTAGGAACTGGGCCAGAAGCTGGA | 360 |
| MT325577.1:28274-29533 | ATGAAAGATCTCAGTCCAAGATGGTATTTCTACTACCTAGGAACTGGGCCAGAAGCTGGA | 360 |
| MT325578.1:28274-29533 | ATGAAAGATCTCAGTCCAAGATGGTATTTCTACTACCTAGGAACTGGGCCAGAAGCTGGA | 360 |
| MT344950.1:28274-29533 | ATGAAAGATCTCAGTCCAAGATGGTATTTCTACTACCTAGGAACTGGGCCAGAAGCTGGA | 360 |
| MT344951.1:28274-29533 | ATGAAAGATCTCAGTCCAAGATGGTATTTCTACTACCTAGGAACTGGGCCAGAAGCTGGA | 360 |
| MT359865.1:28261-29520 | ATGAAAGATCTCAGTCCAAGATGGTATTTCTACTACCTAGGAACTGGGCCAGAAGCTGGA | 360 |
| MT066156.1:28274-29533 | ATGAAAGATCTCAGTCCAAGATGGTATTTCTACTACCTAGGAACTGGGCCAGAAGCTGGA | 360 |

Figure 7 – cont.d

```
MT077125.1:28218-29477   ATGAAAGATCTCAGTCCCAAGATGGTATTTCTACTACCTAGGAACTGGCCCAGAAGCTGGA   360
NC_045512.2:28274-29533  ATGAAAGATCTCAGTCCCAAGATGGTATTTCTACTACCTAGGAACTGGCCCAGAAGCTGGA   360
MT126808.1:28274-29533   ATGAAAGATCTCAGTCCCAAGATGGTATTTCTACTACCTAGGAACTGGCCCAGAAGCTGGA   360
MT007544.1:28274-29533   ATGAAAGATCTCAGTCCCAAGATGGTATTTCTACTACCTAGGAACTGGCCCAGAAGCTGGA   360
MN988668.1:28273-29532   ATGAAAGATCTCAGTCCCAAGATGGTATTTCTACTACCTAGGAACTGGCCCAGAAGCTGGA   360
MT291833.1:28254-29513   ATGAAAGATCTCAGTCCCAAGATGGTATTTCTACTACCTAGGAACTGGCCCAGAAGCTGGA   360
MT123292.2:28274-29533   ATGAAAGATCTCAGTCCCAAGATGGTATTTCTACTACCTAGGAACTGGCCCAGAAGCTGGA   360
MT320538.2:28274-29533   ATGAAAGATCTCAGTCCCAAGATGGTATTTCTACTACCTAGGAACTGGCCCAGAAGCTGGA   360
MT276597.1:28254-29513   ATGAAAGATCTCAGTCCCAAGATGGTATTTCTACTACCTAGGAACTGGCCCAGAAGCTGGA   360
MT374105.1:28272-29531   ATGAAAGATCTCAGTCCCAAGATGGTATTTCTACTACCTAGGAACTGGCCCAGAAGCTGGA   360
MT326027.1:28268-29527   ATGAAAGATCTCAGTCCCAAGATGGTATTTCTACTACCTAGGAACTGGCCCAGAAGCTGGA   360
MT304483.1:28274-29533   ATGAAAGATCTCAGTCCCAAGATGGTATTTCTACTACCTAGGAACTGGCCCAGAAGCTGGA   360
MT371023.1:28219-29478   ATGAAAGATCTCAGTCCCAAGATGGTATTTCTACTACCTAGGAACTGGCCCAGAAGCTGGA   360
MT371015.1:28219-29478   ATGAAAGATCTCAGTCCCAAGATGGTATTTCTACTACCTAGGAACTGGCCCAGAAGCTGGA   360
MT114413.1:28274-29533   ATGAAAGATCTCAGTCCCAAGATGGTATTTCTACTACCTAGGAACTGGCCCAGAAGCTGGA   360
MT114412.1:28272-29531   ATGAAAGATCTCAGTCCCAAGATGGTATTTCTACTACCTAGGAACTGGCCCAGAAGCTGGA   360
                         ************************************************************


MT359866.1:28264-29523   CTTCCCTATGGTGCTAACAAAGACGGCATCATATGGGTTGCAACTGAGGGAGCCTTGAAT   420
MT328033.1:28274-29533   CTTCCCTATGGTGCTAACAAAGACGGCATCATATGGGTTGCAACTGAGGGAGCCTTGAAT   420
MT328032.1:28274-29533   CTTCCCTATGGTGCTAACAAAGACGGCATCATATGGGTTGCAACTGAGGGAGCCTTGAAT   420
MT371569.1:28200-29459   CTTCCCTATGGTGCTAACAAAGACGGCATCATATGGGTTGCAACTGAGGGAGCCTTGAAT   420
MT371568.1:28150-29409   CTTCCCTATGGTGCTAACAAAGACGGCATCATATGGGTTGCAACTGAGGGAGCCTTGAAT   420
MT371572.1:28166-29425   CTTCCCTATGGTGCTAACAAAGACGGCATCATATGGGTTGCAACTGAGGGAGCCTTGAAT   420
MT276598.1:28278-29537   CTTCCCTATGGTGCTAACAAAGACGGCATCATATGGGTTGCAACTGAGGGAGCCTTGAAT   420
MT374109.1:28271-29530   CTTCCCTATGGTGCTAACAAAGACGGCATCATATGGGTTGCAACTGAGGGAGCCTTGAAT   420
MT328035.1:28274-29533   CTTCCCTATGGTGCTAACAAAGACGGCATCATATGGGTTGCAACTGAGGGAGCCTTGAAT   420
MT292571.1:28220-29479   CTTCCCTATGGTGCTAACAAAGACGGCATCATATGGGTTGCAACTGAGGGAGCCTTGAAT   420
MT233519.1:28220-29479   CTTCCCTATGGTGCTAACAAAGACGGCATCATATGGGTTGCAACTGAGGGAGCCTTGAAT   420
MT198652.2:28220-29479   CTTCCCTATGGTGCTAACAAAGACGGCATCATATGGGTTGCAACTGAGGGAGCCTTGAAT   420
MT292570.1:28220-29479   CTTCCCTATGGTGCTAACAAAGACGGCATCATATGATGGGTTGCAACTGAGGGAGCCTTGAAT   420
MT372480.1:28239-29498   CTTCCCTATGGTGCTAACAAAGACGGCATCATATGGGTTGCAACTGAGGGAGCCTTGAAT   420
MT304474.1:28274-29533   CTTCCCTATGGTGCTAACAAAGACGGCATCATATGGGTTGCAACTGAGGGAGCCTTGAAT   420
MT304475.1:28274-29533   CTTCCCTATGGTGCTAACAAAGACGGCATCATATGGGTTGCAACTGAGGGAGCCTTGAAT   420
MT114414.1:28274-29533   CTTCCCTATGGTGCTAACAAAGACGGCATCATATGGGTTGCAACTGAGGGAGCCTTGAAT   420
MT252678.1:28225-29484   CTTCCCTATGGTGCTAACAAAGACGGCATCATATGGGTTGCAACTGAGGGAGCCTTGAAT   420
MT252677.1:28256-29515   CTTCCCTATGGTGCTAACAAAGACGGCATCATATGGGTTGCAACTGAGGGAGCCTTGAAT   420
MT370931.1:28212-29471   CTTCCCTATGGTGCTAACAAAGACGGCATCATATGGGTTGCAACTGAGGGAGCCTTGAAT   420
MT370932.1:28219-29478   CTTCCCTATGGTGCTAACAAAGACGGCATCATATGGGTTGCAACTGAGGGAGCCTTGAAT   420
MT370934.1:28219-29478   CTTCCCTATGGTGCTAACAAAGACGGCATCATATGGGTTGCAACTGAGGGAGCCTTGAAT   420
MT325568.1:28274-29533   CTTCCCTATGGTGCTAACAAAGACGGCATCATATGGGTTGCAACTGAGGGAGCCTTGAAT   420
MT325567.1:28274-29533   CTTCCCTATGGTGCTAACAAAGACGGCATCATATGGGTTGCAACTGAGGGAGCCTTGAAT   420
MT325577.1:28274-29533   CTTCCCTATGGTGCTAACAAAGACGGCATCATATGGGTTGCAACTGAGGGAGCCTTGAAT   420
MT325578.1:28274-29533   CTTCCCTATGGTGCTAACAAAGACGGCATCATATGGGTTGCAACTGAGGGAGCCTTGAAT   420
MT344950.1:28274-29533   CTTCCCTATGGTGCTAACAAAGACGGCATCATATGGGTTGCAACTGAGGGAGCCTTGAAT   420
```

Figure 7 - cont.d

```
MT344951.1:28274-29533   CTTCCCTATGGTGCTAACAAAGACGGCATCATATGGGTTGCAACTGAGGGAGCCTTGAAT   420
MT359865.1:28261-29520   CTTCCCTATGGTGCTAACAAAGACGGCATCATATGGGTTGCAACTGAGGGAGCCTTGAAT   420
MT066156.1:28274-29533   CTTCCCTATGGTGCTAACAAAGACGGCATCATATGGGTTGCAACTGAGGGAGCCTTGAAT   420
MT077125.1:28218-29477   CTTCCCTATGGTGCTAACAAAGACGGCATCATATGGGTTGCAACTGAGGGAGCCTTGAAT   420
NC_045512.2:28274-29533  CTTCCCTATGGTGCTAACAAAGACGGCATCATATGGGTTGCAACTGAGGGAGCCTTGAAT   420
MT126808.1:28274-29533   CTTCCCTATGGTGCTAACAAAGACGGCATCATATGGGTTGCAACTGAGGGAGCCTTGAAT   420
MT007544.1:28274-29533   CTTCCCTATGGTGCTAACAAAGACGGCATCATATGGGTTGCAACTGAGGGAGCCTTGAAT   420
MN988668.1:28273-29532   CTTCCCTATGGTGCTAACAAAGACGGCATCATATGGGTTGCAACTGAGGGAGCCTTGAAT   420
MT291833.1:28254-29513   CTTCCCTATGGTGCTAACAAAGACGGCATCATATGGGTTGCAACTGAGGGAGCCTTGAAT   420
MT123292.2:28274-29533   CTTCCCTATGGTGCTAACAAAGACGGCATCATATGGGTTGCAACTGAGGGAGCCTTGAAT   420
MT320538.2:28274-29533   CTTCCCTATGGTGCTAACAAAGACGGCATCATATGGGTTGCAACTGAGGGAGCCTTGAAT   420
MT276597.1:28254-29513   CTTCCCTATGGTGCTAACAAAGACGGCATCATATGGGTTGCAACTGAGGGAGCCTTGAAT   420
MT374105.1:28272-29531   CTTCCCTATGGTGCTAACAAAGACGGCATCATATGGGTTGCAACTGAGGGAGCCTTGAAT   420
MT326027.1:28268-29527   CTTCCCTATGGTGCTAACAAAGACGGCATCATATGGGTTGCAACTGAGGGAGCCTTGAAT   420
MT304483.1:28274-29533   CTTCCCTATGGTGCTAACAAAGACGGCATCATATGGGTTGCAACTGAGGGAGCCTTGAAT   420
MT371023.1:28219-29478   CTTCCCTATGGTGCTAACAAAGACGGCATCATATGGGTTGCAACTGAGGGAGCCTTGAAT   420
MT371015.1:28219-29478   CTTCCCTATGGTGCTAACAAAGACGGCATCATATGGGTTGCAACTGAGGGAGCCTTGAAT   420
MT114413.1:28274-29533   CTTCCCTATGGTGCTAACAAAGACGGCATCATATGGGTTGCAACTGAGGGAGCCTTGAAT   420
MT114412.1:28272-29531   CTTCCCTATGGTGCTAACAAAGACGGCATCATATGGGTTGCAACTGAGGGAGCCTTGAAT   420
                         ************************************************************


MT359866.1:28264-29523   ACACCAAAAGATCACATTGGCACCCGCAATCCTGCTAACAATGCTGCAATCGTGCTACAA   480
MT328033.1:28274-29533   ACACCAAAAGATCACATTGGCACCCGCAATCCTGCTAACAATGCTGCAATCGTGCTACAA   480
MT328032.1:28274-29533   ACACCAAAAGATCACATTGGCACCCGCAATCCTGCTAACAATGCTGCAATCGTGCTACAA   480
MT371569.1:28200-29459   ACACCAAAAGATCACATTGGCACCCGCAATCCTGCTAACAATGCTGCAATCGTGCTACAA   480
MT371568.1:28150-29409   ACACCAAAAGATCACATTGGCACCCGCAATCCTGCTAACAATGCTGCAATCGTGCTACAA   480
MT371572.1:28166-29425   ACACCAAAAGATCACATTGGCACCCGCAATCCTGCTAACAATGCTGCAATCGTGCTACAA   480
MT276598.1:28278-29537   ACACCAAAAGATCACATTGGCACCCGCAATCCTGCTAACAATGCTGCAATCGTGCTACAA   480
MT374109.1:28271-29530   ACACCAAAAGATCACATTGGCACCCGCAATCCTGCTAACAATGCTGCAATCGTGCTACAA   480
MT328035.1:28274-29533   ACACCAAAAGATCACATTGGCACCCGCAATCCTGCTAACAATGCTGCAATCGTGCTACAA   480
MT292571.1:28220-29479   ACACCAAAAGATCACATTGGCACCCGCAATCCTGCTAACAATGCTGCAATCGTGCTACAA   480
MT233519.1:28220-29479   ACACCAAAAGATCACATTGGCACCCGCAATCCTGCTAACAATGCTGCAATCGTGCTACAA   480
MT198652.2:28220-29479   ACACCAAAAGATCACATTGGCACCCGCAATCCTGCTAACAATGCTGCAATCGTGCTACAA   480
MT292570.1:28220-29479   ACACCAAAAGATCACATTGGCACCCGCAATCCTGCTAACAATGCTGCAATCGTGCTACAA   480
MT372480.1:28239-29498   ACACCAAAAGATCACATTGGCACCCGCAATCCTGCTAACAATGCTGCAATCGTGCTACAA   480
MT304474.1:28274-29533   ACACCAAAAGATCACATTGGCACCCGCAATCCTGCTAACAATGCTGCAATCGTGCTACAA   480
MT304475.1:28274-29533   ACACCAAAAGATCACATTGGCACCCGCAATCCTGCTAACAATGCTGCAATCGTGCTACAA   480
MT114414.1:28274-29533   ACACCAAAAGATCACATTGGCACCCGCAATCCTGCTAACAATGCTGCAATCGTGCTACAA   480
MT252678.1:28225-29484   ACACCAAAAGATCACATTGGCACCCGCAATCCTGCTAACAATGCTGCAATCGTGCTACAA   480
MT252677.1:28256-29515   ACACCAAAAGATCACATTGGCACCCGCAATCCTGCTAACAATGCTGCAATCGTGCTACAA   480
MT370931.1:28212-29471   ACACCAAAAGATCACATTGGCACCCGCAATCCTGCTAACAATGCTGCAATCGTGCTACAA   480
MT370932.1:28219-29478   ACACCAAAAGATCACATTGGCACCCGCAATCCTGCTAACAATGCTGCAATCGTGCTACAA   480
MT370934.1:28219-29478   ACACCAAAAGATCACATTGGCACCCGCAATCCTGCTAACAATGCTGCAATCGTGCTACAA   480
MT325568.1:28274-29533   ACACCAAAAGATCACATTGGCACCCGCAATCCTGCTAACAATGCTGCAATCGTGCTACAA   480
MT325567.1:28274-29533   ACACCAAAAGATCACATTGGCACCCGCAATCCTGCTAACAATGCTGCAATCGTGCTACAA   480
```

27

Figure 7 – cont.d

```
MT325577.1:28274-29533    ACACCAAAAGATCACATTGGCACCCGCAATCCTGCTAACAATGCTGCAATCGTGCTACAA    480
MT325578.1:28274-29533    ACACCAAAAGATCACATTGGCACCCGCAATCCTGCTAACAATGCTGCAATCGTGCTACAA    480
MT344950.1:28274-29533    ACACCAAAAGATCACATTGGCACCCGCAATCCTGCTAACAATGCTGCAATCGTGCTACAA    480
MT344951.1:28274-29533    ACACCAAAAGATCACATTGGCACCCGCAATCCTGCTAACAATGCTGCAATCGTGCTACAA    480
MT359865.1:28261-29520    ACACCAAAAGATCACATTGGCACCCGCAATCCTGCTAACAATGCTGCAATCGTGCTACAA    480
MT066156.1:28274-29533    ACACCAAAAGATCACATTGGCACCCGCAATCCTGCTAACAATGCTGCAATCGTGCTACAA    480
MT077125.1:28218-29477    ACACCAAAAGATCACATTGGCACCCGCAATCCTGCTAACAATGCTGCAATCGTGCTACAA    480
NC_045512.2:28274-29533   ACACCAAAAGATCACATTGGCACCCGCAATCCTGCTAACAATGCTGCAATCGTGCTACAA    480
MT126808.1:28274-29533    ACACCAAAAGATCACATTGGCACCCGCAATCCTGCTAACAATGCTGCAATCGTGCTACAA    480
MT007544.1:28274-29533    ACACCAAAAGATCACATTGGCACCCGCAATCCTGCTAACAATGCTGCAATCGTGCTACAA    480
MN988668.1:28273-29532    ACACCAAAAGATCACATTGGCACCCGCAATCCTGCTAACAATGCTGCAATCGTGCTACAA    480
MT291833.1:28254-29513    ACACCAAAAGATCACATTGGCACCCGCAATCCTGCTAACAATGCTGCAATCGTGCTACAA    480
MT123292.2:28274-29533    ACACCAAAAGATCACATTGGCACCCGCAATCCTGCTAACAATGCTGCAATCGTGCTACAA    480
MT320538.2:28274-29533    ACACCAAAAGATCACATTGGCACCCGCAATCCTGCTAACAATGCTGCAATCGTGCTACAA    480
MT276597.1:28254-29513    ACACCAAAAGATCACATTGGCACCCGCAATCCTGCTAACAATGCTGCAATCGTGCTACAA    480
MT374105.1:28272-29531    ACACCAAAAGATCACATTGGCACCCGCAATCCTGCTAACAATGCTGCAATCGTGCTACAA    480
MT326027.1:28268-29527    ACACCAAAAGATCACATTGGCACCCGCAATCCTGCTAACAATGCTGCAATCGTGCTACAA    480
MT304483.1:28274-29533    ACACCAAAAGATCACATTGGCACCCGCAATCCTGCTAACAATGCTGCAATCGTGCTACAA    480
MT371023.1:28219-29478    ACACCAAAAGATCACATTGGCACCCGCAATCCTGCTAACAATGCTGCAATCGTGCTACAA    480
MT371015.1:28219-29478    ACACCAAAAGATCACATTGGCACCCGCAATCCTGCTAACAATGCTGCAATCGTGCTACAA    480
MT114413.1:28274-29533    ACACCAAAAGATCACATTGGCACCCGCAATCCTGCTAACAATGCTGCAATCGTGCTACAA    480
MT114412.1:28272-29531    ACACCAAAAGATCACATTGGCACCCGCAATCCTGCTAACAATGCTGCAATCGTGCTACAA    480
                          ************************************************************

MT359866.1:28264-29523    CTTCCTCAAGGAACAACATTGCCAAAAGGCTTCTACGCAGAAGGGAGCAGAGGCGGCAGT    540
MT328033.1:28274-29533    CTTCCTCAAGGAACAACATTGCCAAAAGGCTTCTACGCAGAAGGGAGCAGAGGCGGCAGT    540
MT328032.1:28274-29533    CTTCCTCAAGGAACAACATTGCCAAAAGGCTTCTACGCAGAAGGGAGCAGAGGCGGCAGT    540
MT371569.1:28200-29459    CTTCCTCAAGGAACAACATTGCCAAAAGGCTTCTACGCAGAAGGGAGCAGAGGCGGCAGT    540
MT371568.1:28150-29409    CTTCCTCAAGGAACAACATTGCCAAAAGGCTTCTACGCAGAAGGGAGCAGAGGCGGCAGT    540
MT371572.1:28166-29425    CTTCCTCAAGGAACAACATTGCCAAAAGGCTTCTACGCAGAAGGGAGCAGAGGCGGCAGT    540
MT276598.1:28278-29537    CTTCCTCAAGGAACAACATTGCCAAAAGGCTTCTACGCAGAAGGGAGCAGAGGCGGCAGT    540
MT374109.1:28271-29530    CTTCCTCAAGGAACAACATTGCCAAAAGGCTTCTACGCAGAAGGGAGCAGAGGCGGCAGT    540
MT328035.1:28274-29533    CTTCCTCAAGGAACAACATTGCCAAAAGGCTTCTACGCAGAAGGGAGCAGAGGCGGCAGT    540
MT292571.1:28220-29479    CTTCCTCAAGGAACAACATTGCCAAAAGGCTTCTACGCAGAAGGGAGCAGAGGCGGCAGT    540
MT233519.1:28220-29479    CTTCCTCAAGGAACAACATTGCCAAAAGGCTTCTACGCAGAAGGGAGCAGAGGCGGCAGT    540
MT198652.2:28220-29479    CTTCCTCAAGGAACAACATTGCCAAAAGGCTTCTACGCAGAAGGGAGCAGAGGCGGCAGT    540
MT292570.1:28220-29479    CTTCCTCAAGGAACAACATTGCCAAAAGGCTTCTACGCAGAAGGGAGCAGAGGCGGCAGT    540
MT372480.1:28239-29498    CTTCCTCAAGGAACAACATTGCCAAAAGGCTTCTACGCAGAAGGGAGCAGAGGCGGCAGT    540
MT304474.1:28274-29533    CTTCCTCAAGGAACAACATTGCCAAAAGGCTTCTACGCAGAAGGGAGCAGAGGCGGCAGT    540
MT304475.1:28274-29533    CTTCCTCAAGGAACAACATTGCCAAAAGGCTTCTACGCAGAAGGGAGCAGAGGCGGCAGT    540
MT114414.1:28274-29533    CTTCCTCAAGGAACAACATTGCCAAAAGGCTTCTACGCAGAAGGGAGCAGAGGCGGCAGT    540
MT252678.1:28225-29484    CTTCCTCAAGGAACAACATTGCCAAAAGGCTTCTACGCAGAAGGGAGCAGAGGCGGCAGT    540
MT252677.1:28256-29515    CTTCCTCAAGGAACAACATTGCCAAAAGGCTTCTACGCAGAAGGGAGCAGAGGCGGCAGT    540
MT370931.1:28212-29471    CTTCCTCAAGGAACAACATTGCCAAAAGGCTTCTACGCAGAAGGGAGCAGAGGCGGCAGT    540
MT370932.1:28219-29478    CTTCCTCAAGGAACAACATTGCCAAAAGGCTTCTACGCAGAAGGGAGCAGAGGCGGCAGT    540
```

Figure 7 - cont.d

```
MT370934.1:28219-29478    CTTCCTCAAGGAACAACATTGCCAAAAGGCTTCTACGCAGAAGGGAGCAGAGGCGGCAGT    540
MT325568.1:28274-29533    CTTCCTCAAGGAACAACATTGCCAAAAGGCTTCTACGCAGAAGGGAGCAGAGGCGGCAGT    540
MT325567.1:28274-29533    CTTCCTCAAGGAACAACATTGCCAAAAGGCTTCTACGCAGAAGGGAGCAGAGGCGGCAGT    540
MT325577.1:28274-29533    CTTCCTCAAGGAACAACATTGCCAAAAGGCTTCTACGCAGAAGGGAGCAGAGGCGGCAGT    540
MT325578.1:28274-29533    CTTCCTCAAGGAACAACATTGCCAAAAGGCTTCTACGCAGAAGGGAGCAGAGGCGGCAGT    540
MT344950.1:28274-29533    CTTCCTCAAGGAACAACATTGCCAAAAGGCTTCTACGCAGAAGGGAGCAGAGGCGGCAGT    540
MT344951.1:28274-29533    CTTCCTCAAGGAACAACATTGCCAAAAGGCTTCTACGCAGAAGGGAGCAGAGGCGGCAGT    540
MT359865.1:28261-29520    CTTCCTCAAGGAACAACATTGCCAAAAGGCTTCTACGCAGAAGGGAGCAGAGGCGGCAGT    540
MT066156.1:28274-29533    CTTCCTCAAGGAACAACATTGCCAAAAGGCTTCTACGCAGAAGGGAGCAGAGGCGGCAGT    540
MT077125.1:28218-29477    CTTCCTCAAGGAACAACATTGCCAAAAGGCTTCTACGCAGAAGGGAGCAGAGGCGGCAGT    540
NC_045512.2:28274-29533   CTTCCTCAAGGAACAACATTGCCAAAAGGCTTCTACGCAGAAGGGAGCAGAGGCGGCAGT    540
MT126808.1:28274-29533    CTTCCTCAAGGAACAACATTGCCAAAAGGCTTCTACGCAGAAGGGAGCAGAGGCGGCAGT    540
MT007544.1:28274-29533    CTTCCTCAAGGAACAACATTGCCAAAAGGCTTCTACGCAGAAGGGAGCAGAGGCGGCAGT    540
MN988668.1:28273-29532    CTTCCTCAAGGAACAACATTGCCAAAAGGCTTCTACGCAGAAGGGAGCAGAGGCGGCAGT    540
MT291833.1:28254-29513    CTTCCTCAAGGAACAACATTGCCAAAAGGCTTCTACGCAGAAGGGAGCAGAGGCGGCAGT    540
MT123292.2:28274-29533    CTTCCTCAAGGAACAACATTGCCAAAAGGCTTCTACGCAGAAGGGAGCAGAGGCGGCAGT    540
MT320538.2:28274-29533    CTTCCTCAAGGAACAACATTGCCAAAAGGCTTCTACGCAGAAGGGAGCAGAGGCGGCAGT    540
MT276597.1:28254-29513    CTTCCTCAAGGAACAACATTGCCAAAAGGCTTCTACGCAGAAGGGAGCAGAGGCGGCAGT    540
MT374105.1:28272-29531    CTTCCTCAAGGAACAACATTGCCAAAAGGCTTCTACGCAGAAGGGAGCAGAGGCGGCAGT    540
MT326027.1:28268-29527    CTTCCTCAAGGAACAACATTGCCAAAAGGCTTCTACGCAGAAGGGAGCAGAGGCGGCAGT    540
MT304483.1:28274-29533    CTTCCTCAAGGAACAACATTGCCAAAAGGCTTCTACGCAGAAGGGAGCAGAGGCGGCAGT    540
MT371023.1:28219-29478    CTTCCTCAAGGAACAACATTGCCAAAAGGCTTCTACGCAGAAGGGAGCAGAGGCGGCAGT    540
MT371015.1:28219-29478    CTTCCTCAAGGAACAACATTGCCAAAAGGCTTCTACGCAGAAGGGAGCAGAGGCGGCAGT    540
MT114413.1:28274-29533    CTTCCTCAAGGAACAACATTGCCAAAAGGCTTCTACGCAGAAGGGAGCAGAGGCGGCAGT    540
MT114412.1:28272-29531    CTTCCTCAAGGAACAACATTGCCAAAAGGCTTCTACGCAGAAGGGAGCAGAGGCGGCAGT    540
                          ************************************************************

MT359866.1:28264-29523    CAAGCCTCTTCTCGTTCCTCATCACGTAGTCGCAACAGTTCAAGAAATTCAACTCCAGGC    600
MT328033.1:28274-29533    CAAGCCTCTTCTCGTTCCTCATCACGTAGTCGCAACAGTTCAAGAAATTCAACTCCAGGC    600
MT328032.1:28274-29533    CAAGCCTCTTCTCGTTCCTCATCACGTAGTCGCAACAGTTCAAGAAATTCAACTCCAGGC    600
MT371569.1:28200-29459    CAAGCCTCTTCTCGTTCCTCATCACGTAGTCGCAACAGTTCAAGAAATTCAACTCCAGGC    600
MT371568.1:28150-29409    CAAGCCTCTTCTCGTTCCTCATCACGTAGTCGCAACAGTTCAAGAAATTCAACTCCAGGC    600
MT371572.1:28166-29425    CAAGCCTCTTCTCGTTCCTCATCACGTAGTCGCAACAGTTCAAGAAATTCAACTCCAGGC    600
MT276598.1:28278-29537    CAAGCCTCTTCTCGTTCCTCATCACGTAGTCGCAACAGTTCAAGAAATTCAACTCCAGGC    600
MT374109.1:28271-29530    CAAGCCTCTTCTCGTTCCTCATCACGTAGTCGCAACAGTTCAAGAAATTCAACTCCAGGC    600
MT328035.1:28274-29533    CAAGCCTCTTCTCGTTCCTCATCACGTAGTCGCAACAGTTCAAGAAATTCAACTCCAGGC    600
MT292571.1:28220-29479    CAAGCCTCTTCTCGTTCCTCATCACGTAGTCGCAACAGTTCAAGAAATTTAACTCCAGGC    600
MT233519.1:28220-29479    CAAGCCTCTTCTCGTTCCTCATCACGTAGTCGCAACAGTTCAAGAAATTTAACTCCAGGC    600
MT198652.2:28220-29479    CAAGCCTCTTCTCGTTCCTCATCACGTAGTCGCAACAGTTCAAGAAATTTAACTCCAGGC    600
MT292570.1:28220-29479    CAAGCCTCTTCTCGTTCCTCATCACGTAGTCGCAACAGTTCAAGAAATTCAACTCCAGGC    600
MT372480.1:28239-29498    CAAGCCTCTTCTCGTTCCTCATCACGTAGTCGCAACAGTTCAAGAAATTCAACTCCAGGC    600
MT304474.1:28274-29533    CAAGCCTCTTCTCGTTCCTCATCACGTAGTCGCAACAGTTCAAGAAATTCAACTCCAGGC    600
MT304475.1:28274-29533    CAAGCCTCTTCTCGTTCCTCATCACGTAGTCGCAACAGTTCAAGAAATTCAACTCCAGGC    600
MT114414.1:28274-29533    CAAGCCTCTTCTCGTTCCTCATCACGTAGTCGCAACAGTTCAAGAAATTCAACTCCAGGC    600
MT252678.1:28225-29484    CAAGCCTCTTCTCGTTCCTCATCACGTAGTCGCAACAGTTCAAGAAATTCAACTCCAGGC    600
```

EP 4 146 833 B1

Figure 7 - cont.d

```
MT252677.1:28256-29515    CAAGCCTCTTCTCGTTCCTCATCACGTAGTCGCAACAGTTCAAGAAATTCAACTCCAGGC    600
MT370931.1:28212-29471    CAAGCCTCTTCTCGTTCCTCATCACGTAGTCGCAACAGTTCAAGAAATTCAACTCCAGGC    600
MT370932.1:28219-29478    CAAGCCTCTTCTCGTTCCTCATCACGTAGTCGCAACAGTTCAAGAAATTCAACTCCAGGC    600
MT370934.1:28219-29478    CAAGCCTCTTCTCGTTCCTCATCACGTAGTCGCAACAGTTCAAGAAATTCAACTCCAGGC    600
MT325568.1:28274-29533    CAAGCCTCTTCTCGTTCCTCATCACGTAGTCGCAACAGTTCAAGAAATTCAACTCCAGGC    600
MT325567.1:28274-29533    CAAGCCTCTTCTCGTTCCTCATCACGTAGTCGCAACAGTTCAAGAAATTCAACTCCAGGC    600
MT325577.1:28274-29533    CAAGCCTCTTCTCGTTCCTCATCACGTAGTCGCAACAGTTCAAGAAATTCAACTCCAGGC    600
MT325578.1:28274-29533    CAAGCCTCTTCTCGTTCCTCATCACGTAGTCGCAACAGTTCAAGAAATTCAACTCCAGGC    600
MT344950.1:28274-29533    CAAGCCTCTTCTCGTTCCTCATCACGTAGTCGCAACAGTTCAAGAAATTCAACTCCAGGC    600
MT344951.1:28274-29533    CAAGCCTCTTCTCGTTCCTCATCACGTAGTCGCAACAGTTCAAGAAATTCAACTCCAGGC    600
MT359865.1:28261-29520    CAAGCCTCTTCTCGTTCCTCATCACGTAGTCGCAACAGTTCAAGAAATTCAACTCCAGGC    600
MT066156.1:28274-29533    CAAGCCTCTTCTCGTTCCTCATCACGTAGTCGCAACAGTTCAAGAAATTCAACTCCAGGC    600
MT077125.1:28218-29477    CAAGCCTCTTCTCGTTCCTCATCACGTAGTCGCAACAGTTCAAGAAATTCAACTCCAGGC    600
NC_045512.2:28274-29533   CAAGCCTCTTCTCGTTCCTCATCACGTAGTCGCAACAGTTCAAGAAATTCAACTCCAGGC    600
MT126808.1:28274-29533    CAAGCCTCTTCTCGTTCCTCATCACGTAGTCGCAACAGTTCAAGAAATTCAACTCCAGGC    600
MT007544.1:28274-29533    CAAGCCTCTTCTCGTTCCTCATCACGTAGTCGCAACAGTTCAAGAAATTCAACTCCAGGC    600
MN988668.1:28273-29532    CAAGCCTCTTCTCGTTCCTCATCACGTAGTCGCAACAGTTCAAGAAATTCAACTCCAGGC    600
MT291833.1:28254-29513    CAAGCCTCTTCTCGTTCCTCATCACGTAGTCGCAACAGTTCAAGAAATTCAACTCCAGGC    600
MT123292.2:28274-29533    CAAGCCTCTTCTCGTTCCTCATCACGTAGTCGCAACAGTTCAAGAAATTCAACTCCAGGC    600
MT320538.2:28274-29533    CAAGCCTCTTCTCGTTCCTCATCACGTAGTCGCAACAGTTCAAGAAATTCAACTCCAGGC    600
MT276597.1:28254-29513    CAAGCCTCTTCTCGTTCCTCATCACGTAGTCGCAACAGTTCAAGAAATTCAACTCCAGGC    600
MT374105.1:28272-29531    CAAGCCTCTTCTCGTTCCTCATCACGTAGTCGCAACAGTTCAAGAAATTCAACTCCAGGC    600
MT326027.1:28268-29527    CAAGCCTCTTCTCGTTCCTCATCACGTAGTCGCAACAGTTCAAGAAATTCAACTCCAGGC    600
MT304483.1:28274-29533    CAAGCCTCTTCTCGTTCCTCATCACGTAGTCGCAACAGTTCAAGAAATTCAACTCCAGGC    600
MT371023.1:28219-29478    CAAGCCTCTTCTCGTTCCTCATCACGTAGTCGCAACAGTTCAAGAAATTCAACTCCAGGC    600
MT371015.1:28219-29478    CAAGCCTCTTCTCGTTCCTCATCACGTAGTCGCAACAGTTCAAGAAATTCAACTCCAGGC    600
MT114413.1:28274-29533    CAAGCCTCTTCTCGTTCCTCATCACGTAGTCGCAACAGTTCAAGAAACTCAACTCCAGGC    600
MT114412.1:28272-29531    CAAGCCTCTTCTCGTTCCTCATCACGTAGTCGCAACAGTTCAAGAAACTCAACTCCAGGC    600
                          ************************************************** * *********


MT359866.1:28264-29523    AGCAGTAAACGAACTTCTCCTGCTAGAATGGCTGGCAATGGCGGTGATGCTGCTCTTGCT    660
MT328033.1:28274-29533    AGCAGTAAACGAACTTCTCCTGCTAGAATGGCTGGCAATGGCGGTGATGCTGCTCTTGCT    660
MT328032.1:28274-29533    AGCAGTAAACGAACTTCTCCTGCTAGAATGGCTGGCAATGGCGGTGATGCTGCTCTTGCT    660
MT371569.1:28200-29459    AGCAGTAAACGAACTTCTCCTGCTAGAATGGCTGGCAATGGCGGTGATGCTGCTCTTGCT    660
MT371568.1:28150-29409    AGCAGTAAACGAACTTCTCCTGCTAGAATGGCTGGCAATGGCGGTGATGCTGCTCTTGCT    660
MT371572.1:28166-29425    AGCAGTAAACGAACTTCTCCTGCTAGAATGGCTGGCAATGGCGGTGATGCTGCTCTTGCT    660
MT276598.1:28278-29537    AGCAGTAAACGAACTTCTCCTGCTAGAATGGCTGGCAATGGCGGTGATGCTGCTCTTGCT    660
MT374109.1:28271-29530    AGCAGTAAACGAACTTCTCCTGCTAGAATGGCTGGCAATGGCGGTGATGCTGCTCTTGCT    660
MT328035.1:28274-29533    AGCAGTAAACGAATTTCTCCTGCTAGAATGGCTGGCAATGGCGGTGATGCTGCTCTTGCT    660
MT292571.1:28220-29479    AGCAGTAGGGGAACTTCTCCTGCTAGAATGGCTGGCAATGGCGGTGATGCTGCTCTTGCT    660
MT233519.1:28220-29479    AGCAGTAGGGGAACTTCTCCTGCTAGAATGGCTGGCAATGGCGGTGATGCTGCTCTTGCT    660
MT198652.2:28220-29479    AGCAGTAGGGGAACTTCTCCTGCTAGAATGGCTGGCAATGGCGGTGATGCTGCTCTTGCT    660
MT292570.1:28220-29479    AGCAGTAGGGGAACTTCTCCTGCTAGAATGGCTGGCAATGGCGGTGATGCTGCTCTTGCT    660
MT372480.1:28239-29498    AGCAGTAGGGGAACTTCTCCTGCTAGAATGGCTGGCAATGGCGGTGATGCTGCTCTTGCT    660
MT304474.1:28274-29533    AGCAGTAGGGGAACTTCTCCTGCTAGAATGGCTGGCAATGGCGGTGATGCTGCTCTTGCT    660
```

Figure 7 - cont.d

```
MT304475.1:28274-29533    AGCAGTAGGGGAACTTCTCCTGCTAGAATGGCTGGCAATGGCGGTGATGCTGCTCTTGCT    660
MT114414.1:28274-29533    AGCAGTAGGGGAACTTCTCCTGCTAGAATGGCTGGCAATGGCGGTGATGCTGCTCTTGCT    660
MT252678.1:28225-29484    AGCAGTAGGGGAACTTCTCCTGCTAGAATGGCTGGCAATGGCGGTGATGCTGCTCTTGCT    660
MT252677.1:28256-29515    AGCAGTAGGGGAACTTCTCCTGCTAGAATGGCTGGCAATGGCGGTGATGCTGCTCTTGCT    660
MT370931.1:28212-29471    AGCAGTAGGGGAACTTCTCCTGCTAGAATGGCTGGCAATGGCGGTGATGCTGCTCTTGCT    660
MT370932.1:28219-29478    AGCAGTAGGGGAACTTCTCCTGCTAGAATGGCTGGCAATGGCGGTGATGCTGCTCTTGCT    660
MT370934.1:28219-29478    AGCAGTAGGGGAACTTCTCCTGCTAGAATGGCTGGCAATGGCGGTGATGCTGCTCTTGCT    660
MT325568.1:28274-29533    AGCAGTAGGGGAACTTCTCCTGCTAGAATGGCTGGCAATGGCGGTGATGCTGCTCTTGCT    660
MT325567.1:28274-29533    AGCAGTAGGGGAACTTCTCCTGCTAGAATGGCTGGCAATGGCGGTGATGCTGCTCTTGCT    660
MT325577.1:28274-29533    AGCAGTAGGGGAACTTCTCCTGCTAGAATGGCTGGCAATGGCGGTGATGCTGCTCTTGCT    660
MT325578.1:28274-29533    AGCAGTAGGGGAACTTCTCCTGCTAGAATGGCTGGCAATGGCGGTGATGCTGCTCTTGCT    660
MT344950.1:28274-29533    AGCAGTAGGGGAACTTCTCCTGCTAGAATGGCTGGCAATGGCGGTGATGCTGCTCTTGCT    660
MT344951.1:28274-29533    AGCAGTAGGGGAACTTCTCCTGCTAGAATGGCTGGCAATGGCGGTGATGCTGCTCTTGCT    660
MT359865.1:28261-29520    AGCAGTAGGGGAACTTCTCCTGCTAGAATGGCTGGCAATGGCGGTGATGCTGCTCTTGCT    660
MT066156.1:28274-29533    AGCAGTAGGGGAACTTCTCCTGCTAGAATGGCTGGCAATGGCGGTGATGCTGCTCTTGCT    660
MT077125.1:28218-29477    AGCAGTAGGGGAACTTCTCCTGCTAGAATGGCTGGCAATGGCGGTGATGCTGCTCTTGCT    660
NC_045512.2:28274-29533   AGCAGTAGGGGAACTTCTCCTGCTAGAATGGCTGGCAATGGCGGTGATGCTGCTCTTGCT    660
MT126808.1:28274-29533    AGCAGTAGGGGAACTTCTCCTGCTAGAATGGCTGGCAATGGCGGTGATGCTGCTCTTGCT    660
MT007544.1:28274-29533    AGCAGTAGGGGAACTTCTCCTGCTAGAATGGCTGGCAATGGCGGTGATGCTGCTCTTGCT    660
MN988668.1:28273-29532    AGCAGTAGGGGAACTTCTCCTGCTAGAATGGCTGGCAATGGCGGTGATGCTGCTCTTGCT    660
MT291833.1:28254-29513    AGCAGTAGGGGAACTTCTCCTGCTAGAATGGCTGGCAATGGCGGTGATGCTGCTCTTGCT    660
MT123292.2:28274-29533    AGCAGTAGGGGAACTTCTCCTGCTAGAATGGCTGGCAATGGCGGTGATGCTGCTCTTGCT    660
MT320538.2:28274-29533    AGCAGTAGGGGAACTTCTCCTGCTAGAATGGCTGGCAATGGCGGTGATGCTGCTCTTGCT    660
MT276597.1:28254-29513    AGCAGTAGGGGAACTTCTCCTGCTAGAATGGCTGGCAATGGCGGTGATGCTGCTCTTGCT    660
MT374105.1:28272-29531    AGCAGTAGGGGAACTTCTCCTGCTAGAATGGCTGGCAATGGCGGTGATGCTGCTCTTGCT    660
MT326027.1:28268-29527    AGCAGTAGGGGAACTTCTCCTGCTAGAATGGCTGGCAATGGCGGTGATGCTGCTCTTGCT    660
MT304483.1:28274-29533    AGCAGTAGGGGAACTTCTCCTGCTAGAATGGCTGGCAATGGCGGTGATGCTGCTCTTGCT    660
MT371023.1:28219-29478    AGCAGTAGGGGAACTTCTCCTGCTAGAATGGCTGGCAATGGCGGTGATGCTGCTCTTGCT    660
MT371015.1:28219-29478    AGCAGTAGGGGAACTTCTCCTGCTAGAATGGCTGGCAATGGCGGTGATGCTGCTCTTGCT    660
MT114413.1:28274-29533    AGCAGTAGGGGAACTTCTCCTGCTAGAATGGCTGGCAATGGCGGTGATGCTGCTCTTGCT    660
MT114412.1:28272-29531    AGCAGTAGGGGAACTTCTCCTGCTAGAATGGCTGGCAATGGCGGTGATGCTGCTCTTGCT    660

                          ******    *** ***********************************************


MT359866.1:28264-29523    TTGCTGCTGCTTGACAGATTGAACCAGCTTGAGAGCAAAATGTCTGGTAAAGGCCAACAA    720
MT328033.1:28274-29533    TTGCTGCTGCTTGACAGATTGAACCAGCTTGAGAGCAAAATGTCTGGTAAAGGCCAACAA    720
MT328032.1:28274-29533    TTGCTGCTGCTTGACAGATTGAACCAGCTTGAGAGCAAAATGTCTGGTAAAGGCCAACAA    720
MT371569.1:28200-29459    TTGCTGCTGCTTGACAGATTGAACCAGCTTGAGAGCAAAATGTCTGGTAAAGGCCAACAA    720
MT371568.1:28150-29409    TTGCTGCTGCTTGACAGATTGAACCAGCTTGAGAGCAAAATGTCTGGTAAAGGCCAACAA    720
MT371572.1:28166-29425    TTGCTGCTGCTTGACAGATTGAACCAGCTTGAGAGCAAAATGTCTGGTAAAGGCCAACAA    720
MT276598.1:28278-29537    TTGCTGCTGCTTGACAGATTGAACCAGCTTGAGAGCAAAATGTCTGGTAAAGGCCAACAA    720
MT374109.1:28271-29530    TTGCTGCTGCTTGACAGATTGAACCAGCTTGAGAGCAAAATGTCTGGTAAAGGCCAACAA    720
MT328035.1:28274-29533    TTGCTGCTGCTTGACAGATTGAACCAGCTTGAGAGCAAAATGTCTGGTAAAGGCCAACAA    720
MT292571.1:28220-29479    TTGCTGCTGCTTGACAGATTGAACCAGCTTGAGAGCAAAATGTCTGGTAAAGGCCAACAA    720
MT233519.1:28220-29479    TTGCTGCTGCTTGACAGATTGAACCAGCTTGAGAGCAAAATGTCTGGTAAAGGCCAACAA    720
MT198652.2:28220-29479    TTGCTGCTGCTTGACAGATTGAACCAGCTTGAGAGCAAAATGTCTGGTAAAGGCCAACAA    720
```

EP 4 146 833 B1

Figure 7 - cont.d

```
MT292570.1:28220-29479    TTGCTGCTGCTTGACAGATTGAACCAGCTTGAGAGCAAAATGTCTGGTAAAGGCCAACAA    720
MT372480.1:28239-29498    TTGCTGCTGCTTGACAGATTGAACCAGCTTGAGAGCAAAATGTCTGGTAAAGGCCAACAA    720
MT304474.1:28274-29533    TTGCTGCTGCTTGACAGATTGAACCAGCTTGAGAGCAAAATGTCTGGTAAAGGCCAACAA    720
MT304475.1:28274-29533    TTGCTGCTGCTTGACAGATTGAACCAGCTTGAGAGCAAAATGTCTGGTAAAGGCCAACAA    720
MT114414.1:28274-29533    TTGCTGCTGCTTGACAGATTGAACCAGCTTGAGAGCAAAATGTCTGGTAAAGGCCAACAA    720
MT252678.1:28225-29484    TTGCTGCTGCTTGACAGATTGAACCAGCTTGAGAGCAAAATGTCTGGTAAAGGCCAACAA    720
MT252677.1:28256-29515    TTGCTGCTGCTTGACAGATTGAACCAGCTTGAGAGCAAAATGTCTGGTAAAGGCCAACAA    720
MT370931.1:28212-29471    TTGCTGCTGCTTGACAGATTGAACCAGCTTGAGAGCAAAATGTCTGGTAAAGGCCAACAA    720
MT370932.1:28219-29478    TTGCTGCTGCTTGACAGATTGAACCAGCTTGAGAGCAAAATGTCTGGTAAAGGCCAACAA    720
MT370934.1:28219-29478    TTGCTGCTGCTTGACAGATTGAACCAGCTTGAGAGCAAAATGTCTGGTAAAGGCCAACAA    720
MT325568.1:28274-29533    TTGCTGCTGCTTGACAGATTGAACCAGCTTGAGAGCAAAATGTCTGGTAAAGGCCAACAA    720
MT325567.1:28274-29533    TTGCTGCTGCTTGACAGATTGAACCAGCTTGAGAGCAAAATGTCTGGTAAAGGCCAACAA    720
MT325577.1:28274-29533    TTGCTGCTGCTTGACAGATTGAACCAGCTTGAGAGCAAAATGTCTGGTAAAGGCCAACAA    720
MT325578.1:28274-29533    TTGCTGCTGCTTGACAGATTGAACCAGCTTGAGAGCAAAATGTCTGGTAAAGGCCAACAA    720
MT344950.1:28274-29533    TTGCTGCTGCTTGACAGATTGAACCAGCTTGAGAGCAAAATGTCTGGTAAAGGCCAACAA    720
MT344951.1:28274-29533    TTGCTGCTGCTTGACAGATTGAACCAGCTTGAGAGCAAAATGTCTGGTAAAGGCCAACAA    720
MT359865.1:28261-29520    TTGCTGCTGCTTGACAGATTGAACCAGCTTGAGAGCAAAATGTCTGGTAAAGGCCAACAA    720
MT066156.1:28274-29533    TTGCTGCTGCTTGACAGATTGAACCAGCTTGAGAGCAAAATGTCTGGTAAAGGCCAACAA    720
MT077125.1:28218-29477    TTGCTGCTGCTTGACAGATTGAACCAGCTTGAGAGCAAAATGTCTGGTAAAGGCCAACAA    720
NC_045512.2:28274-29533   TTGCTGCTGCTTGACAGATTGAACCAGCTTGAGAGCAAAATGTCTGGTAAAGGCCAACAA    720
MT126808.1:28274-29533    TTGCTGCTGCTTGACAGATTGAACCAGCTTGAGAGCAAAATGTCTGGTAAAGGCCAACAA    720
MT007544.1:28274-29533    TTGCTGCTGCTTGACAGATTGAACCAGCTTGAGAGCAAAATGTCTGGTAAAGGCCAACAA    720
MN988668.1:28273-29532    TTGCTGCTGCTTGACAGATTGAACCAGCTTGAGAGCAAAATGTCTGGTAAAGGCCAACAA    720
MT291833.1:28254-29513    TTGCTGCTGCTTGACAGATTGAACCAGCTTGAGAGCAAAATGTCTGGTAAAGGCCAACAA    720
MT123292.2:28274-29533    TTGCTGCTGCTTGACAGATTGAACCAGCTTGAGAGCAAAATGTCTGGTAAAGGCCAACAA    720
MT320538.2:28274-29533    TTGCTGCTGCTTGACAGATTGAACCAGCTTGAGAGCAAAATGTCTGGTAAAGGCCAACAA    720
MT276597.1:28254-29513    TTGCTGCTGCTTGACAGATTGAACCAGCTTGAGAGCAAAATGTCTGGTAAAGGCCAACAA    720
MT374105.1:28272-29531    TTGCTGCTGCTTGACAGATTGAACCAGCTTGAGAGCAAAATGTCTGGTAAAGGCCAACAA    720
MT326027.1:28268-29527    TTGCTGCTGCTTGACAGATTGAACCAGCTTGAGAGCAAAATGTCTGGTAAAGGCCAACAA    720
MT304483.1:28274-29533    TTGCTGCTGCTTGACAGATTGAACCAGCTTGAGAGCAAAATGTCTGGTAAAGGCCAACAA    720
MT371023.1:28219-29478    TTGCTGCTGCTTGACAGATTGAACCAGCTTGAGAGCAAAATGTCTGGTAAAGGCCAACAA    720
MT371015.1:28219-29478    TTGCTGCTGCTTGACAGATTGAACCAGCTTGAGAGCAAAATGTCTGGTAAAGGCCAACAA    720
MT114413.1:28274-29533    TTGCTGCTGCTTGACAGATTGAACCAGCTTGAGAGCAAAATGTCTGGTAAAGGCCAACAA    720
MT114412.1:28272-29531    TTGCTGCTGCTTGACAGATTGAACCAGCTTGAGAGCAAAATGTCTGGTAAAGGCCAACAA    720
                          ************************************************************

MT359866.1:28264-29523    CAACAAGGCCAAACTGTCACTAAGAAATCTGCTGCTGAGGCTTCTAAGAAGCCTCGGCAA    780
MT328033.1:28274-29533    CAACAAGGCCAAACTGTCACTAAGAAATCTGCTGCTGAGGCTTCTAAGAAGCCTCGGCAA    780
MT328032.1:28274-29533    CAACAAGGCCAAACTGTCACTAAGAAATCTGCTGCTGAGGCTTCTAAGAAGCCTCGGCAA    780
MT371569.1:28200-29459    CAACAAGGCCAAACTGTCACTAAGAAATCTGCTGCTGAGGCTTCTAAGAAGCCTCGGCAA    780
MT371568.1:28150-29409    CAACAAGGCCAAACTGTCACTAAGAAATCTGCTGCTGAGGCTTCTAAGAAGCCTCGGCAA    780
MT371572.1:28166-29425    CAACAAGGCCAAACTGTCACTAAGAAATCTGCTGCTGAGGCTTCTAAGAAGCCTCGGCAA    780
MT276598.1:28278-29537    CAACAAGGCCAAACTGTCACTAAGAAATCTGCTGCTGAGGCTTCTAAGAAGCCTCGGCAA    780
MT374109.1:28271-29530    CAACAAGGCCAAACTGTCACTAAGAAATCTGCTGCTGAGGCTTCTAAGAAGCCTCGGCAA    780
MT328035.1:28274-29533    CAACAAGGCCAAACTGTCACTAAGAAATCTGCTGCTGAGGCTTCTAAGAAGCCTCGGCAA    780
```

EP 4 146 833 B1

Figure 7 – cont.d

| | | |
|---|---|---|
| MT292571.1:28220-29479 | CAACAAGGCCAAACTGTCACTAAGAAATCTGCTGCTGAGGCTTCTAAGAAGCCTCGGCAA | 780 |
| MT233519.1:28220-29479 | CAACAAGGCCAAACTGTCACTAAGAAATCTGCTGCTGAGGCTTCTAAGAAGCCTCGGCAA | 780 |
| MT198652.2:28220-29479 | CAACAAGGCCAAACTGTCACTAAGAAATCTGCTGCTGAGGCTTCTAAGAAGCCTCGGCAA | 780 |
| MT292570.1:28220-29479 | CAACAAGGCCAAACTGTCACTAAGAAATCTGCTGCTGAGGCTTCTAAGAAGCCTCGGCAA | 780 |
| MT372480.1:28239-29498 | CAACAAGGCCAAACTGTCACTAAGAAATCTGCTGCTGAGGCTTCTAAGAAGCCTCGGCAA | 780 |
| MT304474.1:28274-29533 | CAACAAGGCCAAACTGTCACTAAGAAATCTGCTGCTGAGGCTTCTAAGAAGCCTCGGCAA | 780 |
| MT304475.1:28274-29533 | CAACAAGGCCAAACTGTCACTAAGAAATCTGCTGCTGAGGCTTCTAAGAAGCCTCGGCAA | 780 |
| MT114414.1:28274-29533 | CAACAAGGCCAAACTGTCACTAAGAAATCTGCTGCTGAGGCTTCTAAGAAGCCTCGGCAA | 780 |
| MT252678.1:28225-29484 | CAACAAGGCCAAACTGTCACTAAGAAATCTGCTGCTGAGGCTTCTAAGAAGCCTCGGCAA | 780 |
| MT252677.1:28256-29515 | CAACAAGGCCAAACTGTCACTAAGAAATCTGCTGCTGAGGCTTCTAAGAAGCCTCGGCAA | 780 |
| MT370931.1:28212-29471 | CAACAAGGCCAAACTGTCACTAAGAAATCTGCTGCTGAGGCTTCTAAGAAGCCTCGGCAA | 780 |
| MT370932.1:28219-29478 | CAACAAGGCCAAACTGTCACTAAGAAATCTGCTGCTGAGGCTTCTAAGAAGCCTCGGCAA | 780 |
| MT370934.1:28219-29478 | CAACAAGGCCAAACTGTCACTAAGAAATCTGCTGCTGAGGCTTCTAAGAAGCCTCGGCAA | 780 |
| MT325568.1:28274-29533 | CAACAAGGCCAAACTGTCACTAAGAAATCTGCTGCTGAGGCTTCTAAGAAGCCTCGGCAA | 780 |
| MT325567.1:28274-29533 | CAACAAGGCCAAACTGTCACTAAGAAATCTGCTGCTGAGGCTTCTAAGAAGCCTCGGCAA | 780 |
| MT325577.1:28274-29533 | CAACAAGGCCAAACTGTCACTAAGAAATCTGCTGCTGAGGCTTCTAAGAAGCCTCGGCAA | 780 |
| MT325578.1:28274-29533 | CAACAAGGCCAAACTGTCACTAAGAAATCTGCTGCTGAGGCTTCTAAGAAGCCTCGGCAA | 780 |
| MT344950.1:28274-29533 | CAACAAGGCCAAACTGTCACTAAGAAATCTGCTGCTGAGGCTTCTAAGAAGCCTCGGCAA | 780 |
| MT344951.1:28274-29533 | CAACAAGGCCAAACTGTCACTAAGAAATCTGCTGCTGAGGCTTCTAAGAAGCCTCGGCAA | 780 |
| MT359865.1:28261-29520 | CAACAAGGCCAAACTGTCACTAAGAAATCTGCTGCTGAGGCTTCTAAGAAGCCTCGGCAA | 780 |
| MT066156.1:28274-29533 | CAACAAGGCCAAACTGTCACTAAGAAATCTGCTGCTGAGGCTTCTAAGAAGCCTCGGCAA | 780 |
| MT077125.1:28218-29477 | CAACAAGGCCAAACTGTCACTAAGAAATCTGCTGCTGAGGCTTCTAAGAAGCCTCGGCAA | 780 |
| NC_045512.2:28274-29533 | CAACAAGGCCAAACTGTCACTAAGAAATCTGCTGCTGAGGCTTCTAAGAAGCCTCGGCAA | 780 |
| MT126808.1:28274-29533 | CAACAAGGCCAAACTGTCACTAAGAAATCTGCTGCTGAGGCTTCTAAGAAGCCTCGGCAA | 780 |
| MT007544.1:28274-29533 | CAACAAGGCCAAACTGTCACTAAGAAATCTGCTGCTGAGGCTTCTAAGAAGCCTCGGCAA | 780 |
| MN988668.1:28273-29532 | CAACAAGGCCAAACTGTCACTAAGAAATCTGCTGCTGAGGCTTCTAAGAAGCCTCGGCAA | 780 |
| MT291833.1:28254-29513 | CAACAAGGCCAAACTGTCACTAAGAAATCTGCTGCTGAGGCTTCTAAGAAGCCTCGGCAA | 780 |
| MT123292.2:28274-29533 | CAACAAGGCCAAACTGTCACTAAGAAATCTGCTGCTGAGGCTTCTAAGAAGCCTCGGCAA | 780 |
| MT320538.2:28274-29533 | CAACAAGGCCAAACTGTCACTAAGAAATCTGCTGCTGAGGCTTCTAAGAAGCCTCGGCAA | 780 |
| MT276597.1:28254-29513 | CAACAAGGCCAAACTGTCACTAAGAAATCTGCTGCTGAGGCTTCTAAGAAGCCTCGGCAA | 780 |
| MT374105.1:28272-29531 | CAACAAGGCCAAACTGTCACTAAGAAATCTGCTGCTGAGGCTTCTAAGAAGCCTCGGCAA | 780 |
| MT326027.1:28268-29527 | CAACAAGGCCAAACTGTCACTAAGAAATCTGCTGCTGAGGCTTCTAAGAAGCCTCGGCAA | 780 |
| MT304483.1:28274-29533 | CAACAAGGCCAAACTGTCACTAAGAAATCTGCTGCTGAGGCTTCTAAGAAGCCTCGGCAA | 780 |
| MT371023.1:28219-29478 | CAACAAGGCCAAACTGTCACTAAGAAATCTGCTGCTGAGGCTTCTAAGAAGCCTCGGCAA | 780 |
| MT371015.1:28219-29478 | CAACAAGGCCAAACTGTCACTAAGAAATCTGCTGCTGAGGCTTCTAAGAAGCCTCGGCAA | 780 |
| MT114413.1:28274-29533 | CAACAAGGCCAAACTGTCACTAAGAAATCTGCTGCTGAGGCTTCTAAGAAGCCTCGGCAA | 780 |
| MT114412.1:28272-29531 | CAACAAGGCCAAACTGTCACTAAGAAATCTGCTGCTGAGGCTTCTAAGAAGCCTCGGCAA | 780 |
| | ************************************************************ | |
| | | |
| MT359866.1:28264-29523 | AAACGTACTGCCACTAAAGCATACAATGTAACACAAGCTTTCGGCAGACGTGGTCCAGAA | 840 |
| MT328033.1:28274-29533 | AAACGTACTGCCACTAAAGCATACAATGTAACACAAGCTTTCGGCAGACGTGGTCCAGAA | 840 |
| MT328032.1:28274-29533 | AAACGTACTGCCACTAAAGCATACAATGTAACACAAGCTTTCGGCAGACGTGGTCCAGAA | 840 |
| MT371569.1:28200-29459 | AAACGTACTGCCACTAAAGCATACAATGTAACACAAGCTTTCGGCAGACGTGGTCCAGAA | 840 |
| MT371568.1:28150-29409 | AAACGTACTGCCACTAAAGCATACAATGTAACACAAGCTTTCGGCAGACGTGGTCCAGAA | 840 |
| MT371572.1:28166-29425 | AAACGTACTGCCACTAAAGCATACAATGTAACACAAGCTTTCGGCAGACGTGGTCCAGAA | 840 |

EP 4 146 833 B1

Figure 7 - cont.d

```
MT276598.1:28278-29537   AAACGTACTGCCACTAAAGCATACAATGTAACACAAGCTTTCGGCAGACGTGGTCCAGAA   840
MT374109.1:28271-29530   AAACGTACTGCCACTAAAGCATACAATGTAACACAAGCTTTCGGCAGACGTGGTCCAGAA   840
MT328035.1:28274-29533   AAACGTACTGCCACTAAAGCATACAATGTAACACAAGCTTTCGGCAGACGTGGTCCAGAA   840
MT292571.1:28220-29479   AAACGTACTGCCACTAAAGCATACAATGTAACACAAGCTTTCGGCAGACGTGGTCCAGAA   840
MT233519.1:28220-29479   AAACGTACTGCCACTAAAGCATACAATGTAACACAAGCTTTCGGCAGACGTGGTCCAGAA   840
MT198652.2:28220-29479   AAACGTACTGCCACTAAAGCATACAATGTAACACAAGCTTTCGGCAGACGTGGTCCAGAA   840
MT292570.1:28220-29479   AAACGTACTGCCACTAAAGCATACAATGTAACACAAGCTTTCGGCAGACGTGGTCCAGAA   840
MT372480.1:28239-29498   AAACGTACTGCCACTAAAGCATACAATGTAACACAAGCTTTCGGCAGACGTGGTCCAGAA   840
MT304474.1:28274-29533   AAACGTACTGCCACTAAAGCATACAATGTAACACAAGCTTTCGGCAGACGTGGTCCAGAA   840
MT304475.1:28274-29533   AAACGTACTGCCACTAAAGCATACAATGTAACACAAGCTTTCGGCAGACGTGGTCCAGAA   840
MT114414.1:28274-29533   AAACGTACTGCCACTAAAGCATACAATGTAACACAAGCTTTCGGCAGACGTGGTCCAGAA   840
MT252678.1:28225-29484   AAACGTACTGCCACTAAAGCATACAATGTAACACAAGCTTTCGGCAGACGTGGTCCAGAA   840
MT252677.1:28256-29515   AAACGTACTGCCACTAAAGCATACAATGTAACACAAGCTTTCGGCAGACGTGGTCCAGAA   840
MT370931.1:28212-29471   AAACGTACTGCCACTAAAGCATACAATGTAACACAAGCTTTCGGCAGACGTGGTCCAGAA   840
MT370932.1:28219-29478   AAACGTACTGCCACTAAAGCATACAATGTAACACAAGCTTTCGGCAGACGTGGTCCAGAA   840
MT370934.1:28219-29478   AAACGTACTGCCACTAAAGCATACAATGTAACACAAGCTTTCGGCAGACGTGGTCCAGAA   840
MT325568.1:28274-29533   AAACGTACTGCCACTAAAGCATACAATGTAACACAAGCTTTCGGCAGACGTGGTCCAGAA   840
MT325567.1:28274-29533   AAACGTACTGCCACTAAAGCATACAATGTAACACAAGCTTTCGGCAGACGTGGTCCAGAA   840
MT325577.1:28274-29533   AAACGTACTGCCACTAAAGCATACAATGTAACACAAGCTTTCGGCAGACGTGGTCCAGAA   840
MT325578.1:28274-29533   AAACGTACTGCCACTAAAGCATACAATGTAACACAAGCTTTCGGCAGACGTGGTCCAGAA   840
MT344950.1:28274-29533   AAACGTACTGCCACTAAAGCATACAATGTAACACAAGCTTTCGGCAGACGTGGTCCAGAA   840
MT344951.1:28274-29533   AAACGTACTGCCACTAAAGCATACAATGTAACACAAGCTTTCGGCAGACGTGGTCCAGAA   840
MT359865.1:28261-29520   AAACGTACTGCCACTAAAGCATACAATGTAACACAAGCTTTCGGCAGACGTGGTCCAGAA   840
MT066156.1:28274-29533   AAACGTACTGCCACTAAAGCATACAATGTAACACAAGCTTTCGGCAGACGTGGTCCAGAA   840
MT077125.1:28218-29477   AAACGTACTGCCACTAAAGCATACAATGTAACACAAGCTTTCGGCAGACGTGGTCCAGAA   840
NC_045512.2:28274-29533  AAACGTACTGCCACTAAAGCATACAATGTAACACAAGCTTTCGGCAGACGTGGTCCAGAA   840
MT126808.1:28274-29533   AAACGTACTGCCACTAAAGCATACAATGTAACACAAGCTTTCGGCAGACGTGGTCCAGAA   840
MT007544.1:28274-29533   AAACGTACTGCCACTAAAGCATACAATGTAACACAAGCTTTCGGCAGACGTGGTCCAGAA   840
MN988668.1:28273-29532   AAACGTACTGCCACTAAAGCATACAATGTAACACAAGCTTTCGGCAGACGTGGTCCAGAA   840
MT291833.1:28254-29513   AAACGTACTGCCACTAAAGCATACAATGTAACACAAGCTTTCGGCAGACGTGGTCCAGAA   840
MT123292.2:28274-29533   AAACGTACTGCCACTAAAGCATACAATGTAACACAAGCTTTCGGCAGACGTGGTCCAGAA   840
MT320538.2:28274-29533   AAACGTACTGCCACTAAAGCATACAATGTAACACAAGCTTTCGGCAGACGTGGTCCAGAA   840
MT276597.1:28254-29513   AAACGTACTGCCACTAAAGCATACAATGTAACACAAGCTTTCGGCAGACGTGGTCCAGAA   840
MT374105.1:28272-29531   AAACGTACTGCCACTAAAGCATACAATGTAACACAAGCTTTCGGCAGACGTGGTCCAGAA   840
MT326027.1:28268-29527   AAACGTACTGCCACTAAAGCATACAATGTAACACAAGCTTTCGGCAGACGTGGTCCAGAA   840
MT304483.1:28274-29533   AAACGTACTGCCACTAAAGCATACAATGTAACACAAGCTTTCGGCAGACGTGGTCCAGAA   840
MT371023.1:28219-29478   AAACGTACTGCCACTAAAGCATACAATGTAACACAAGCTTTCGGCAGACGTGGTCCAGAA   840
MT371015.1:28219-29478   AAACGTACTGCCACTAAAGCATACAATGTAACACAAGCTTTCGGCAGACGTGGTCCAGAA   840
MT114413.1:28274-29533   AAACGTACTGCCACTAAAGCATACAATGTAACACAAGCTTTCGGCAGACGTGGTCCAGAA   840
MT114412.1:28272-29531   AAACGTACTGCCACTAAAGCATACAATGTAACACAAGCTTTCGGCAGACGTGGTCCAGAA   840
                         ************************************************************

MT359866.1:28264-29523   CAAACCCAAGGAAATTTTGGGGACCAGGAACTAATCAGACAAGGAACTGATTACAAACAT   900
MT328033.1:28274-29533   CAAACCCAAGGAAATTTTGGGGACCAGGAACTAATCAGACAAGGAACTGATTACAAACAT   900
MT328032.1:28274-29533   CAAACCCAAGGAAATTTTGGGGACCAGGAACTAATCAGACAAGGAACTGATTACAAACAT   900
```

EP 4 146 833 B1

Figure 7 - cont.d

| Accession | Sequence | |
|---|---|---|
| MT371569.1:28200-29459 | CAAAACCCCAAGGAAATTTTGGGGACCAGGAA...CTAATCAGACAGA...CAAGGAACTGATTACAAAACAT | 900 |
| MT371568.1:28150-29409 | CAAAACCCCAAGGAAATTTTGGGGACCAGGAA...CTAATCAGACAGA...CAAGGAACTGATTACAAAACAT | 900 |
| MT371572.1:28166-29425 | CAAAACCCCAAGGAAATTTTGGGGACCAGGAA...CTAATCAGACAGA...CAAGGAACTGATTACAAAACAT | 900 |
| MT276598.1:28278-29537 | CAAAACCCCAAGGAAATTTTGGGGACCAGGAA...CTAATCAGACAGA...CAAGGAACTGATTACAAAACAT | 900 |
| MT374109.1:28271-29530 | CAAAACCCCAAGGAAATTTTGGGGACCAGGAA...CTAATCAGACAGA...CAAGGAACTGATTACAAAACAT | 900 |
| MT328035.1:28274-29533 | CAAAACCCCAAGGAAATTTTGGGGACCAGGAA...CTAATCAGACAGA...CAAGGAACTGATTACAAAACAT | 900 |
| MT292571.1:28220-29479 | CAAAACCCCAAGGAAATTTTGGGGACCAGGAA...CTAATCAGACAGA...CAAGGAACTGATTACAAAACAT | 900 |
| MT233519.1:28220-29479 | CAAAACCCCAAGGAAATTTTGGGGACCAGGAA...CTAATCAGACAGA...CAAGGAACTGATTACAAAACAT | 900 |
| MT198652.2:28220-29479 | CAAAACCCCAAGGAAATTTTGGGGACCAGGAA...CTAATCAGACAGA...CAAGGAACTGATTACAAAACAT | 900 |
| MT292570.1:28220-29479 | CAAAACCCCAAGGAAATTTTGGGGACCAGGAA...CTAATCAGACAGA...CAAGGAACTGATTACAAAACAT | 900 |
| MT372480.1:28239-29498 | CAAAACCCCAAGGAAATTTTGGGGACCAGGAA...CTAATCAGACAGA...CAAGGAACTGATTACAAAACAT | 900 |
| MT304474.1:28274-29533 | CAAAACCCCAAGGAAATTTTGGGGACCAGGAA...CTAATCAGACAGA...CAAGGAACTGATTACAAAACAT | 900 |
| MT304475.1:28274-29533 | CAAAACCCCAAGGAAATTTTGGGGACCAGGAA...CTAATCAGACAGA...CAAGGAACTGATTACAAAACAT | 900 |
| MT114414.1:28274-29533 | CAAAACCCCAAGGAAATTTTGGGGACCAGGAA...CTAATCAGACAGA...CAAGGAACTGATTACAAAACAT | 900 |
| MT252678.1:28225-29484 | CAAAACCCCAAGGAAATTTTGGGGACCAGGAA...CTAATCAGACAGA...CAAGGAACTGATTACAAAACAT | 900 |
| MT252677.1:28256-29515 | CAAAACCCCAAGGAAATTTTGGGGACCAGGAA...CTAATCAGACAGA...CAAGGAACTGATTACAAAACAT | 900 |
| MT370931.1:28212-29471 | CAAAACCCCAAGGAAATTTTGGGGACCAGGAA...CTAATCAGACAGA...CAAGGAACTGATTACAAAACAT | 900 |
| MT370932.1:28219-29478 | CAAAACCCCAAGGAAATTTTGGGGACCAGGAA...CTAATCAGACAGA...CAAGGAACTGATTACAAAACAT | 900 |
| MT370934.1:28219-29478 | CAAAACCCCAAGGAAATTTTGGGGACCAGGAA...CTAATCAGACAGA...CAAGGAACTGATTACAAAACAT | 900 |
| MT325568.1:28274-29533 | CAAAACCCCAAGGAAATTTTGGGGACCAGGAA...CTAATCAGACAGA...CAAGGAACTGATTACAAAACAT | 900 |
| MT325567.1:28274-29533 | CAAAACCCCAAGGAAATTTTGGGGACCAGGAA...CTAATCAGACAGA...CAAGGAACTGATTACAAAACAT | 900 |
| MT325577.1:28274-29533 | CAAAACCCCAAGGAAATTTTGGGGACCAGGAA...CTAATCAGACAGA...CAAGGAACTGATTACAAAACAT | 900 |
| MT325578.1:28274-29533 | CAAAACCCCAAGGAAATTTTGGGGACCAGGAA...CTAATCAGACAGA...CAAGGAACTGATTACAAAACAT | 900 |
| MT344950.1:28274-29533 | CAAAACCCCAAGGAAATTTTGGGGACCAGGAA...CTAATCAGACAGA...CAAGGAACTGATTACAAAACAT | 900 |
| MT344951.1:28274-29533 | CAAAACCCCAAGGAAATTTTGGGGACCAGGAA...CTAATCAGACAGA...CAAGGAACTGATTACAAAACAT | 900 |
| MT359865.1:28261-29520 | CAAAACCCCAAGGAAATTTTGGGGACCAGGAA...CTAATCAGACAGA...CAAGGAACTGATTACAAAACAT | 900 |
| MT066156.1:28274-29533 | CAAAACCCCAAGGAAATTTTGGGGACCAGGAA...CTAATCAGACAGA...CAAGGAACTGATTACAAAACAT | 900 |
| MT077125.1:28218-29477 | CAAAACCCCAAGGAAATTTTGGGGACCAGGAA...CTAATCAGACAGA...CAAGGAACTGATTACAAAACAT | 900 |
| NC_045512.2:28274-29533 | CAAAACCCCAAGGAAATTTTGGGGACCAGGAA...CTAATCAGACAGA...CAAGGAACTGATTACAAAACAT | 900 |
| MT126808.1:28274-29533 | CAAAACCCCAAGGAAATTTTGGGGACCAGGAA...CTAATCAGACAGA...CAAGGAACTGATTACAAAACAT | 900 |
| MT007544.1:28274-29533 | CAAAACCCCAAGGAAATTTTGGGGACCAGGAA...CTAATCAGACAGA...CAAGGAACTGATTACAAAACAT | 900 |
| MN988668.1:28273-29532 | CAAAACCCCAAGGAAATTTTGGGGACCAGGAA...CTAATCAGACAGA...CAAGGAACTGATTACAAAACAT | 900 |
| MT291833.1:28254-29513 | CAAAACCCCAAGGAAATTTTGGGGACCAGGAA...CTAATCAGACAGA...CAAGGAACTGATTACAAAACAT | 900 |
| MT123292.2:28274-29533 | CAAAACCCCAAGGAAATTTTGGGGACCAGGAA...CTAATCAGACAGA...CAAGGAACTGATTACAAAACAT | 900 |
| MT320538.2:28274-29533 | CAAAACCCCAAGGAAATTTTGGGGACCAGGAA...CTAATCAGACAGA...CAAGGAACTGATTACAAAACAT | 900 |
| MT276597.1:28254-29513 | CAAAACCCCAAGGAAATTTTGGGGACCAGGAA...CTAATCAGACAGA...CAAGGAACTGATTACAAAACAT | 900 |
| MT374105.1:28272-29531 | CAAAACCCCAAGGAAATTTTGGGGACCAGGAA...CTAATCAGACAGA...CAAGGAACTGATTACAAAACAT | 900 |
| MT326027.1:28268-29527 | CAAAACCCCAAGGAAATTTTGGGGACCAGGAA...CTAATCAGACAGA...CAAGGAACTGATTACAAAACAT | 900 |
| MT304483.1:28274-29533 | CAAAACCCCAAGGAAATTTTGGGGACCAGGAA...CTAATCAGACAGA...CAAGGAACTGATTACAAAACAT | 900 |
| MT371023.1:28219-29478 | CAAAACCCCAAGGAAATTTTGGGGACCAGGAA...CTAATCAGACAGA...CAAGGAACTGATTACAAAACAT | 900 |
| MT371015.1:28219-29478 | CAAAACCCCAAGGAAATTTTGGGGACCAGGAA...CTAATCAGACAGA...CAAGGAACTGATTACAAAACAT | 900 |
| MT114413.1:28274-29533 | CAAAACCCCAAGGAAATTTTGGGGACCAGGAA...CTAATCAGACAGA...CAAGGAACTGATTACAAAACAT | 900 |
| MT114412.1:28272-29531 | CAAAACCCCAAGGAAATTTTGGGGACCAGGAA...CTAATCAGACAGA...CAAGGAACTGATTACAAAACAT | 900 |
| | *********************************** ********************* | |

Figure 7 – cont.d

```
MT359866.1:28264-29523   TGGCCGCAAATTGCACAATTTGCCCCCAGCGCTTCAGCGTTCTTCGGAATGTCGCGCATT   960
MT328033.1:28274-29533   TGGCCGCAAATTGCACAATTTGCCCCCAGCGCTTCAGCGTTCTTCGGAATGTCGCGCATT   960
MT328032.1:28274-29533   TGGCCGCAAATTGCACAATTTGCCCCCAGCGCTTCAGCGTTCTTCGGAATGTCGCGCATT   960
MT371569.1:28200-29459   TGGCCGCAAATTGCACAATTTGCCCCCAGCGCTTCAGCGTTCTTCGGAATGTCGCGCATT   960
MT371568.1:28150-29409   TGGCCGCAAATTGCACAATTTGCCCCCAGCGCTTCAGCGTTCTTCGGAATGTCGCGCATT   960
MT371572.1:28166-29425   TGGCCGCAAATTGCACAATTTGCCCCCAGCGCTTCAGCGTTCTTCGGAATGTCGCGCATT   960
MT276598.1:28278-29537   TGGCCGCAAATTGCACAATTTGCCCCCAGCGCTTCAGCGTTCTTCGGAATGTCGCGCATT   960
MT374109.1:28271-29530   TGGCCGCAAATTGCACAATTTGCCCCCAGCGCTTCAGCGTTCTTCGGAATGTCGCGCATT   960
MT328035.1:28274-29533   TGGCCGCAAATTGCACAATTTGCCCCCAGCGCTTCAGCGTTCTTCGGAATGTCGCGCATT   960
MT292571.1:28220-29479   TGGCCGCAAATTGCACAATTTGCCCCCAGCGCTTCAGCGTTCTTCGGAATGTCGCGCATT   960
MT233519.1:28220-29479   TGGCCGCAAATTGCACAATTTGCCCCCAGCGCTTCAGCGTTCTTCGGAATGTCGCGCATT   960
MT198652.2:28220-29479   TGGCCGCAAATTGCACAATTTGCCCCCAGCGCTTCAGCGTTCTTCGGAATGTCGCGCATT   960
MT292570.1:28220-29479   TGGCCGCAAATTGCACAATTTGCCCCCAGCGCTTCAGCGTTCTTCGGAATGTCGCGCATT   960
MT372480.1:28239-29498   TGGCCGCAAATTGCACAATTTGCCCCCAGCGCTTCAGCGTTCTTCGGAATGTCGCGCATT   960
MT304474.1:28274-29533   TGGCCGCAAATTGCACAATTTGCCCCCAGCGCTTCAGCGTTCTTCGGAATGTCGCGCATT   960
MT304475.1:28274-29533   TGGCCGCAAATTGCACAATTTGCCCCCAGCGCTTCAGCGTTCTTCGGAATGTCGCGCATT   960
MT114414.1:28274-29533   TGGCCGCAAATTGCACAATTTGCCCCCAGCGCTTCAGCGTTCTTCGGAATGTCGCGCATT   960
MT252678.1:28225-29484   TGGCCGCAAATTGCACAATTTGCCCCCAGCGCTTCAGCGTTCTTCGGAATGTCGCGCATT   960
MT252677.1:28256-29515   TGGCCGCAAATTGCACAATTTGCCCCCAGCGCTTCAGCGTTCTTCGGAATGTCGCGCATT   960
MT370931.1:28212-29471   TGGCCGCAAATTGCACAATTTGCCCCCAGCGCTTCAGCGTTCTTCGGAATGTCGCGCATT   960
MT370932.1:28219-29478   TGGCCGCAAATTGCACAATTTGCCCCCAGCGTTCAGCGTTCTTCGGAATGTCGCGCATT    960
MT370934.1:28219-29478   TGGCCGCAAATTGCACAATTTGCCCCCAGCGCTTCAGCGTTCTTCGGAATGTCGCGCATT   960
MT325568.1:28274-29533   TGGCCGCAAATTGCACAATTTGCCCCCAGCGCTTCAGCGTTCTTCGGAATGTCGCGCATT   960
MT325567.1:28274-29533   TGGCCGCAAATTGCACAATTTGCCCCCAGCGCTTCAGCGTTCTTCGGAATGTCGCGCATT   960
MT325577.1:28274-29533   TGGCCGCAAATTGCACAATTTGCCCCCAGCGCTTCAGCGTTCTTCGGAATGTCGCGCATT   960
MT325578.1:28274-29533   TGGCCGCAAATTGCACAATTTGCCCCCAGCGCTTCAGCGTTCTTCGGAATGTCGCGCATT   960
MT344950.1:28274-29533   TGGCCGCAAATTGCACAATTTGCCCCCAGCGCTTCAGCGTTCTTCGGAATGTCGCGCATT   960
MT344951.1:28274-29533   TGGCCGCAAATTGCACAATTTGCCCCCAGCGCTTCAGCGTTCTTCGGAATGTCGCGCATT   960
MT359865.1:28261-29520   TGGCCGCAAATTGCACAATTTGCCCCCAGCGCTTCAGCGTTCTTCGGAATGTCGCGCATT   960
MT066156.1:28274-29533   TGGCCGCAAATTGCACAATTTGCCCCCAGCGCTTCAGCGTTCTTCGGAATGTCGCGCATT   960
MT077125.1:28218-29477   TGGCCGCAAATTGCACAATTTGCCCCCAGCGCTTCAGCGTTCTTCGGAATGTCGCGCATT   960
NC_045512.2:28274-29533  TGGCCGCAAATTGCACAATTTGCCCCCAGCGCTTCAGCGTTCTTCGGAATGTCGCGCATT   960
MT126808.1:28274-29533   TGGCCGCAAATTGCACAATTTGCCCCCAGCGCTTCAGCGTTCTTCGGAATGTCGCGCATT   960
MT007544.1:28274-29533   TGGCCGCAAATTGCACAATTTGCCCCCAGCGCTTCAGCGTTCTTCGGAATGTCGCGCATT   960
MN988668.1:28273-29532   TGGCCGCAAATTGCACAATTTGCCCCCAGCGCTTCAGCGTTCTTCGGAATGTCGCGCATT   960
MT291833.1:28254-29513   TGGCCGCAAATTGCACAATTTGCCCCCAGCGCTTCAGCGTTCTTCGGAATGTCGCGCATT   960
MT123292.2:28274-29533   TGGCCGCAAATTGCACAATTTGCCCCCAGCGCTTCAGCGTTCTTCGGAATGTCGCGCATT   960
MT320538.2:28274-29533   TGGCCGCAAATTGCACAATTTGCCCCCAGCGCTTCAGCGTTCTTCGGAATGTCGCGCATT   960
MT276597.1:28254-29513   TGGCCGCAAATTGCACAATTTGCCCCCAGCGCTTCAGCGTTCTTCGGAATGTCGCGCATT   960
MT374105.1:28272-29531   TGGCCGCAAATTGCACAATTTGCCCCCAGCGCTTCAGCGTTCTTCGGAATGTCGCGCATT   960
MT326027.1:28268-29527   TGGCCGCAAATTGCACAATTTGCCCCCAGCGCTTCAGCGTTCTTCGGAATGTCGCGCATT   960
MT304483.2:28274-29533   TGGCCGCAAATTGCACAATTTGCCCCCAGCGCTTCAGCGTTCTTCGGAATGTCGCGCATT   960
MT371023.1:28219-29478   TGGCCGCAAATTGCACAATTTGCCCCCAGCGCTTCAGCGTTCTTCGGAATGTCGCGCATT   960
MT371015.1:28219-29478   TGGCCGCAAATTGCACAATTTGCCCCCAGCGCTTCAGCGTTCTTCGGAATGTCGCGCATT   960
MT114413.1:28274-29533   TGGCCGCAAATTGCACAATTTGCCCCCAGCGCTTCAGCGTTCTTCGGAATGTCGCGCATT   960
```

```
Figure 7 - cont.d
MT114412.1:28272-29531   TGGCCGCAAATTGCACAATTGCCCCCAGCGCTTCAGCGTTCTTCGGAATGTGCGCATT   960
                         **************************************************

MT359866.1:28264-29523   GGCATGGAAGTCACACCTTCGGGAACGTGGTTGACCTACACAGGTGCCATCAAATTGGAT   1020
MT328033.1:28274-29533   GGCATGGAAGTCACACCTTCGGGAACGTGGTTGACCTACACAGGTGCCATCAAATTGGAT   1020
MT328032.1:28274-29533   GGCATGGAAGTCACACCTTCGGGAACGTGGTTGACCTACACAGGTGCCATCAAATTGGAT   1020
MT371569.1:28200-29459   GGCATGGAAGTCACACCTTCGGGAACGTGGTTGACCTACACAGGTGCCATCAAATTGGAT   1020
MT371568.1:28150-29409   GGCATGGAAGTCACACCTTCGGGAACGTGGTTGACCTACACAGGTGCCATCAAATTGGAT   1020
MT371572.1:28166-29425   GGCATGGAAGTCACACCTTCGGGAACGTGGTTGACCTACACAGGTGCCATCAAATTGGAT   1020
MT276598.1:28278-29537   GGCATGGAAGTCACACCTTCGGGAACGTGGTTGACCTACACAGGTGCCATCAAATTGGAT   1020
MT374109.1:28271-29530   GGCATGGAAGTCACACCTTCGGGAACGTGGTTGACCTACACAGGTGCCATCAAATTGGAT   1020
MT328035.1:28274-29533   GGCATGGAAGTCACACCTTCGGGAACGTGGTTGACCTACACAGGTGCCATCAAATTGGAT   1020
MT292571.1:28220-29479   GGCATGGAAGTCACACCTTCGGGAACGTGGTTGACCTACACAGGTGCCATCAAATTGGAT   1020
MT233519.1:28220-29479   GGCATGGAAGTCACACCTTCGGGAACGTGGTTGACCTACACAGGTGCCATCAAATTGGAT   1020
MT198652.2:28220-29479   GGCATGGAAGTCACACCTTCGGGAACGTGGTTGACCTACACAGGTGCCATCAAATTGGAT   1020
MT292570.1:28220-29479   GGCATGGAAGTCACACCTTCGGGAACGTGGTTGACCTACACAGGTGCCATCAAATTGGAT   1020
MT372480.1:28239-29498   GGCATGGAAGTCACACCTTCGGGAACGTGGTTGACCTACACAGGTGCCATCAAATTGGAT   1020
MT304474.1:28274-29533   GGCATGGAAGTCACACCTTCGGGAACGTGGTTGACCTACACAGGTGCCATCAAATTGGAT   1020
MT304475.1:28274-29533   GGCATGGAAGTCACACCTTCGGGAACGTGGTTGACCTACACAGGTGCCATCAAATTGGAT   1020
MT114414.1:28274-29533   GGCATGGAAGTCACACCTTCGGGAACGTGGTTGACCTACACAGGTGCCATCAAATTGGAT   1020
MT252678.1:28225-29484   GGCATGGAAGTCACACCTTCGGGAACGTGGTTGACCTACACAGGTGCCATCAAATTGGAT   1020
MT252677.1:28256-29515   GGCATGGAAGTCACACCTTCGGGAACGTGGTTGACCTACACAGGTGCCATCAAATTGGAT   1020
MT370931.1:28212-29471   GGCATGGAAGTCACACCTTCGGGAACGTGGTTGACCTACACAGGTGCCATCAAATTGGAT   1020
MT370932.1:28219-29478   GGCATGGAAGTCACACCTTCGGGAACGTGGTTGACCTACACAGGTGCCATCAAATTGGAT   1020
MT370934.1:28219-29478   GGCATGGAAGTCACACCTTCGGGAACGTGGTTGACCTACACAGGTGCCATCAAATTGGAT   1020
MT325568.1:28274-29533   GGCATGGAAGTCACACCTTCGGGAACGTGGTTGACCTACACAGGTGCCATCAAATTGGAT   1020
MT325567.1:28274-29533   GGCATGGAAGTCACACCTTCGGGAACGTGGTTGACCTACACAGGTGCCATCAAATTGGAT   1020
MT325577.1:28274-29533   GGCATGGAAGTCACACCTTCGGGAACGTGGTTGACCTACACAGGTGCCATCAAATTGGAT   1020
MT325578.1:28274-29533   GGCATGGAAGTCACACCTTCGGGAACGTGGTTGACCTACACAGGTGCCATCAAATTGGAT   1020
MT344950.1:28274-29533   GGCATGGAAGTCACACCTTCGGGAACGTGGTTGACCTACACAGGTGCCATCAAATTGGAT   1020
MT344951.1:28274-29533   GGCATGGAAGTCACACCTTCGGGAACGTGGTTGACCTACACAGGTGCCATCAAATTGGAT   1020
MT359865.1:28261-29520   GGCATGGAAGTCACACCTTCGGGAACGTGGTTGACCTACACAGGTGCCATCAAATTGGAT   1020
MT066156.1:28274-29533   GGCATGGAAGTCACACCTTCGGGAACGTGGTTGACCTACACAGGTGCCATCAAATTGGAT   1020
MT077125.1:28218-29477   GGCATGGAAGTCACACCTTCGGGAACGTGGTTGACCTACACAGGTGCCATCAAATTGGAT   1020
NC_045512.2:28274-29533  GGCATGGAAGTCACACCTTCGGGAACGTGGTTGACCTACACAGGTGCCATCAAATTGGAT   1020
MT126808.1:28274-29533   GGCATGGAAGTCACACCTTCGGGAACGTGGTTGACCTACACAGGTGCCATCAAATTGGAT   1020
MT007544.1:28274-29533   GGCATGGAAGTCACACCTTCGGGAACGTGGTTGACCTACACAGGTGCCATCAAATTGGAT   1020
MN988668.1:28273-29532   GGCATGGAAGTCACACCTTCGGGAACGTGGTTGACCTACACAGGTGCCATCAAATTGGAT   1020
MT291833.1:28254-29513   GGCATGGAAGTCACACCTTCGGGAACGTGGTTGACCTACACAGGTGCCATCAAATTGGAT   1020
MT123292.2:28274-29533   GGCATGGAAGTCACACCTTCGGGAACGTGGTTGACCTACACAGGTGCCATCAAATTGGAT   1020
MT320538.2:28274-29533   GGCATGGAAGTCACACCTTCGGGAACGTGGTTGACCTACACAGGTGCCATCAAATTGGAT   1020
MT276597.1:28254-29513   GGCATGGAAGTCACACCTTCGGGAACGTGGTTGACCTACACAGGTGCCATCAAATTGGAT   1020
MT374105.1:28272-29531   GGCATGGAAGTCACACCTTCGGGAACGTGGTTGACCTACACAGGTGCCATCAAATTGGAT   1020
MT326027.1:28268-29527   GGCATGGAAGTCACACCTTCGGGAACGTGGTTGACCTACACAGGTGCCATCAAATTGGAT   1020
MT304483.1:28274-29533   GGCATGGAAGTCACACCTTCGGGAACGTGGTTGACCTACACAGGTGCCATCAAATTGGAT   1020
```

Figure 7 - cont.d

```
MT371023.1:28219-29478    GGCATGGAAGTCACACCTTCGGGAACGTGGTTGACCTACACAGGTGCCATCAAATTGGAT    1020
MT371015.1:28219-29478    GGCATGGAAGTCACACCTTCGGGAACGTGGTTGACCTACACAGGTGCCATCAAATTGGAT    1020
MT114413.1:28274-29533    GGCATGGAAGTCACACCTTCGGGAACGTGGTTGACCTACACAGGTGCCATCAAATTGGAT    1020
MT114412.1:28272-29531    GGCATGGAAGTCACACCTTCGGGAACGTGGTTGACCTACACAGGTGCCATCAAATTGGAT    1020
                          ************************************************************


MT359866.1:28264-29523    GACAAAGATCCAAATTTCAAAGATCAAGTCATTTTGCTGAATAAGCATATTGACGCATAC    1080
MT328033.1:28274-29533    GACAAAGATCCAAATTTCAAAGATCAAGTCATTTTGCTGAATAAGCATATTGACGCATAC    1080
MT328032.1:28274-29533    GACAAAGATCCAAATTTCAAAGATCAAGTCATTTTGCTGAATAAGCATATTGACGCATAC    1080
MT371569.1:28200-29459    GACAAAGATCCAAATTTCAAAGATCAAGTCATTTTGCTGAATAAGCATATTGACGCATAC    1080
MT371568.1:28150-29409    GACAAAGATCCAAATTTCAAAGATCAAGTCATTTTGCTGAATAAGCATATTGACGCATAC    1080
MT371572.1:28166-29425    GACAAAGATCCAAATTTCAAAGATCAAGTCATTTTGCTGAATAAGCATATTGACGCATAC    1080
MT276598.1:28278-29537    GACAAAGATCCAAATTTCAAAGATCAAGTCATTTTGCTGAATAAGCATATTGACGCATAC    1080
MT374109.1:28271-29530    GACAAAGATCCAAATTTCAAAGATCAAGTCATTTTGCTGAATAAGCATATTGACGCATAC    1080
MT328035.1:28274-29533    GACAAAGATCCAAATTTCAAAGATCAAGTCATTTTGCTGAATAAGCATATTGACGCATAC    1080
MT292571.1:28220-29479    GACAAAGATCCAAATTTCAAAGATCAAGTCATTTTGCTGAATAAGCATATTGACGCATAC    1080
MT233519.1:28220-29479    GACAAAGATCCAAATTTCAAAGATCAAGTCATTTTGCTGAATAAGCATATTGACGCATAC    1080
MT198652.2:28220-29479    GACAAAGATCCAAATTTCAAAGATCAAGTCATTTTGCTGAATAAGCATATTGACGCATAC    1080
MT292570.1:28220-29479    GACAAAGATCCAAATTTCAAAGATCAAGTCATTTTGCTGAATAAGCATATTGACGCATAC    1080
MT372480.1:28239-29498    GACAAAGATCCAAATTTCAAAGATCAAGTCATTTTGCTGAATAAGCATATTGACGCATAC    1080
MT304474.1:28274-29533    GACAAAGATCCAAATTTCAAAGATCAAGTCATTTTGCTGAATAAGCATATTGACGCATAC    1080
MT304475.1:28274-29533    GACAAAGATCCAAATTTCAAAGATCAAGTCATTTTGCTGAATAAGCATATTGACGCATAC    1080
MT114414.1:28274-29533    GACAAAGATCCAAATTTCAAAGATCAAGTCATTTTGCTGAATAAGCATATTGACGCATAC    1080
MT252678.1:28225-29484    GACAAAGATCCAAATTTCAAAGATCAAGTCATTTTGCTGAATAAGCATATTGACGCATAC    1080
MT252677.1:28256-29515    GACAAAGATCCAAATTTCAAAGATCAAGTCATTTTGCTGAATAAGCATATTGACGCATAC    1080
MT370931.1:28212-29471    GACAAAGATCCAAATTTCAAAGATCAAGTCATTTTGCTGAATAAGCATATTGACGCATAC    1080
MT370932.1:28219-29478    GACAAAGATCCAAATTTCAAAGATCAAGTCATTTTGCTGAATAAGCATATTGACGCATAC    1080
MT370934.1:28219-29478    GACAAAGATCCAAATTTCAAAGATCAAGTCATTTTGCTGAATAAGCATATTGACGCATAC    1080
MT325568.1:28274-29533    GACAAAGATCCAAATTTCAAAGATCAAGTCATTTTGCTGAATAAGCATATTGACGCATAC    1080
MT325567.1:28274-29533    GACAAAGATCCAAATTTCAAAGATCAAGTCATTTTGCTGAATAAGCATATTGACGCATAC    1080
MT325577.1:28274-29533    GACAAAGATCCAAATTTCAAAGATCAAGTCATTTTGCTGAATAAGCATATTGACGCATAC    1080
MT325578.1:28274-29533    GACAAAGATCCAAATTTCAAAGATCAAGTCATTTTGCTGAATAAGCATATTGACGCATAC    1080
MT344950.1:28274-29533    GACAAAGATCCAAATTTCAAAGATCAAGTCATTTTGCTGAATAAGCATATTGACGCATAC    1080
MT344951.1:28274-29533    GACAAAGATCCAAATTTCAAAGATCAAGTCATTTTGCTGAATAAGCATATTGACGCATAC    1080
MT359865.1:28261-29520    GACAAAGATCCAAATTTCAAAGATCAAGTCATTTTGCTGAATAAGCATATTGACGCATAC    1080
MT066156.1:28274-29533    GACAAAGATCCAAATTTCAAAGATCAAGTCATTTTGCTGAATAAGCATATTGACGCATAC    1080
MT077125.1:28218-29477    GACAAAGATCCAAATTTCAAAGATCAAGTCATTTTGCTGAATAAGCATATTGACGCATAC    1080
NC_045512.2:28274-29533   GACAAAGATCCAAATTTCAAAGATCAAGTCATTTTGCTGAATAAGCATATTGACGCATAC    1080
MT126808.1:28274-29533    GACAAAGATCCAAATTTCAAAGATCAAGTCATTTTGCTGAATAAGCATATTGACGCATAC    1080
MT007544.1:28274-29533    GACAAAGATCCAAATTTCAAAGATCAAGTCATTTTGCTGAATAAGCATATTGACGCATAC    1080
MN988668.1:28273-29532    GACAAAGATCCAAATTTCAAAGATCAAGTCATTTTGCTGAATAAGCATATTGACGCATAC    1080
MT291833.1:28254-29513    GACAAAGATCCAAATTTCAAAGATCAAGTCATTTTGCTGAATAAGCATATTGACGCATAC    1080
MT123292.2:28274-29533    GACAAAGATCCAAATTTCAAAGATCAAGTCATTTTGCTGAATAAGCATATTGACGCATAC    1080
MT320538.2:28274-29533    GACAAAGATCCAAATTTCAAAGATCAAGTCATTTTGCTGAATAAGCATATTGACGCATAC    1080
MT276597.1:28254-29513    GACAAAGATCCAAATTTCAAAGATCAAGTCATTTTGCTGAATAAGCATATTGACGCATAC    1080
```

EP 4 146 833 B1

## Figure 7 – cont.d

```
MT374105.1:28272-29531    GACAAAGATCCAAATTTCAAAGATCAAGTCATTTTGCTGAATAAGCATATTGACGCATAC    1080
MT326027.1:28268-29527    GACAAAGATCCAAATTTCAAAGATCAAGTCATTTTGCTGAATAAGCATATTGACGCATAC    1080
MT304483.1:28274-29533    GACAAAGATCCAAATTTCAAAGATCAAGTCATTTTGCTGAATAAGCATATTGACGCATAC    1080
MT371023.1:28219-29478    GACAAAGATCCAAATTTCAAAGATCAAGTCATTTTGCTGAATAAGCATATTGACGCATAC    1080
MT371015.1:28219-29478    GACAAAGATCCAAATTTCAAAGATCAAGTCATTTTGCTGAATAAGCATATTGACGCATAC    1080
MT114413.1:28274-29533    GACAAAGATCCAAATTTCAAAGATCAAGTCATTTTGCTGAATAAGCATATTGACGCATAC    1080
MT114412.1:28272-29531    GACAAAGATCCAAATTTCAAAGATCAAGTCATTTTGCTGAATAAGCATATTGACGCATAC    1080
                          ************************************************************


MT359866.1:28264-29523    AAAACATTCCCACCAACAGAGCCTAAAAAGGACAAAAAGAAGAAGGCTGATGAAACTCAA    1140
MT328033.1:28274-29533    AAAACATTCCCACCAACAGAGCCTAAAAAGGACAAAAAGAAGAAGGCTGATGAAACTCAA    1140
MT328032.1:28274-29533    AAAACATTCCCACCAACAGAGCCTAAAAAGGACAAAAAGAAGAAGGCTGATGAAACTCAA    1140
MT371569.1:28200-29459    AAAACATTCCCACCAACAGAGCCTAAAAAGGACAAAAAGAAGAAGGCTGATGAAACTCAA    1140
MT371568.1:28150-29409    AAAACATTCCCACCAACAGAGCCTAAAAAGGACAAAAAGAAGAAGGCTGATGAAACTCAA    1140
MT371572.1:28166-29425    AAAACATTCCCACCAACAGAGCCTAAAAAGGACAAAAAGAAGAAGGCTGATGAAACTCAA    1140
MT276598.1:28278-29537    AAAACATTCCCACCAACAGAGCCTAAAAAGGACAAAAAGAAGAAGGCTGATGAAACTCAA    1140
MT374109.1:28271-29530    AAAACATTCCCACCAACAGAGCCTAAAAAGGACAAAAAGAAGAAGGCTGATGAAACTCAA    1140
MT328035.1:28274-29533    AAAACATTCCCACCAACAGAGCCTAAAAAGGACAAAAAGAAGAAGGCTGATGAAACTCAA    1140
MT292571.1:28220-29479    AAAACATTCCCACCAACAGAGCCTAAAAAGGACAAAAAGAAGAAGGCTGATGAAACTCAA    1140
MT233519.1:28220-29479    AAAACATTCCCACCAACAGAGCCTAAAAAGGACAAAAAGAAGAAGGCTGATGAAACTCAA    1140
MT198652.2:28220-29479    AAAACATTCCCACCAACAGAGCCTAAAAAGGACAAAAAGAAGAAGGCTGATGAAACTCAA    1140
MT292570.1:28220-29479    AAAACATTCCCACCAACAGAGCCTAAAAAGGACAAAAAGAAGAAGGCTGATGAAACTCAA    1140
MT372480.1:28239-29498    AAAACATTCCCACCAACAGAGCCTAAAAAGGACAAAAAGAAGAAGGCTGATGAAACTCAA    1140
MT304474.1:28274-29533    AAAACATTCCCACCAACAGAGCCTAAAAAGGACAAAAAGAAGAAGGCTGATGAAACTCAA    1140
MT304475.1:28274-29533    AAAACATTCCCACCAACAGAGCCTAAAAAGGACAAAAAGAAGAAGGCTGATGAAACTCAA    1140
MT114414.1:28274-29533    AAAACATTCCCACCAACAGAGCCTAAAAAGGACAAAAAGAAGAAGGCTGATGAAACTCAA    1140
MT252678.1:28225-29484    AAAACATTCCCACCAACAGAGCCTAAAAAGGACAAAAAGAAGAAGGCTGATGAAACTCAA    1140
MT252677.1:28256-29515    AAAACATTCCCACCAACAGAGCCTAAAAAGGACAAAAAGAAGAAGGCTGATGAAACTCAA    1140
MT370931.1:28212-29471    AAAACATTCCCACCAACAGAGCCTAAAAAGGACAAAAAGAAGAAGGCTGATGAAACTCAA    1140
MT370932.1:28219-29478    AAAACATTCCCACCAACAGAGCCTAAAAAGGACAAAAAGAAGAAGGCTGATGAAACTCAA    1140
MT370934.1:28219-29478    AAAACATTCCCACCAACAGAGCCTAAAAAGGACAAAAAGAAGAAGGCTGATGAAACTCAA    1140
MT325568.1:28274-29533    AAAACATTCCCACCAACAGAGCCTAAAAAGGACAAAAAGAAGAAGGCTGATGAAACTCAA    1140
MT325567.1:28274-29533    AAAACATTCCCACCAACAGAGCCTAAAAAGGACAAAAAGAAGAAGGCTGATGAAACTCAA    1140
MT325577.1:28274-29533    AAAACATTCCCACCAACAGAGCCTAAAAAGGACAAAAAGAAGAAGGCTGATGAAACTCAA    1140
MT325578.1:28274-29533    AAAACATTCCCACCAACAGAGCCTAAAAAGGACAAAAAGAAGAAGGCTGATGAAACTCAA    1140
MT344950.1:28274-29533    AAAACATTCCCACCAACAGAGCCTAAAAAGGACAAAAAGAAGAAGGCTGATGAAACTCAA    1140
MT344951.1:28274-29533    AAAACATTCCCACCAACAGAGCCTAAAAAGGACAAAAAGAAGAAGGCTGATGAAACTCAA    1140
MT359865.1:28261-29520    AAAACATTCCCACCAACAGAGCCTAAAAAGGACAAAAAGAAGAAGGCTGATGAAACTCAA    1140
MT066156.1:28274-29533    AAAACATTCCCACCAACAGAGCCTAAAAAGGACAAAAAGAAGAAGGCTGATGAAACTCAA    1140
MT077125.1:28218-29477    AAAACATTCCCACCAACAGAGCCTAAAAAGGACAAAAAGAAGAAGGCTGATGAAACTCAA    1140
NC_045512.2:28274-29533   AAAACATTCCCACCAACAGAGCCTAAAAAGGACAAAAAGAAGAAGGCTGATGAAACTCAA    1140
MT126808.1:28274-29533    AAAACATTCCCACCAACAGAGCCTAAAAAGGACAAAAAGAAGAAGGCTGATGAAACTCAA    1140
MT007544.1:28274-29533    AAAACATTCCCACCAACAGAGCCTAAAAAGGACAAAAAGAAGAAGGCTGATGAAACTCAA    1140
MN988668.1:28273-29532    AAAACATTCCCACCAACAGAGCCTAAAAAGGACAAAAAGAAGAAGGCTGATGAAACTCAA    1140
MT291833.1:28254-29513    AAAACATTCCCACCAACAGAGCCTAAAAAGGACAAAAAGAAGAAGGCTGATGAAACTCAA    1140
```

Figure 7 - cont.d

```
MT123292.2:28274-29533    AAAACATTCCCACCAACAGAGCCTAAAAAGGACAAAAAGAAGAAGGCTGATGAAACTCAA    1140
MT320538.2:28274-29533    AAAACATTCCCACCAACAGAGCCTAAAAAGGACAAAAAGAAGAAGGCTGATGAAACTCAA    1140
MT276597.1:28254-29513    AAAACATTCCCACCAACAGAGCCTAAAAAGGACAAAAAGAAGAAGGCTGATGAAACTCAA    1140
MT374105.1:28272-29531    AAAACATTCCCACCAACAGAGCCTAAAAAGGACAAAAAGAAGAAGGCTGATGAAACTCAA    1140
MT326027.1:28268-29527    AAAACATTCCCACCAACAGAGCCTAAAAAGGACAAAAAGAAGAAGGCTGATGAAACTCAA    1140
MT304483.1:28274-29533    AAAACATTCCCACCAACAGAGCCTAAAAAGGACAAAAAGAAGAAGGCTGATGAAACTCAA    1140
MT371023.1:28219-29478    AAAACATTCCCACCAACAGAGCCTAAAAAGGACAAAAAGAAGAAGGCTGATGAAACTCAA    1140
MT371015.1:28219-29478    AAAACATTCCCACCAACAGAGCCTAAAAAGGACAAAAAGAAGAAGGCTGATGAAACTCAA    1140
MT114413.1:28274-29533    AAAACATTCCCACCAACAGAGCCTAAAAAGGACAAAAAGAAGAAGGCTGATGAAACTCAA    1140
MT114412.1:28272-29531    AAAACATTCCCACCAACAGAGCCTAAAAAGGACAAAAAGAAGAAGGCTGATGAAACTCAA    1140
                          ************************************************************


MT359866.1:28264-29523    GCCTTACCGCAGAGACAGAAGAAACAGCAAACTGTGACTCTTCTTCCTGCTGCAGATTTG    1200
MT328033.1:28274-29533    GCCTTACCGCAGAGACAGAAGAAACAGCAAACTGTGACTCTTCTTCCTGCTGCAGATTTG    1200
MT328032.1:28274-29533    GCCTTACCGCAGAGACAGAAGAAACAGCAAACTGTGACTCTTCTTCCTGCTGCAGATTTG    1200
MT371569.1:28200-29459    GCCTTACCGCAGAGACAGAAGAAACAGCAAACTGTGACTCTTCTTCCTGCTGCAGATTTG    1200
MT371568.1:28150-29409    GCCTTACCGCAGAGACAGAAGAAACAGCAAACTGTGACTCTTCTTCCTGCTGCAGATTTG    1200
MT371572.1:28166-29425    GCCTTACCGCAGAGACAGAAGAAACAGCAAACTGTGACTCTTCTTCCTGCTGCAGATTTG    1200
MT276598.1:28278-29537    GCCTTACCGCAGAGACAGAAGAAACAGCAAACTGTGACTCTTCTTCCTGCTGCAGATTTG    1200
MT374109.1:28271-29530    GCCTTACCGCAGAGACAGAAGAAACAGCAAACTGTGACTCTTCTTCCTGCTGCAGATTTG    1200
MT328035.1:28274-29533    GCCTTACCGCAGAGACAGAAGAAACAGCAAACTGTGACTCTTCTTCCTGCTGCAGATTTG    1200
MT292571.1:28220-29479    GCCTTACCGCAGAGACAGAAGAAACAGCAAACTGTGACTCTTCTTCCTGCTGCAGATTTG    1200
MT233519.1:28220-29479    GCCTTACCGCAGAGACAGAAGAAACAGCAAACTGTGACTCTTCTTCCTGCTGCAGATTTG    1200
MT198652.2:28220-29479    GCCTTACCGCAGAGACAGAAGAAACAGCAAACTGTGACTCTTCTTCCTGCTGCAGATTTG    1200
MT292570.1:28220-29479    GCCTTACCGCAGAGACAGAAGAAACAGCAAACTGTGACTCTTCTTCCTGCTGCAGATTTG    1200
MT372480.1:28239-29498    GCCTTACCGCAGAGACAGAAGAAACAGCAAACTGTGACTCTTCTTCCTGCTGCAGATTTG    1200
MT304474.1:28274-29533    GCCTTACCGCAGAGACAGAAGAAACAGCAAACTGTGACTCTTCTTCCTGCTGCAGATTTG    1200
MT304475.1:28274-29533    GCCTTACCGCAGAGACAGAAGAAACAGCAAACTGTGACTCTTCTTCCTGCTGCAGATTTG    1200
MT114414.1:28274-29533    GCCTTACCGCAGAGACAGAAGAAACAGCAAACTGTGACTCTTCTTCCTGCTGCAGATTTG    1200
MT252678.1:28225-29484    GCCTTACCGCAGAGACAGAAGAAACAGCAAACTGTGACTCTTCTTCCTGCTGCAGATTTG    1200
MT252677.1:28256-29515    GCCTTACCGCAGAGACAGAAGAAACAGCAAACTGTGACTCTTCTTCCTGCTGCAGATTTG    1200
MT370931.1:28212-29471    GCCTTACCGCAGAGACAGAAGAAACAGCAAACTGTGACTCTTCTTCCTGCTGCAGATTTG    1200
MT370932.1:28219-29478    GCCTTACCGCAGAGACAGAAGAAACAGCAAACTGTGACTCTTCTTCCTGCTGCAGATTTG    1200
MT370934.1:28219-29478    GCCTTACCGCAGAGACAGAAGAAACAGCAAACTGTGACTCTTCTTCCTGCTGCAGATTTG    1200
MT325568.1:28274-29533    GCCTTACCGCAGAGACAGAAGAAACAGCAAACTGTGACTCTTCTTCCTGCTGCAGATTTG    1200
MT325567.1:28274-29533    GCCTTACCGCAGAGACAGAAGAAACAGCAAACTGTGACTCTTCTTCCTGCTGCAGATTTG    1200
MT325577.1:28274-29533    GCCTTACCGCAGAGACAGAAGAAACAGCAAACTGTGACTCTTCTTCCTGCTGCAGATTTG    1200
MT325578.1:28274-29533    GCCTTACCGCAGAGACAGAAGAAACAGCAAACTGTGACTCTTCTTCCTGCTGCAGATTTG    1200
MT344950.1:28274-29533    GCCTTACCGCAGAGACAGAAGAAACAGCAAACTGTGACTCTTCTTCCTGCTGCAGATTTG    1200
MT344951.1:28274-29533    GCCTTACCGCAGAGACAGAAGAAACAGCAAACTGTGACTCTTCTTCCTGCTGCAGATTTG    1200
MT359865.1:28261-29520    GCCTTACCGCAGAGACAGAAGAAACAGCAAACTGTGACTCTTCTTCCTGCTGCAGATTTG    1200
MT066156.1:28274-29533    GCCTTACCGCAGAGACAGAAGAAACAGCAAACTGTGACTCTTCTTCCTGCTGCAGATTTG    1200
MT077125.1:28218-29477    GCCTTACCGCAGAGACAGAAGAAACAGCAAACTGTGACTCTTCTTCCTGCTGCAGATTTG    1200
NC_045512.2:28274-29533   GCCTTACCGCAGAGACAGAAGAAACAGCAAACTGTGACTCTTCTTCCTGCTGCAGATTTG    1200
MT126808.1:28274-29533    GCCTTACCGCAGAGACAGAAGAAACAGCAAACTGTGACTCTTCTTCCTGCTGCAGATTTG    1200
```

## Figure 7 - cont.d

```
MT007544.1:28274-29533    GCCTTACCGCAGAGACAGAAGAAACAGCAAACTGTGACTCTTCTTCCTGCTGCAGATTTG    1200
MN988668.1:28273-29532    GCCTTACCGCAGAGACAGAAGAAACAGCAAACTGTGACTCTTCTTCCTGCTGCAGATTTG    1200
MT291833.1:28254-29513    GCCTTACCGCAGAGACAGAAGAAACAGCAAACTGTGACTCTTCTTCCTGCTGCAGATTTG    1200
MT123292.2:28274-29533    GCCTTACCGCAGAGACAGAAGAAACAGCAAACTGTGACTCTTCTTCCTGCTGCAGATTTG    1200
MT320538.2:28274-29533    GCCTTACCGCAGAGACAGAAGAAACAGCAAACTGTGACTCTTCTTCCTGCTGCAGATTTG    1200
MT276597.1:28254-29513    GCCTTACCGCAGAGACAGAAGAAACAGCAAACTGTGACTCTTCTTCCTGCTGCAGATTTG    1200
MT374105.1:28272-29531    GCCTTACCGCAGAGACAGAAGAAACAGCAAACTGTGACTCTTCTTCCTGCTGCAGATTTG    1200
MT326027.1:28268-29527    GCCTTACCGCAGAGACAGAAGAAACAGCAAACTGTGACTCTTCTTCCTGCTGCAGATTTG    1200
MT304483.1:28274-29533    GCCTTACCGCAGAGACAGAAGAAACAGCAAACTGTGACTCTTCTTCCTGCTGCAGATTTG    1200
MT371023.1:28219-29478    GCCTTACCGCAGAGACAGAAGAAACAGCAAACTGTGACTCTTCTTCCTGCTGCAGATTTG    1200
MT371015.1:28219-29478    GCCTTACCGCAGAGACAGAAGAAACAGCAAACTGTGACTCTTCTTCCTGCTGCAGATTTG    1200
MT114413.1:28274-29533    GCCTTACCGCAGAGACAGAAGAAACAGCAAACTGTGACTCTTCTTCCTGCTGCAGATTTG    1200
MT114412.1:28272-29531    GCCTTACCGCAGAGACAGAAGAAACAGCAAACTGTGACTCTTCTTCCTGCTGCAGATTTG    1200
                          ************************************************************


MT359866.1:28264-29523    GATGATTTCTCCAAACAATTGCAACAATCCATGAGCAGTGCTGACTCAACTCAGGCCTAA    1260
MT328033.1:28274-29533    GATGATTTCTCCAAACAATTGCAACAATCCATGAGCAGTGCTGACTCAACTCAGGCCTAA    1260
MT328032.1:28274-29533    GATGATTTCTCCAAACAATTGCAACAATCCATGAGCAGTGCTGACTCAACTCAGGCCTAA    1260
MT371569.1:28200-29459    GATGATTTCTCCAAACAATTGCAACAATCCATGAGCAGTGCTGACTCAACTCAGGCCTAA    1260
MT371568.1:28150-29409    GATGATTTCTCCAAACAATTGCAACAATCCATGAGCAGTGCTGACTCAACTCAGGCCTAA    1260
MT371572.1:28166-29425    GATGATTTCTCCAAACAATTGCAACAATCCATGAGCAGTGCTGACTCAACTCAGGCCTAA    1260
MT276598.1:28278-29537    GATGATTTCTCCAAACAATTGCAACAATCCATGAGCAGTGCTGACTCAACTCAGGCCTAA    1260
MT374109.1:28271-29530    GATGATTTCTCCAAACAATTGCAACAATCCATGAGCAGTGCTGACTCAACTCAGGCCTAA    1260
MT328035.1:28274-29533    GATGATTTCTCCAAACAATTGCAACAATCCATGAGCAGTGCTGACTCAACTCAGGCCTAA    1260
MT292571.1:28220-29479    GATGATTTCTCCAAACAATTGCAACAATCCATGAGCAGTGCTGACTCAACTCAGGCCTAA    1260
MT233519.1:28220-29479    GATGATTTCTCCAAACAATTGCAACAATCCATGAGCAGTGCTGACTCAACTCAGGCCTAA    1260
MT198652.2:28220-29479    GATGATTTCTCCAAACAATTGCAACAATCCATGAGCAGTGCTGACTCAACTCAGGCCTAA    1260
MT292570.1:28220-29479    GATGATTTCTCCAAACAATTGCAACAATCCATGAGCAGTGCTGACTCAACTCAGGCCTAA    1260
MT372480.1:28239-29498    GATGATTTCTCCAAACAATTGCAACAATCCATGAGCAGTGCTGACTCAACTCAGGCCTAA    1260
MT304474.1:28274-29533    GATGATTTCTCCAAACAATTGCAACAATCCATGAGCAGTGCTGACTCAACTCAGGCCTAA    1260
MT304475.1:28274-29533    GATGATTTCTCCAAACAATTGCAACAATCCATGAGCAGTGCTGACTCAACTCAGGCCTAA    1260
MT114414.1:28274-29533    GATGATTTCTCCAAACAATTGCAACAATCCATGAGCAGTGCTGACTCAACTCAGGCCTAA    1260
MT252678.1:28225-29484    GATGATTTCTCCAAACAATTGCAACAATCCATGAGCAGTGCTGACTCAACTCAGGCCTAA    1260
MT252677.1:28256-29515    GATGATTTCTCCAAACAATTGCAACAATCCATGAGCAGTGCTGACTCAACTCAGGCCTAA    1260
MT370931.1:28212-29471    GATGATTTCTCCAAACAATTGCAACAATCCATGAGCAGTGCTGACTCAACTCAGGCCTAA    1260
MT370932.1:28219-29478    GATGATTTCTCCAAACAATTGCAACAATCCATGAGCAGTGCTGACTCAACTCAGGCCTAA    1260
MT370934.1:28219-29478    GATGATTTCTCCAAACAATTGCAACAATCCATGAGCAGTGCTGACTCAACTCAGGCCTAA    1260
MT325568.1:28274-29533    GATGATTTCTCCAAACAATTGCAACAATCCATGAGCAGTGCTGACTCAACTCAGGCCTAA    1260
MT325567.1:28274-29533    GATGATTTCTCCAAACAATTGCAACAATCCATGAGCAGTGCTGACTCAACTCAGGCCTAA    1260
MT325577.1:28274-29533    GATGATTTCTCCAAACAATTGCAACAATCCATGAGCAGTGCTGACTCAACTCAGGCCTAA    1260
MT325578.1:28274-29533    GATGATTTCTCCAAACAATTGCAACAATCCATGAGCAGTGCTGACTCAACTCAGGCCTAA    1260
MT344950.1:28274-29533    GATGATTTCTCCAAACAATTGCAACAATCCATGAGCAGTGCTGACTCAACTCAGGCCTAA    1260
MT344951.1:28274-29533    GATGATTTCTCCAAACAATTGCAACAATCCATGAGCAGTGCTGACTCAACTCAGGCCTAA    1260
MT359865.1:28261-29520    GATGATTTCTCCAAACAATTGCAACAATCCATGAGCAGTGCTGACTCAACTCAGGCCTAA    1260
MT066156.1:28274-29533    GATGATTTCTCCAAACAATTGCAACAATCCATGAGCAGTGCTGACTCAACTCAGGCCTAA    1260
```

EP 4 146 833 B1

Figure 7 – cont.d

```
MT077125.1:28218-29477    GATGATTTCTCCAAACAATTGCAACAATCCATGAGCAGTGCTGACTCAACTCAGGCCTAA    1260
NC_045512.2:28274-29533   GATGATTTCTCCAAACAATTGCAACAATCCATGAGCAGTGCTGACTCAACTCAGGCCTAA    1260
MT126808.1:28274-29533    GATGATTTCTCCAAACAATTGCAACAATCCATGAGCAGTGCTGACTCAACTCAGGCCTAA    1260
MT007544.1:28274-29533    GATGATTTCTCCAAACAATTGCAACAATCCATGAGCAGTGCTGACTCAACTCAGGCCTAA    1260
MN988668.1:28273-29532    GATGATTTCTCCAAACAATTGCAACAATCCATGAGCAGTGCTGACTCAACTCAGGCCTAA    1260
MT291833.1:28254-29513    GATGATTTCTCCAAACAATTGCAACAATCCATGAGCAGTGCTGACTCAACTCAGGCCTAA    1260
MT123292.2:28274-29533    GATGATTTCTCCAAACAATTGCAACAATCCATGAGCAGTGCTGACTCAACTCAGGCCTAA    1260
MT320538.2:28274-29533    GATGATTTCTCCAAACAATTGCAACAATCCATGAGCAGTGCTGACTCAACTCAGGCCTAA    1260
MT276597.1:28254-29513    GATGATTTCTCCAAACAATTGCAACAATCCATGAGCAGTGCTGACTCAACTCAGGCCTAA    1260
MT374105.1:28272-29531    GATGATTTCTCCAAACAATTGCAACAATCCATGAGCAGTGCTGACTCAACTCAGGCCTAA    1260
MT326027.1:28268-29527    GATGATTTCTCCAAACAATTGCAACAATCCATGAGCAGTGCTGACTCAACTCAGGCCTAA    1260
MT304483.1:28274-29533    GATGATTTCTCCAAACAATTGCAACAATCCATGAGCAGTGCTGACTCAACTCAGGCCTAA    1260
MT371023.1:28219-29478    GATGATTTCTCCAAACAATTGCAACAATCCATGAGCAGTGCTGACTCAACTCAGGCCTAA    1260
MT371015.1:28219-29478    GATGATTTCTCCAAACAATTGCAACAATCCATGAGCAGTGCTGACTCAACTCAGGCCTAA    1260
MT114413.1:28274-29533    GATGATTTCTCCAAACAATTGCAACAATCCATGAGCAGTGCTGACTCAACTCAGGCCTAA    1260
MT114412.1:28272-29531    GATGATTTCTCCAAACAATTGCAACAATCCATGAGCAGTGCTGACTCAACTCAGGCCTAA    1260
                          ************************************************************
```

Figure 8

```
GTGGTGTTTA TTACCCTGAC AAAGTTTTCA GATCCTCAGT TTTACATTCA ACTCAGGACT TGTTCTTACC TTTCTTTTCC AATGTTACTT GGTTCCATGC TATACATGTC TCTGGGACCA ATGGTACTAA GAGGTTTGAT
101        111        121        131        141        151        161        171        181        191        201        211        221        231
    GGTGTTTA TTACCCTGAC AAAGTTTTCA GATCCTCAGT TTTACATTC  tgagtcctga acaagaatgg aaagaaaagg ttacaatgaa ccaaggtac              C TCTGGGACCA ATGGTACTAA GAG
              F3                              F2                        LF                            F1c                                      B1c

AACCCTGTCC TACCATTTAA TGATGGTGTT TATTTTGCTT CCACTGAGAA GTCTAACATA ATAAGAGGCT GGATTTTTGG TACTACTTTA
241        251        261        271        281        291        301        311        321
    CTGTCC TACCATTTAA TGATGGTGT       acaaa gatgactctt cag        auauuuu uuuuuuuuuu auu
              LB                           B2                              B3
```

Figure 9

Figure 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0028082 A1
- WO 0224902 A1
- WO 0177317 A1
- CN 103146847 B
- US 8389221 B2
- CN 102286637 B

**Non-patent literature cited in the description**

- **YH BAEK et al.** Development of a reverse transcription-loop-mediated isothermal amplification as a rapid early-detection method for novel SARS-CoV-2. *Emerging Microbes & Infections*, 2020, vol. 9 (1), 998-1007

- **HUANG et al.** RT-LAMP for rapid diagnosis of coronavirus SARS-CoV-2. *MICROBIAL BIOTECHNOLOGY*, 25 April 2020, vol. 13 (4), 950-961